# EUROPEAN PATENT APPLICATION

(11) **EP 4 810 034 A2**
(43) Date of publication of application: **23.09.2026**
(21) Application number: 26193444.2
(22) Date of filing: 08.11.2019
(51) Int. Cl.: A61K 31/404

(54) **METHODS AND COMPOSITIONS FOR TREATING OR PREVENTING THE DEVELOPMENT OF FOOD ALLERGIES**

(30) Priority: 09.11.2018 US 201862758161 P; 29.01.2019 US 201962798224 P
(62) Document number(s) of the earlier application(s) in accordance with Art. 76 EPC: 19882419.5 / 3 876 966
(71) Applicant: CHILDREN'S MEDICAL CENTER CORPORATION, Boston, MA 02115 (US)
(72) Inventor: CHATILA, Talal A., Boston, 02511 (US); ABDEL-GADIR, Azza, Boston, 02511 (US); VICTOR, Emmanuel Stephen, 070019 Mysuru (IN); RACHID, Rima, Belmont, 02478 (US)
(74) Representative: Purdylucey Intellectual Property

(57) **Abstract**

Described herein are methods and compositions for treating or preventing a food allergy. Aspects of the invention relates to administering to a subject an agent that induces a regulatory T cell, or population thereof, that expresses RORγt. Another aspect of the invention relates to administering a fecal matter transplant to a subject having reduced RORyt-expressing regulatory T cells to treat or prevent a food allergy.

## Description

### CROSS-REFERENCE TO RELATED APPLICATIONS

This application claims benefit under 35 U.S.C. § 119(e) of U.S. Provisional Application No. 62/758,161 filed November 9, 2018, and U.S. Provisional Application No. 62/798,224 filed January 29, 2019, the contents of which are incorporated herein by reference in their entirety.

### FIELD OF THE INVENTION

The field of the invention relates to the treatment or prevention of food allergies.

### GOVERNMENT SUPPORT

This invention was made with Government support under Grant Nos 1R56AI117983 and 1R01AI126915 awarded by the National Institutes of Health. The Government has certain rights in the invention.

### SEQUENCE LISTING

The instant application contains a Sequence Listing which has been submitted in ASCII format via EFS-Web and is hereby incorporated by reference in its entirety. Said ASCII copy, created on November 7, 2019, is named 701039-093960WOPT-SL_ST25.txt and is 80,585 bytes in size.

### BACKGROUND

Food allergies are a growing public health problem in both developed and rapidly developing countries and affects large numbers of children and adults. The incidence of food allergy has increased dramatically in the last few decades. This increase can be associated with sensitization to multiple foods in up to 50% of subjects. Growing evidence indicates that the microbial flora and their associated metabolites are a key environmental influence in programming oral tolerance.

### SUMMARY

The present invention is based, in part, on the finding that infants having a food allergy (FA infants) manifest an evolving dysbiosis that impacts beneficial gut commensals. Administration of a fecal matter transplant of human-origin successfully prevented FA and suppressed established disease in food allergy (FA)-prone Il4raF709 (*Il4ra*^{F709}) mice. The fecal matter conferred protection by inducing ROR-γt+ Treg cells, which are deficient in FA subjects and FA-prone mice. These results thus identify a common mechanism by which commensals prevent FA.

Accordingly, one aspect provided herein is a method for treating or preventing the development of a food allergy in a subject comprising administering to a subject an agent that induces a regulatory T cell, or population thereof, that expresses RORγt.

In one embodiment of any aspect, the method further comprises, prior to administration, diagnosing a subject as having, or likely to develop, a food allergy.

In one embodiment of any aspect, the method further comprises, prior to administration, receiving the results of an assay that diagnoses a subject as having or is likely to develop a food allergy.

In one embodiment of any aspect, the subject is a newborn, an infant, a toddler, a child, or an adult. In one embodiment of any aspect, the subject is selected from the group consisting of a newborn, an infant, a toddler, a child, or an adult.

In one embodiment of any aspect, the agent is administered prior to the first exposure to a potential food allergen.

In another embodiment of any aspect, the agent is administered upon clinical signs of atopic symptoms.

In one embodiment of any aspect, the subject has been diagnosed with at least one food allergy. For example, the subject can have previously been diagnosed with having a soy allergy prior to being diagnosed as having a nut allergy.

In one embodiment of any aspect, the food allergy comprises allergy to soy, wheat, eggs, dairy, peanuts, tree nuts, shellfish, fish, mushrooms, stone fruits and other fruits.

In one embodiment of any aspect, the agent is selected from the group consisting of: a small molecule, a compound, an antibody, a peptide, and an expression vector encoding RORγt.

In one embodiment of any aspect, the vector is non-integrative or integrative.

In one embodiment of any aspect, the non-integrative vector is selected from the group consisting of an episomal vector, an EBNA1 vector, a minicircle vector, a non-integrative adenovirus, a non-integrative RNA, and a Sendai virus.

In one embodiment of any aspect, the vector is a lentivirus vector.

In one embodiment of any aspect, the compound is indoxyl 3 sulfate (I3S).

In one embodiment of any aspect, the compound is indole-3-carboxaldehyde (CA).

In one embodiment of any aspect, the agent prevents and/or reverses Th2 programming of Tregs and other mucosal T cell populations.

In one embodiment of any aspect, the regulatory T cell expressing RORγt has low expression of the Helios marker as compared to a regulatory T cell that does not express RORγt.

In one embodiment of any aspect, the expression of RORγt is increased by at least 2x, 3x, 4x, 5x, 6x, 7x, 8x, 9x, or more as compared to an appropriate control.

In one embodiment of any aspect, the agent upmodulates the expression of RORγt in a regulatory T cell, or population thereof.

Another aspect described herein provides a method for treating or preventing the onset of a food allergy in a subject comprising administering to a subject an agent that induces a regulatory T cell, or population thereof, that expresses RORγt, wherein the agent is I3S or CA.

Another aspect described herein provides a method for inducing tolerance in a subject, the method comprising: administering to a subject an agent that induces a regulatory T cell, or population thereof, that expresses RORγt, wherein the agent is I3S or CA.

Yet another aspect described herein provides a method for reducing or eliminating a subject's immune reaction to a food allergen comprising administering to a subject an agent that induces a regulatory T cell, or population thereof, that expresses RORγt, thereby reducing or eliminating a subject's immune reaction to a food allergen.

In another aspect, described herein is a method for treating or preventing the onset of a food allergy in a subject, the method comprising: (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (b) is statistically lower than the level in a healthy subject; and/or (d) if the subject has decreased RORγt in regulatory T cells, then administering an agent that induces regulatory T cells that express RORγt to the subject.

In some embodiments of any of the aspects, the method further comprises, prior to step (a), isolating regulatory T cells from the subject.

In another aspect, described herein is a method for treating or preventing the onset of a food allergy in a subject, the method comprising: (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (b) is statistically lower than the level in a healthy subject; (d) identifying whether the subject has, or is likely to develop, a food allergy; and (e) if the subject has decreased RORγt in regulatory T cells and has, or is likely to develop, a food allergy, then administering an agent that induces regulatory T cells that express RORγt to the subject.

In some embodiments of any of the aspects, the method further comprises, prior to step (a), isolating regulatory T cells from the subject.

In another aspect, described herein is a method for treating or preventing the onset of a food allergy in a subject, the method comprising: (a) receiving the results of an assay that determines if the subject has decreased levels of RORγt in regulatory T cells; and (b) if the subject has decreased RORγt in regulatory T cells, then administering an agent that induces regulatory T cells that express RORγt to the subject.

In another aspect, described herein is a method for treating or preventing the onset of a food allergy in a subject, the method comprising: (a) receiving the results of an assay that determines if the subject has decreased levels of RORγt in regulatory T cells; (b) receiving the results of an assay that determines if the subject has, or is likely to develop, a food allergy; and (c) if the subject has decreased RORγt in regulatory T cells and has, or is likely to develop, a food allergy, then administering an agent that induces regulatory T cells that express RORγt to the subject.

Another aspect described herein provides a composition comprising an agent that induces a regulatory T cell, or population thereof, that expresses RORγt.

In some embodiments of any of the aspects, the agent is selected from the group consisting of: a small molecule, a compound, an antibody, a peptide, and an expression vector encoding RORγt.

In some embodiments of any of the aspects, the compound is indoxyl 3 sulfate (I3S).

In some embodiments of any of the aspects, the compound is indole-3-carboxaldehyde (CA).

In one embodiment of any aspect, the composition further comprises a pharmaceutically acceptable carrier.

Another aspect described herein provides a use of any of the compositions described herein for the treatment or prevention of a food allergy in a subject having or likely to develop a food allergy.

Another aspect described herein provides a use of any of the compositions described herein for reducing or eliminating a subject's immune reaction to a food allergen.

Another aspect described herein provides a method of identifying a microorganism that induces a regulatory T cell, or a population thereof, that expresses RORγt, the method comprising (a) introducing at least one microorganism to a subject for a time sufficient to allow for the colonization the at least one microorganism in the gut of the subject; (b) measuring a level of expression of RORγt in regulatory T cells in the germ-free mouse model; and (c) identifying the at least one microorganism as an inducer of a regulatory T cell, or a population thereof, that expresses RORγt if the level of expression from step (b) is greater than zero.

In some embodiments of any of the aspects, the subject comprises a germ-free mouse model.

Yet another aspect described herein provides a method for treating or preventing the onset of a food allergy in a subject, the method comprising: (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (a) is statistically lower than the level in a healthy subject; and (d) if the subject has decreased RORγt in regulatory T cells, then administering a fecal matter transplant to the subject, wherein the fecal matter is obtained from a healthy subject.

In one embodiment of any aspect, the method further comprises prior to step (a), isolating regulatory T cells from the subject.

Finally, another aspect described herein provides a method for treating or preventing the onset of a food allergy in a subject, the method comprising: (a) receiving the results of an assay that determines if the subject has decreased levels of RORγt in regulatory T cells; and (b) if the subject has decreased RORγt in regulatory T cells, then administering a fecal matter transplant to the subject, wherein the fecal matter is obtained from a healthy subject.

### BRIEF DESCRIPTION OF THE DRAWINGS

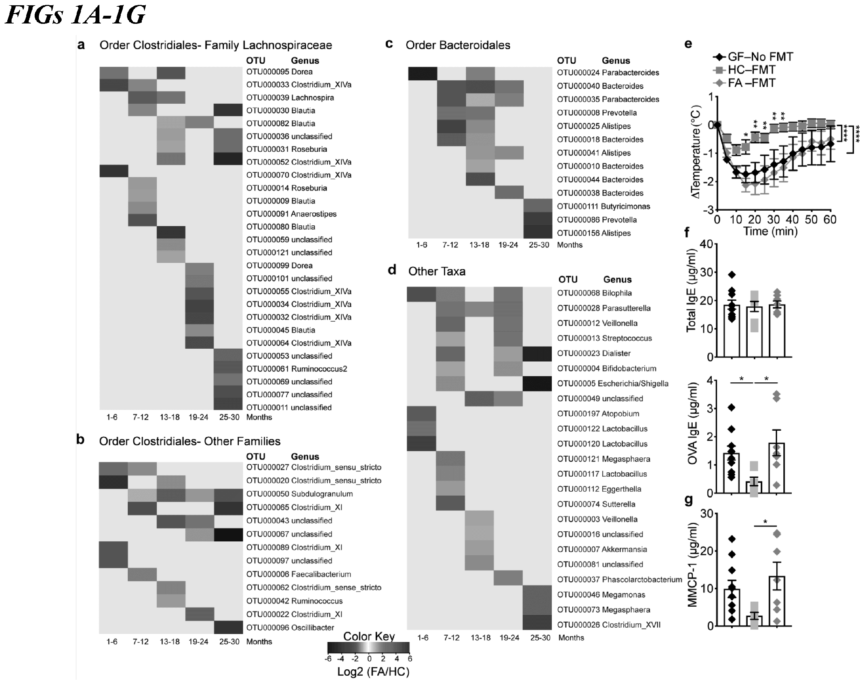

FIGs. 1A-1G show FA infants exhibit an evolving dysbiosis of their gut microbiota. Heat map representations of log2 fold relative abundances of fecal bacterial taxa between FA and control (C) infants displayed across the different age groups: 1-6, 7-12, 3-18, 19-24, and 25-30 months. For subject characteristics, see e.g., Table 1. Taxa represented included those from the order *Clostridiales*, family *Lachnospiraceae* (FIG. 1A), order *Clostridiales*, other families (FIG. 1B), order *Bacteroidales* (FIG. 1C) and other miscellaneous taxa (FIG. 1D). Dark grey represents higher abundance in control subjects, and grey represents higher abundance in FA subjects. Taxonomic information is on the right side of the respective panel. Analysis was carried out using the DESeq2 software package as described herein. To assess the functional significance of dysbiosis in FA, *Il4ra*^{F709} mice were employed. *Il4ra*^{F709} mice are genetically prone to develop FA upon oral sensitization with food allergens. Adult germ-free (GF) *Il4ra*^{F709} mice that were either left un-reconstituted or that received fecal matter transplants (FMT) from HC or FA infants were sensitized with chicken egg ovalbumin (OVA) in the presence of the mucosal adjuvant staphylococcal enterotoxin B (SEB) and subsequently challenged with OVA. GF *Il4ra*^{F709} mice or those that received FMT from FA subjects exhibited a rapid and sustained drop in their core body temperature, consistent with anaphylaxis, whereas those that received FMT from HC had a mild drop that rapidly reversed (FIG. 1E). Furthermore, and whereas the total serum IgE concentrations in the three OVA-sensitized mouse groups were similar, induction of OVA-specific IgE was markedly decreased in mice that received FMT from HC subjects as compared to those that received FMT from FA subjects or were GF (FIG. 1F). Also, the increase in serum mouse mast cell protease 1 (MMCP1) concentrations post anaphylaxis was notably higher in those *Il4ra*^{F709} mice that were GF or that have received FMT from FA subjects as compared to mice that received FMT from healthy subjects (FIG. 1G). These results indicated that the capacity of the gut commensal flora to impart protection against FA was profoundly impaired in FA as compared to HC subjects.

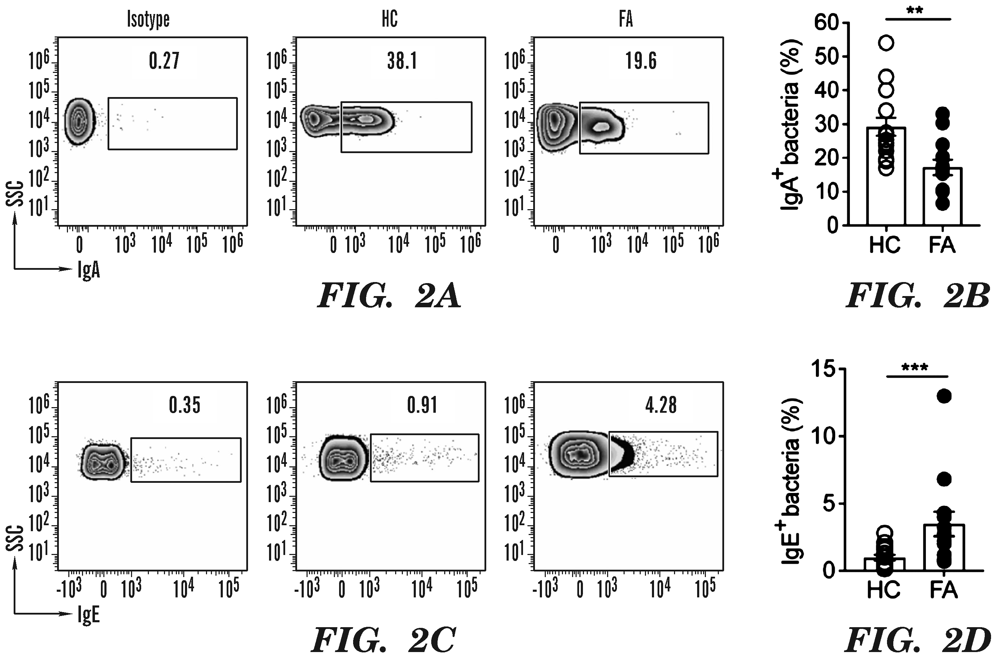

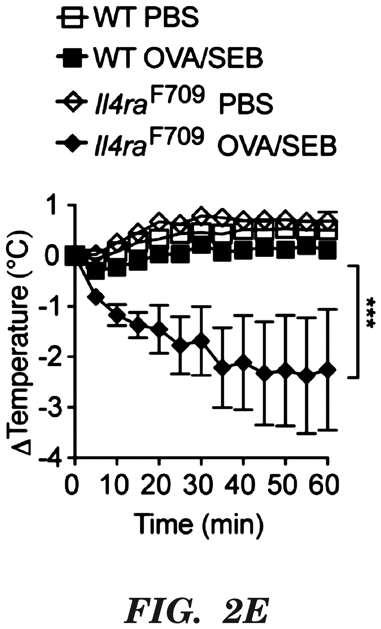

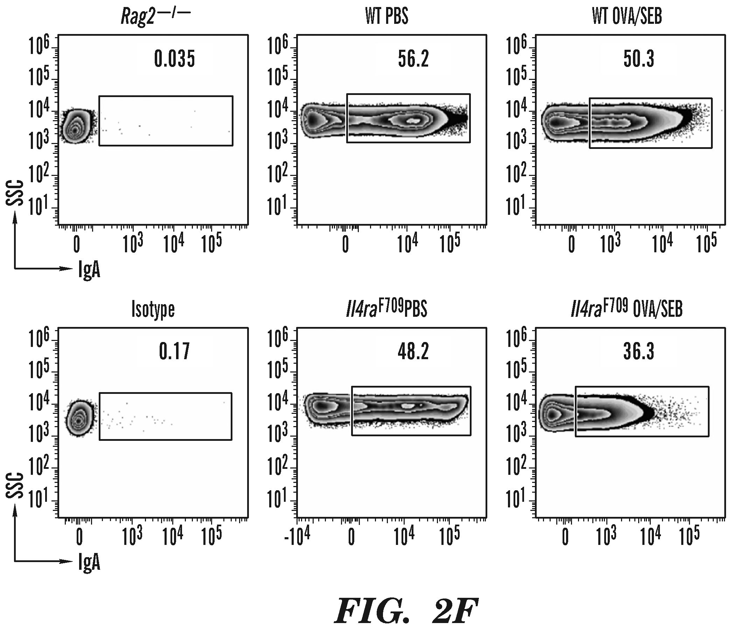

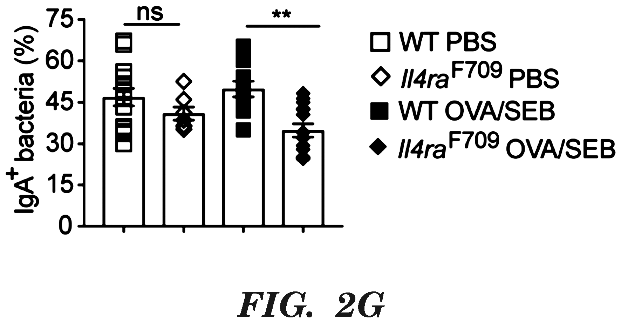

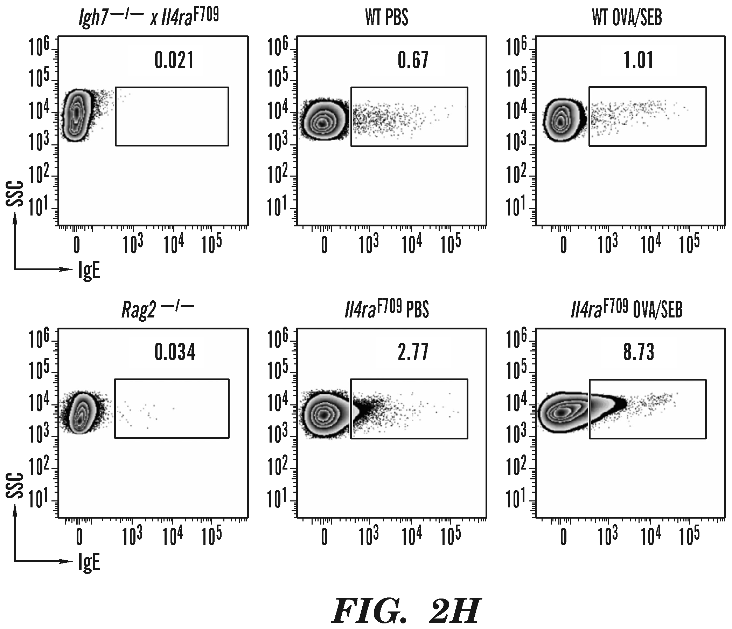

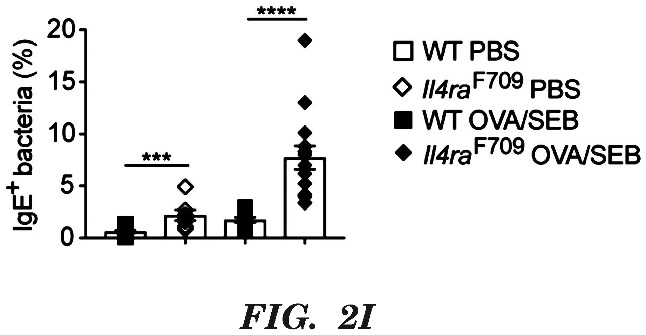

FIGs. 2A-2I show FA is associated with altered mucosal antibody responses to the gut commensal flora. (FIGs 2A-2D) Flow cytometric analysis and frequencies of human fecal bacteria of FA subjects and healthy control (HC) subjects stained with a PE-conjugated isotype control mAb or phycoerythrin (PE)-conjugated mouse anti-human IgA (FIGs 2A and 2B) or IgE mAb (FIGs 2C and 2D). Each symbol represents a result from one subject. N= 15 subjects for the HC and 13 for the FA group. Core body temperature changes (FIG. 2E) in WT and *Il4ra*^{F709} mice that have been either shame sensitized (PBS) or sensitized with OVA/SEB, as indicated, and challenged with OVA. Flow cytometric analysis and frequencies of IgA (FIGs 2F and 2G) and IgE (FIGs 2H and 2I) staining of fecal bacteria of WT and *Il4ra*^{F709} mice that were either sham sensitized (PBS) or sensitized with OVA/SEB (as shown in FIG. 2E). Staining was carried out with PE-conjugated rat isotype control mAb or rat anti-mouse IgA or IgE mAb, as indicated. Fecal pellets of Rag2-deficient (Rag2-/-) mice and IgE-deficient *Il4ra*^{F709} (*Il4ra*^{F709}Igh7-/-) mice were used as negative controls for sIgA and IgE staining, respectively. Each symbol represents one mouse. N= 7-14 mice/group. (FIGs 2B and 2D) **P< 0.01, ***P< 0.001 by Student's unpaired two tailed t test. (FIGs 2G and 2I) *P<0.05, **P< 0.01, ***P< 0.001 by one-way analysis of variance (ANOVA) with Dunnett post hoc analysis. For core body temperature measurements ***P < 0.001 by repeat measures two-way ANOVA.

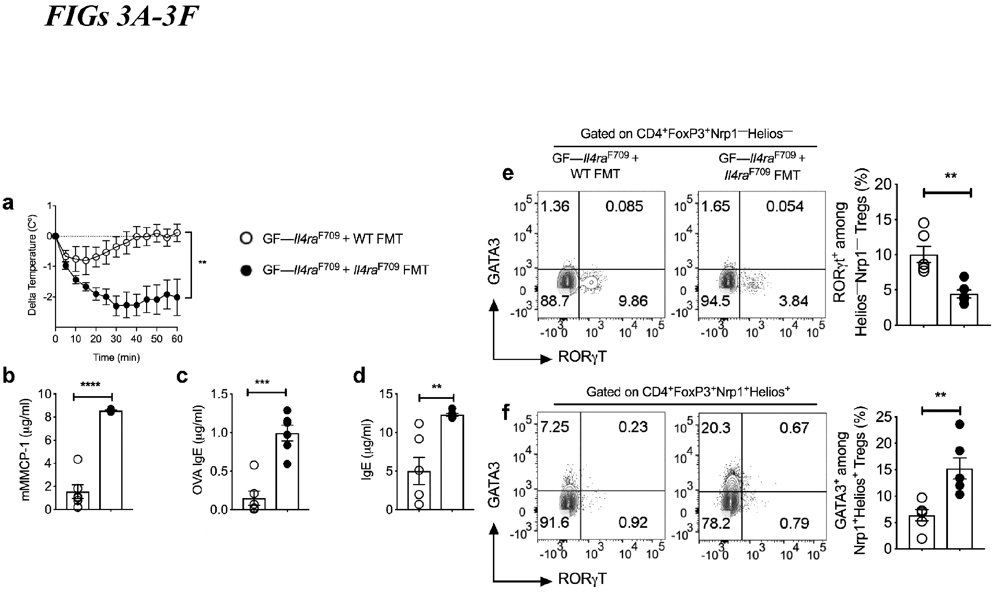

FIGS. 3A-3F show that similar to FA human subjects, *Il4ra*^{F709} mice also exhibit dysbiotic microbiota which, upon transfer to germ free BALB/c mice heightens their susceptibility to food allergy induction. The capacity of microbiota derived from specific pathogen-free (SPF) WT BALB/c mice were analyzed, which are relatively resistant to FA induction, to rescue the FA phenotype of *Il4ra*^{F709} mice. GF *Il4ra*^{F709} mice that were reconstituted with FMT from WT BALB/c mice then sensitized with OVA/SEB and challenged with OVA were resistant to the induction of FA, while those that were reconstituted with microbiota derived from SPF *Il4ra*^{F709} mice developed disease, as evidenced by a precipitous drop in core body temperature (FIG. 3A) and increased serum MMCP1 concentrations upon oral challenge with OVA (FIG. 3B), with increased total and OVA-specific serum IgE concentrations (FIG. 3C). These results indicated that dysbiosis is also an essential pathogenic attribute of FA in the *Il4ra*^{F709} mice, and that the promotion by dysbiosis of FA in both the human subjects and mouse model can proceed by common mechanisms. The expression of GATA3 and RORγT was also analyzed by flow cytometry in CD4⁺FoxP3⁺Nrp1⁻Helios⁻ and CD4⁺FoxP3⁺Nrp1⁻Helios⁺ gut T reg cells (FIG. 3E-3F).

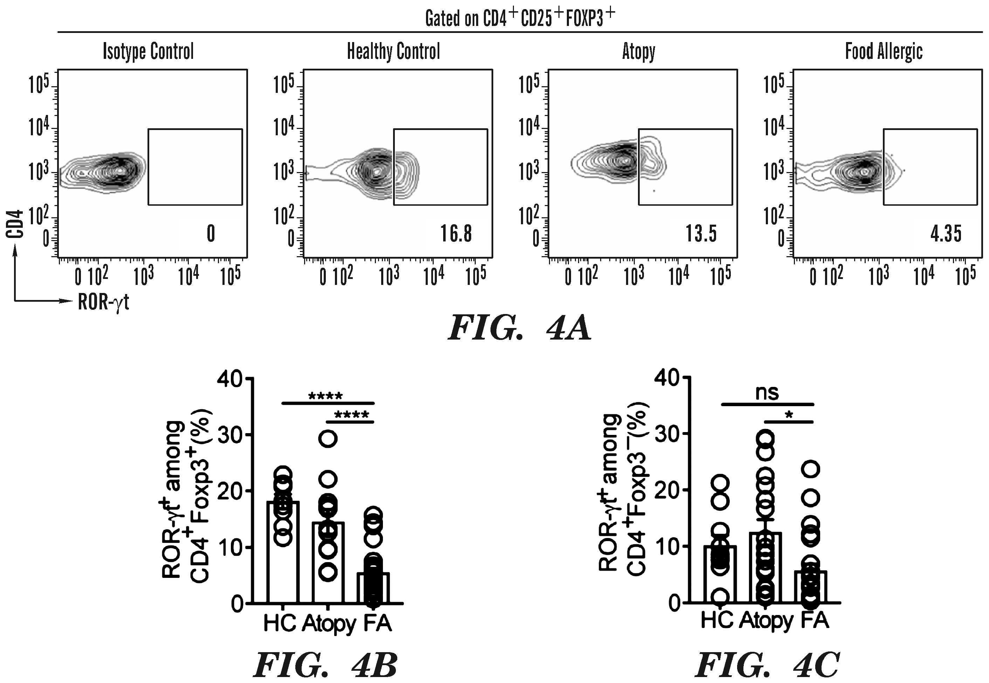

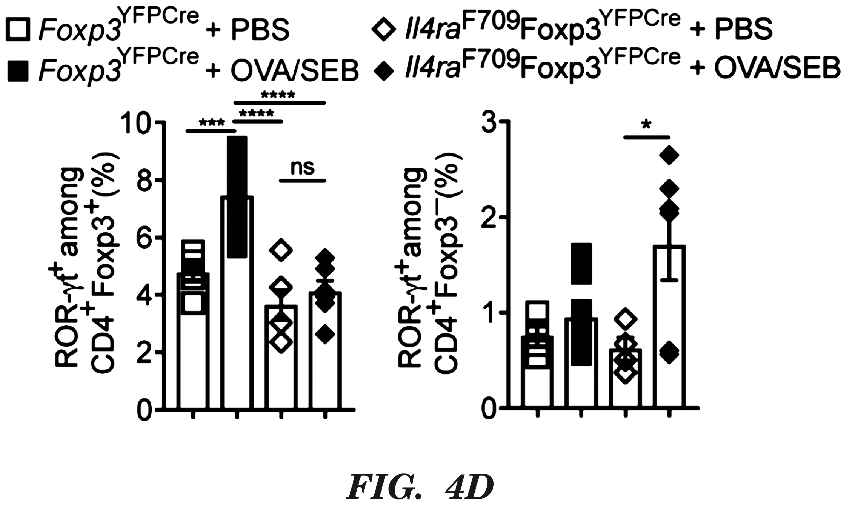

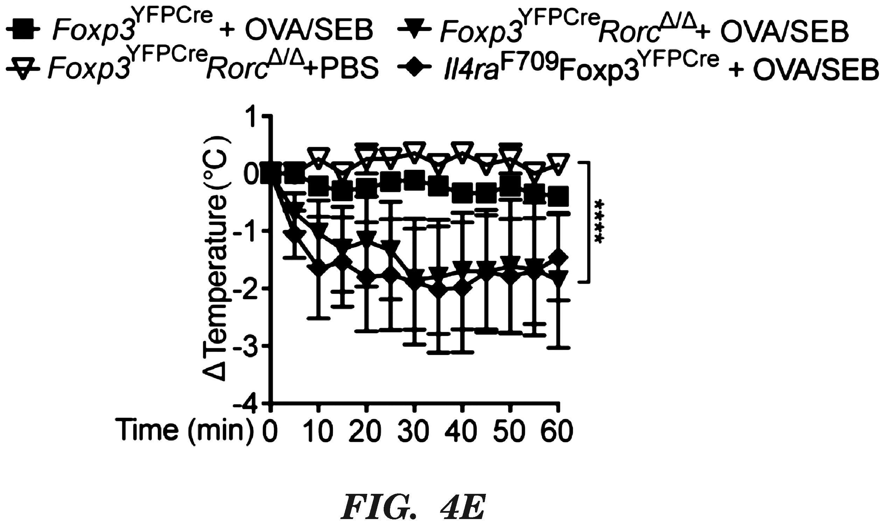

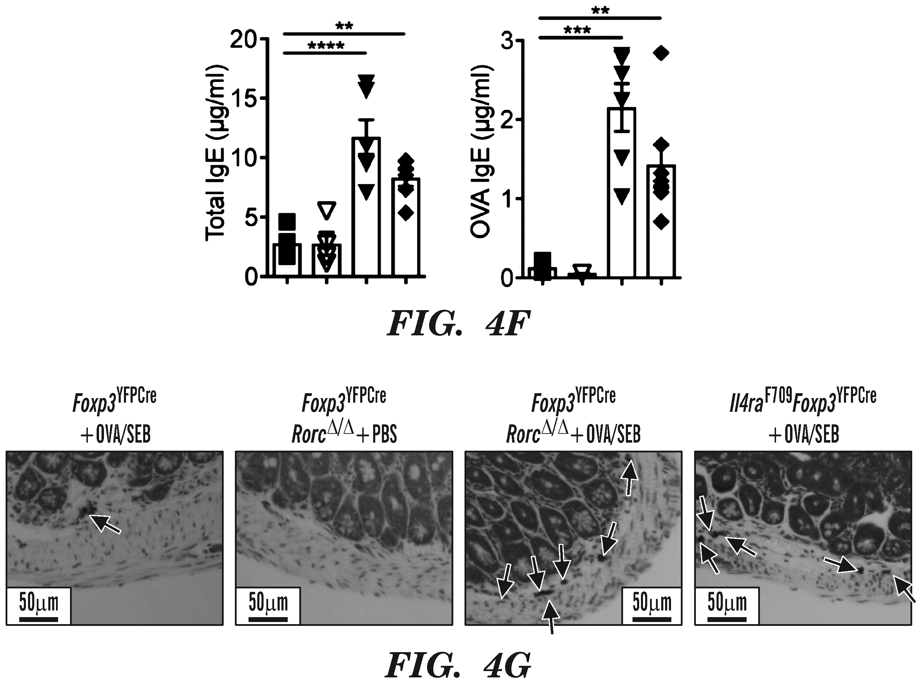

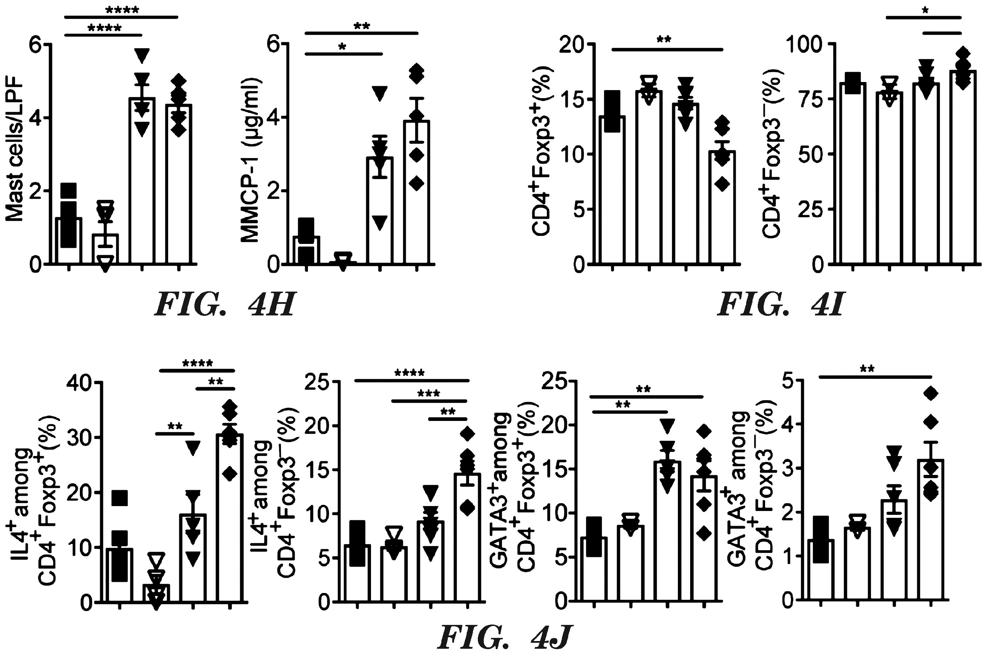

FIGs. 4A-4J show that ROR-γt+ Treg cell deficiency promotes FA. (FIG. 4A) Flow cytometric analysis and frequencies of circulating ROR-γt+Foxp3+ Treg cells from FA, atopic (atopy) and healthy controls (HC) subjects n=9-28 subjects/group. For subject characteristics, see e.g., Table 2. (FIG. 4B and 4C) Frequencies of circulating ROR-γt+Foxp3+ and ROR-γt+Foxp3- - T cells in the respective group. (FIG. 4D) Frequencies of MLN ROR-γγt+Foxp3+ and ROR-γt+Foxp3-- T cells in Foxp3YFPCre and Il4raF709Foxp3YFPCre mice that were sham (PBS) or OVA/SEB-sensitized, as indicated, then challenged with OVA n=5-9 mice/group. (FIG. 4E) Core body temperature changes in Foxp3YFPCre, Foxp3YFPCreRorcΔ/Δ and Il4raF709Foxp3YFPCre mice that were sensitized as indicated and challenged with OVA. (FIG. 4F) Total and OVA-specific IgE responses. (FIG. 4G and 4H) Jejunal mast cells (arrows). (FIG. 4G), mast cell numbers/low power field and serum MMCP1 concentrations (FIG. 4H) post OVA challenge in the indicated mouse groups. (FIG. 4I-4J) Frequencies of CD4+Foxp3+ and CD4+Foxp3- T cells (FIG. 4I), IL-4+Foxp3+ and IL-4+Foxp3- T cells, GATA3+Foxp3+ and GATA3+Foxp3- T cells (FIG. 4J) in MLN of the indicated mouse groups. N=5-8 mice/group. *p<0.05, **p<0.01, ***p<0.001, ****p<0.0001 by one-way ANOVA with Dunnett post hoc analysis. For core body temperature measurements ***P < 0.0001 by repeat measures two-way ANOVA.

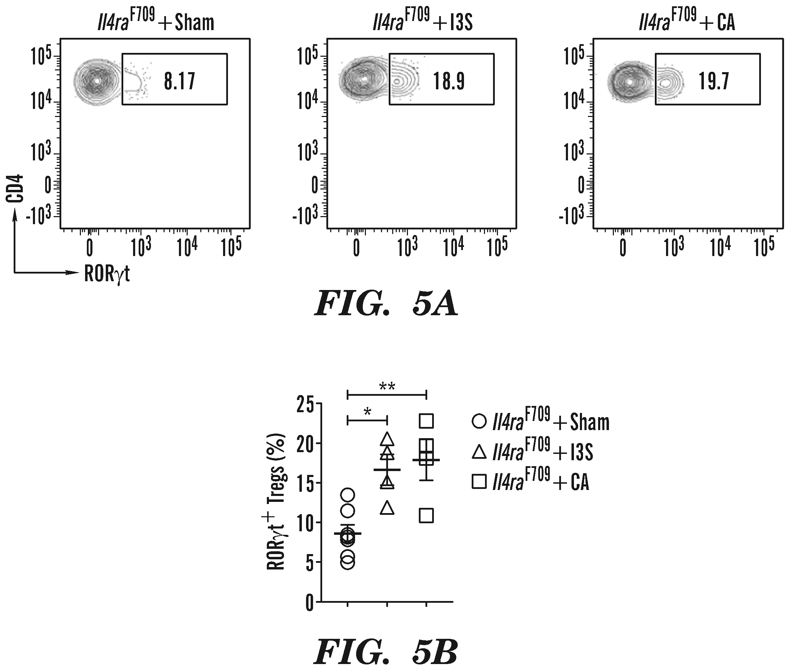

FIGs. 5A-5B show induction of ROR-γt Treg cells by bacterial metabolites. (FIG. 5A) Representative flow cytometric analysis of ROR-γt⁺ staining in CD4⁺Foxp3⁺ Treg cells in mice treated with indoxyl-3-sulfate (I3S), at 100 mg/kg in PBS given intraperitoneally once weekly, while being orally sensitized with OVA/SEB once weekly for 8 weeks; or mice treated with indol-3-carboxaldehyde (CA), at 100 mg/kg in PBS given orally once weekly, while being orally sensitized with OVA/SEB once weekly for 8 weeks. (FIG. 5B) scatter plot analysis of the staining results shown in (FIG. 5A). Each point represents one mouse. *P<0.05, **P< 0.01, by one-way ANOVA with Tukey post-test analysis.

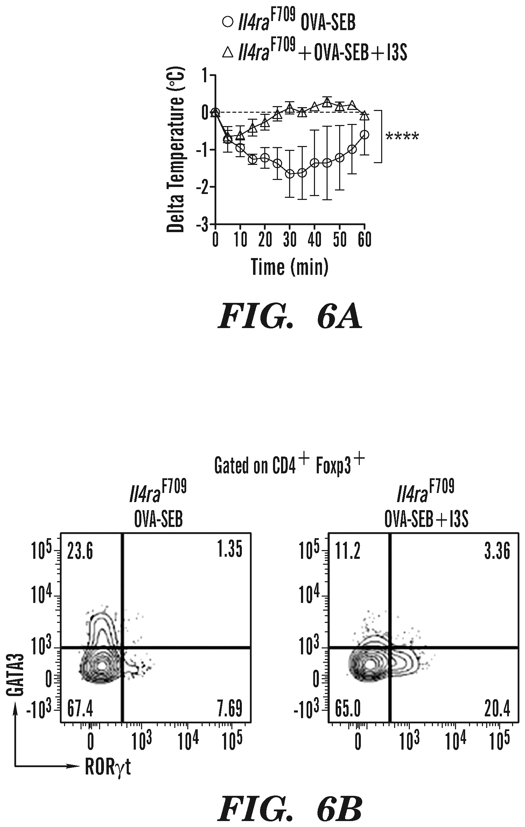

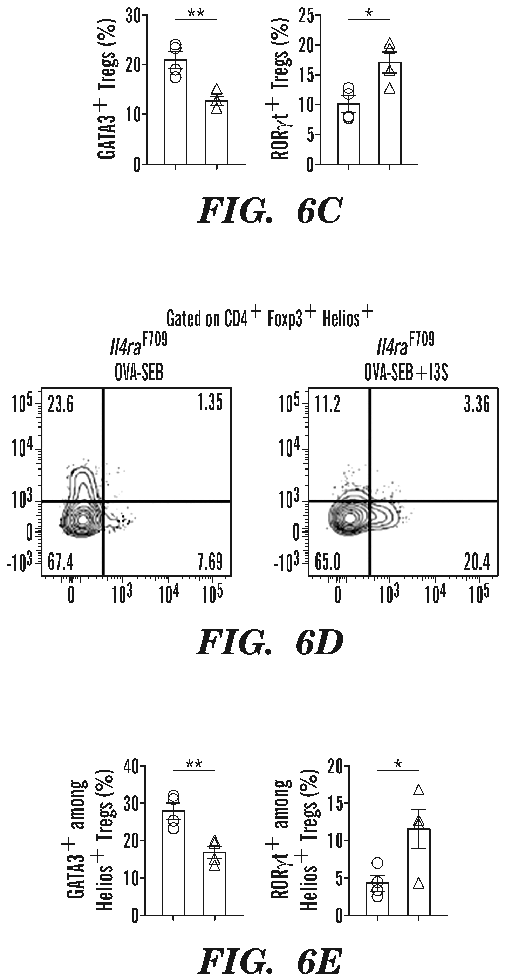

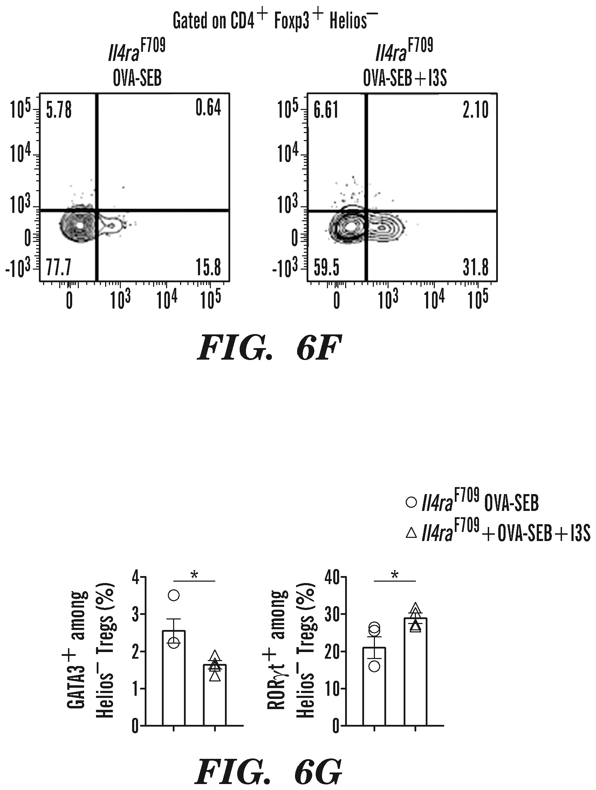

FIGs. 6A-6G show that Indoxyl 3 Sulfate (I3S) expands gut ROR-γt⁺ Treg cells and protects against food allergy in *Il4ra*^{F709} mice. (FIG. 6A), Core body temperature changes in *Il4ra*^{F709} mice that were either sham treated (PBS) or treated with I3S (at 100 mg/kg in PBS given intraperitoneally once weekly) while being orally sensitized with OVA/SEB once weekly for 8 weeks, then challenged with OVA. (FIG. 6B-6C) Representative flow cytometric analysis (FIG. 6B) and scatter plot representation (FIG. 6C) of GATA3⁺ and ROR-γt⁺ staining in total gut Treg cells. (FIG. 6D-6E), Representative flow cytometric analysis (FIG. 6D) and scatter plot representation (FIG. 6E) of Helios+ Treg cells. (FIG. 6F-6G) Representative flow cytometric analysis (FIG. 6F) and scatter plot representation (FIG. 6G) of Helios-Treg cells. N=4 mice/group. Each point in FIG. 6C, FIG. 6E, and FIG. 6G represents one mouse. *P<0.05, **P< 0.01, by Student's unpaired two tailed *t* test. For core body temperature measurements *****P* < 0.0001 by repeat measures two-way ANOVA.

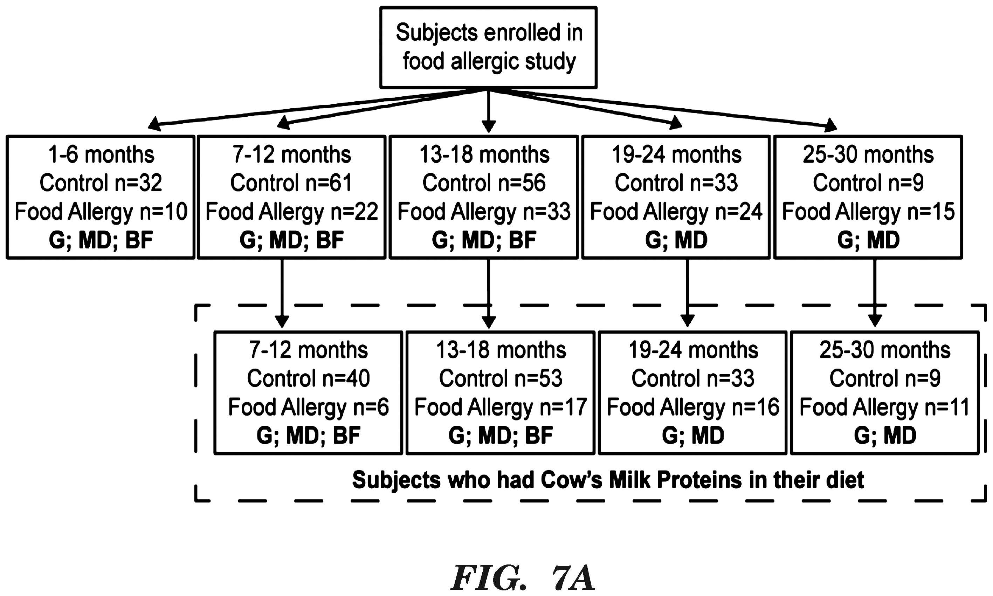

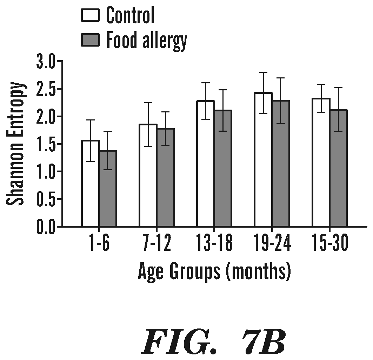

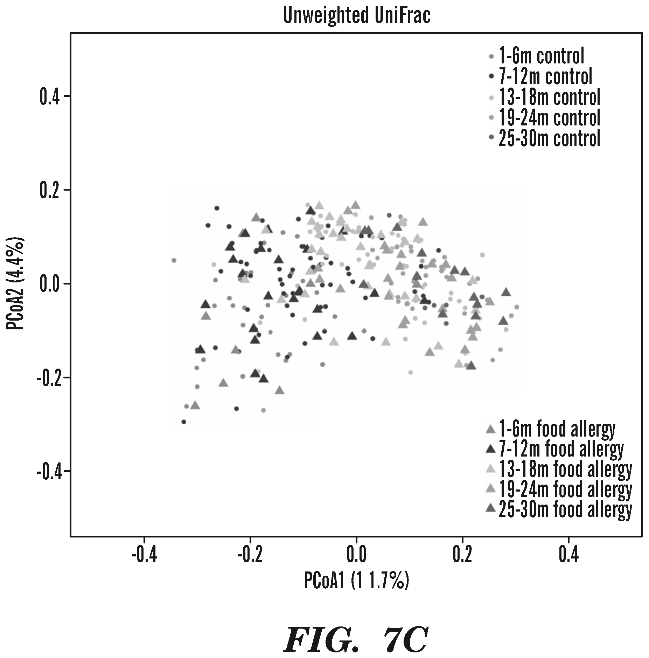

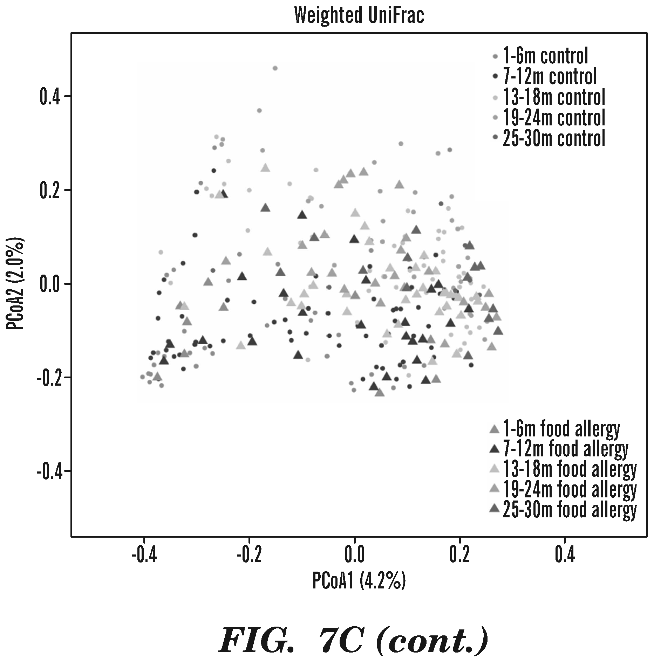

FIGs. 7A-7C show microbiota analysis in FA and healthy control infants. (FIG. 7A) Design of analyses of demographic variables of human FA and control subject groups. FA and control subjects were stratified into 5 age groups spaced at 6-month age intervals starting at age 1-6 months. Analyses were carried out using DESeq2, and were controlled for variables including gender (G), mode of delivery (MD) and breast feeding (BF). A subgroup analysis was also performed on subjects consuming cow's milk proteins. (FIG. 7B) Alpha diversity of gut microbiome of all age groups. Alpha diversity values were calculated using Shannon entropy to measure diversity in each sample. Bars represents average Shannon entropy values, and error bars represents S.E.M. Open bars represents control subjects, and gray represents FA subjects. (FIG. 7C) Beta diversity of gut microbiome in all subjects. Beta-diversity values were calculated using the unweighted/weighted Unifrac dissimilarity measures, to assess differences in overall microbial community structure. Overall community structure differed significantly among age groups (adjusted P<0.001), but not between control and FA subjects, using Analysis of Molecular Variance for statistical hypothesis testing.

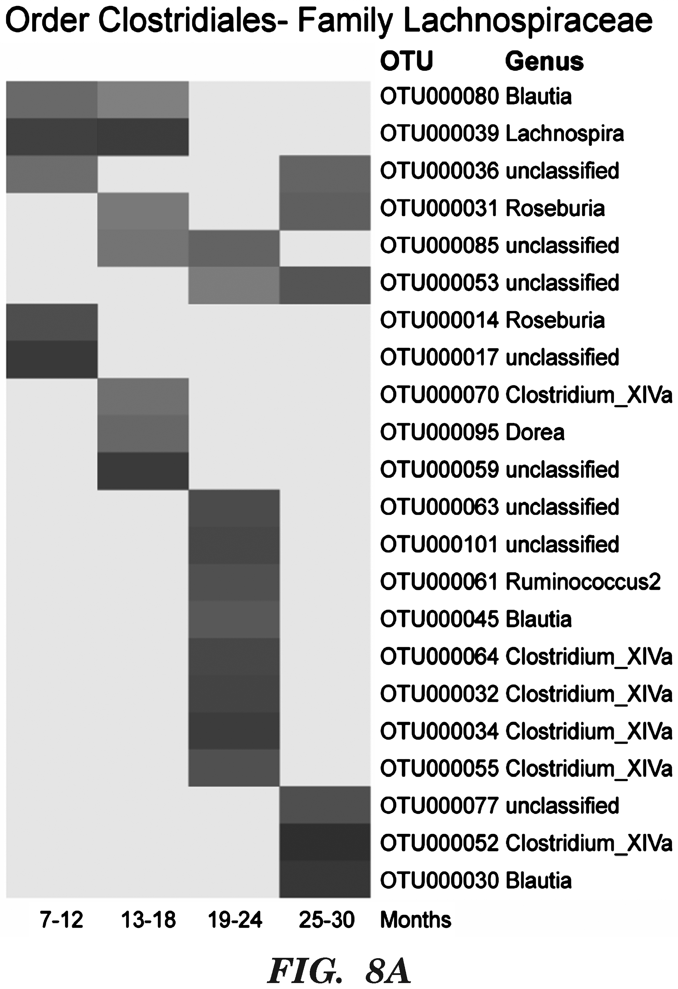

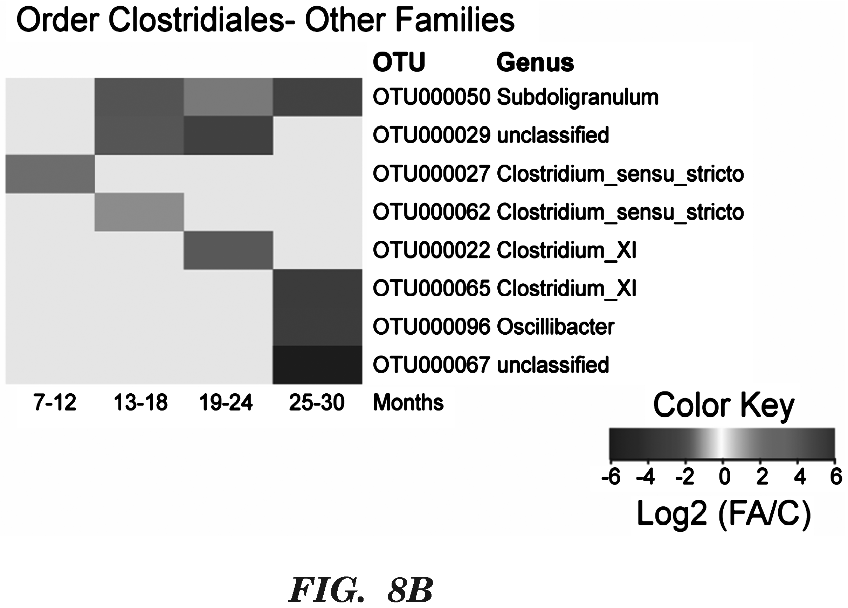

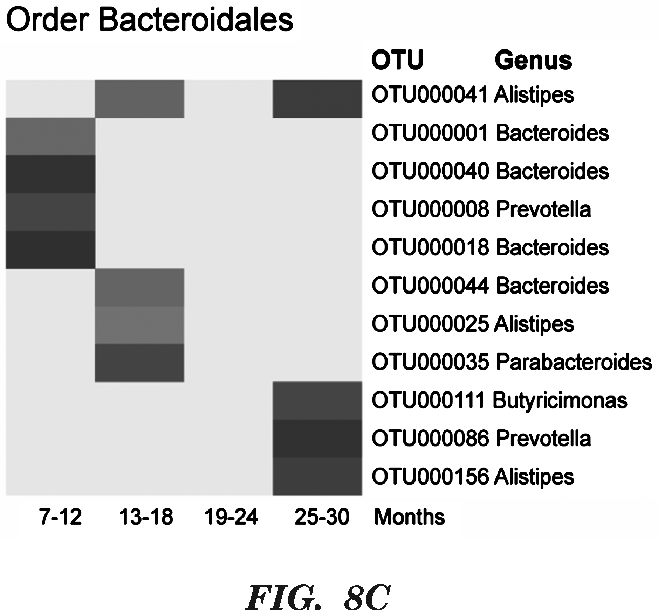

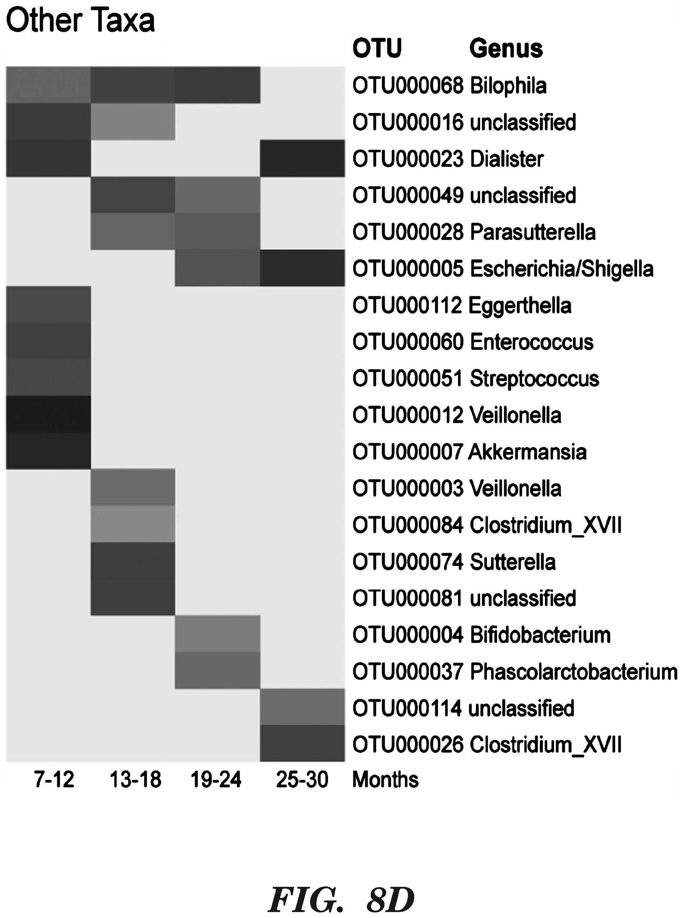

FIGs. 8A-8D show FA infants tolerant to cow milk protein (CMP) exhibit dysbiosis. Heat map representations of log2 fold relative abundances of fecal bacterial taxa between FA (milk tolerant) and control (C) infants displayed across the different age groups: 7-12, 3-18, 19-24, and 25-30 months. All the infants in the 1-6 month group were milk-allergic and were accordingly excluded from the analysis. Taxa represented included those from the order *Clostridiales*, family *Lachnospiraceae* (FIG. 8A), order *Clostridiales*, other families (FIG. 8B), order *Bacteroidales* (FIG. 8C) and other miscellaneous taxa (FIG. 8D). Dark grey represents higher abundance in control subjects, and grey represents higher abundance in FA subjects. Taxonomic information is on the right side of the respective panel. Analysis was carried out using the DESeq2 software package as described herein.

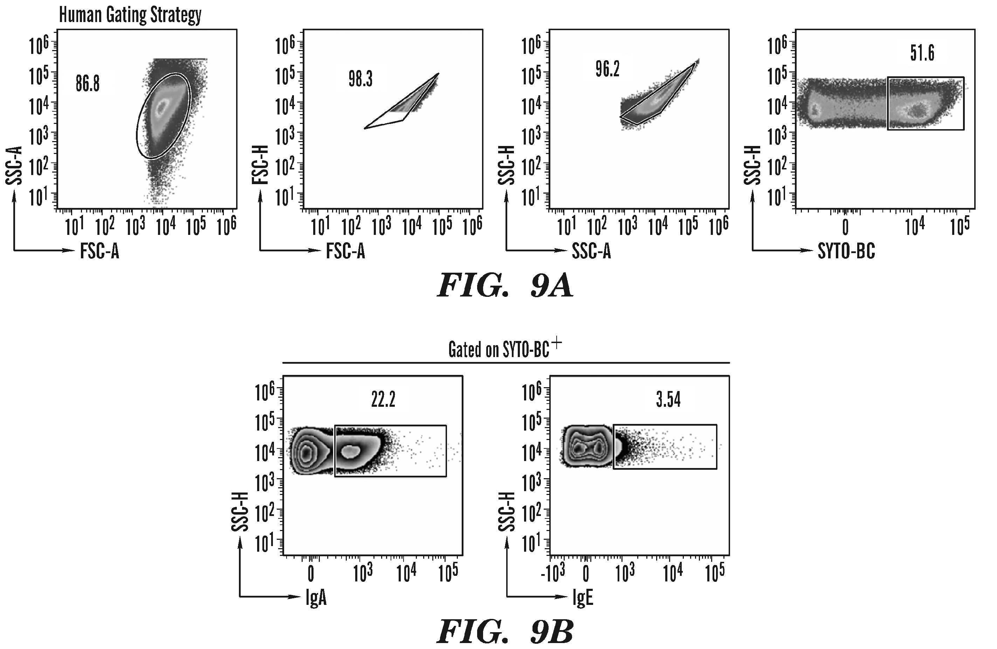

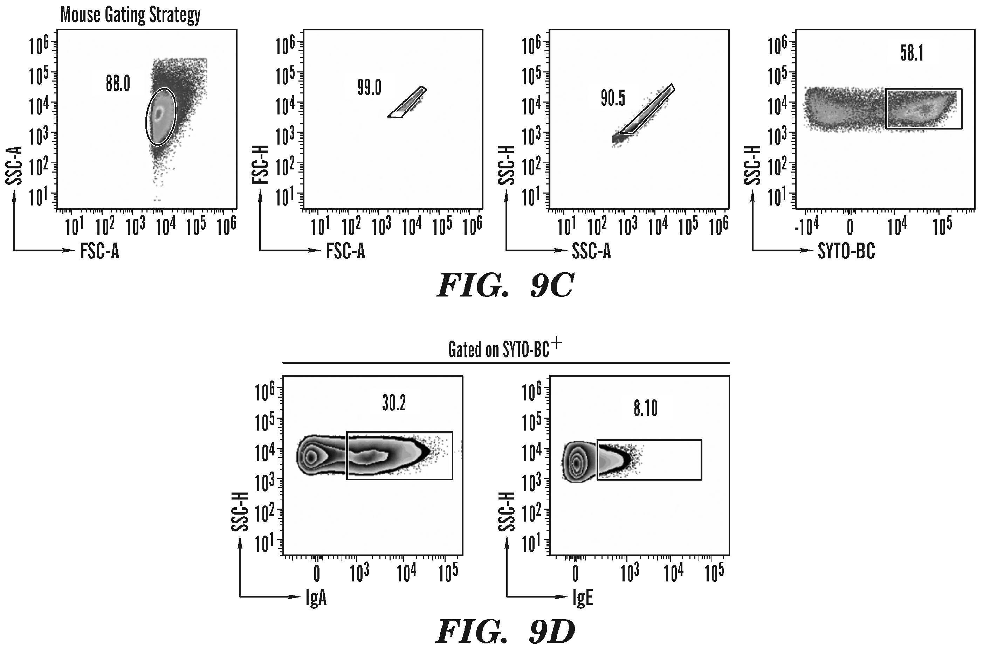

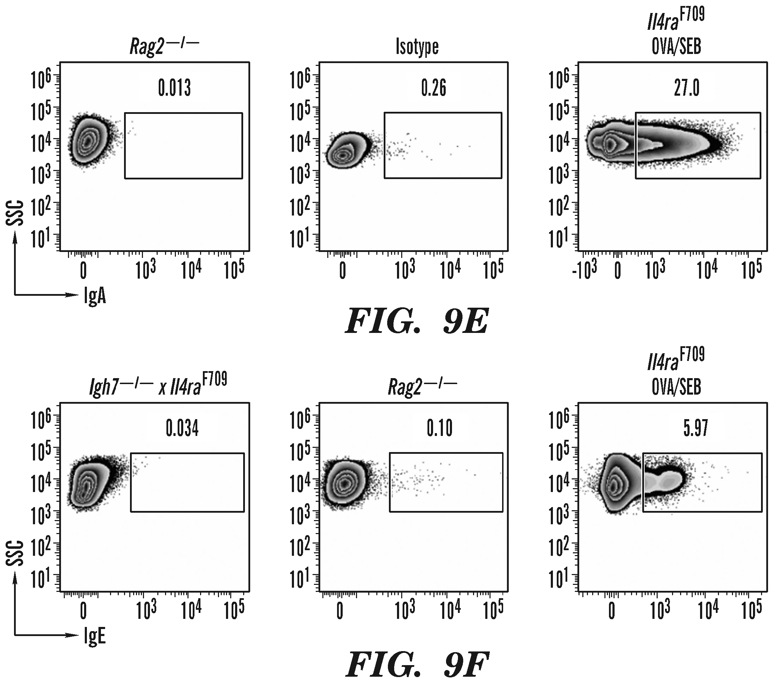

FIGs. 9A-9F shows gating strategy for analyzing IgA and IgE bound bacteria in human and mouse fecal samples. (FIG. 9A and 9C) Representative FACS plots showing the gating strategy for human (FIG. 9A) and mouse (FIG. 9C) fecal bacteria. IgA- and IgE-bound fecal bacteria were analyzed by first gating on forward versus side scatter area (FSC-A versus SSC-A) on a log-log scale (Far left panels). Doublets were discriminated by gating on the forward scatter height (FSC-H) versus FSC-A and subsequently gating on SSC-H versus SSC-A (Second and third panels from the left, respectively). Bacteria present in the feces was further identified by gating on SYTO-BC+ events (right side panels). (FIG. 9B and 9D) frequencies of IgA- and IgE-bound bacteria as assessed by gating on bacteria-bound with the respective PE-labelled anti-IgA and anti-IgE antibodies, as shown in panels FIG. 9A and FIG. 9C. (FIG. 9E and 9F). Flow cytometric analysis and frequencies of sIgA+ (FIG. 9E) and IgE+ (FIG. 9F) fecal bacteria of *Il4ra*^{F709} mice sensitized with OVA/SEB. Fecal pellets of Rag2-/- and Igh7-/- *Il4ra*^{F709} mice were used as negative controls for the respective antibody staining.

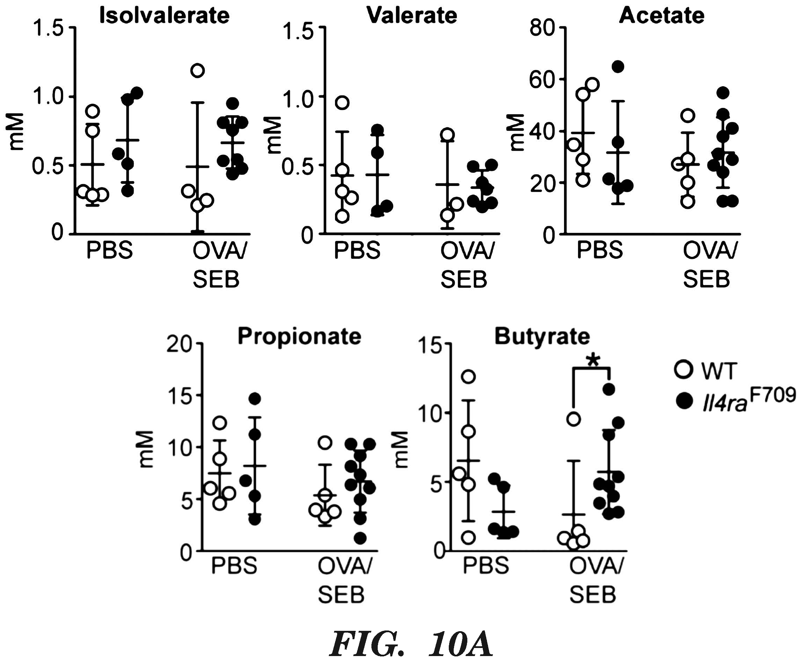

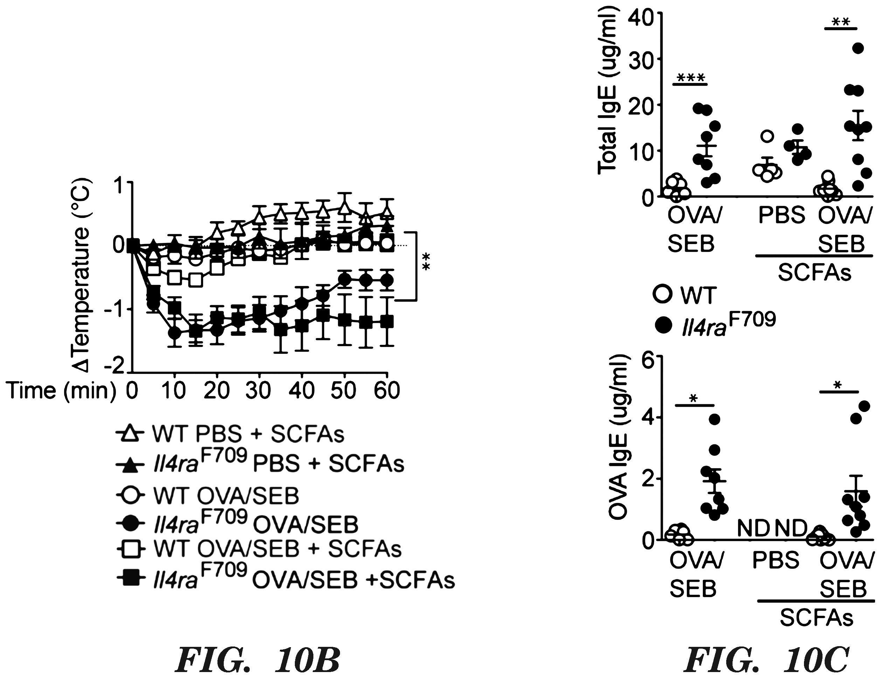

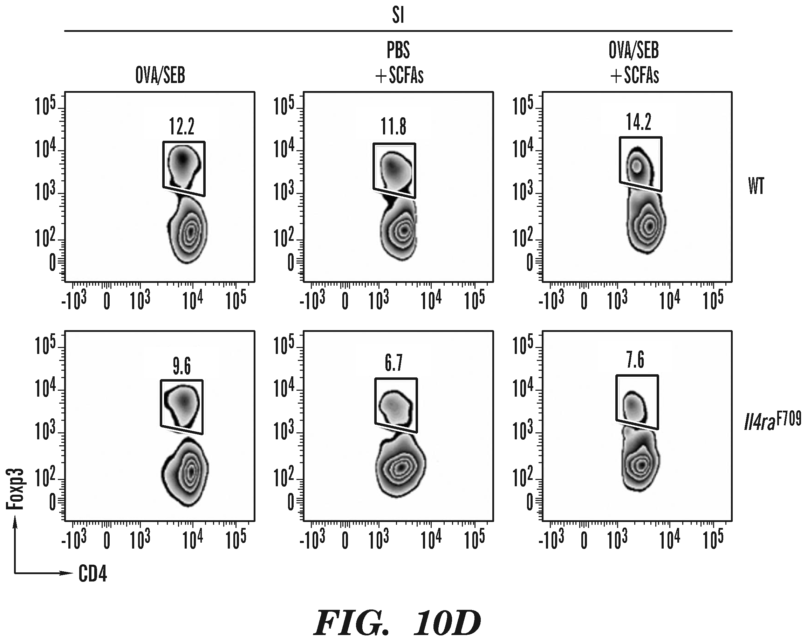

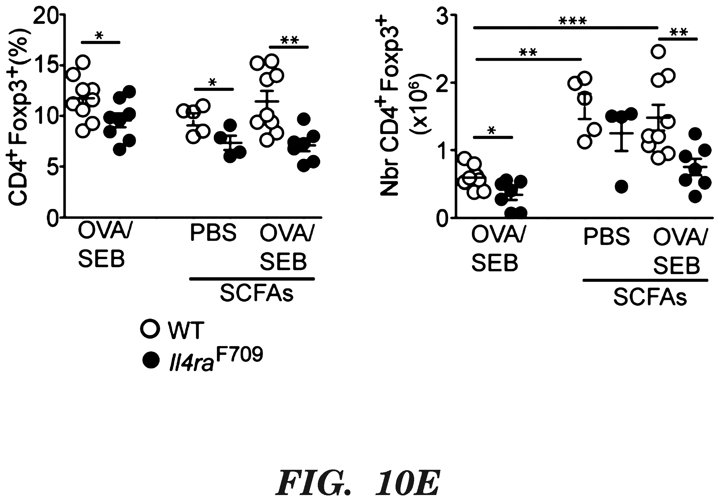

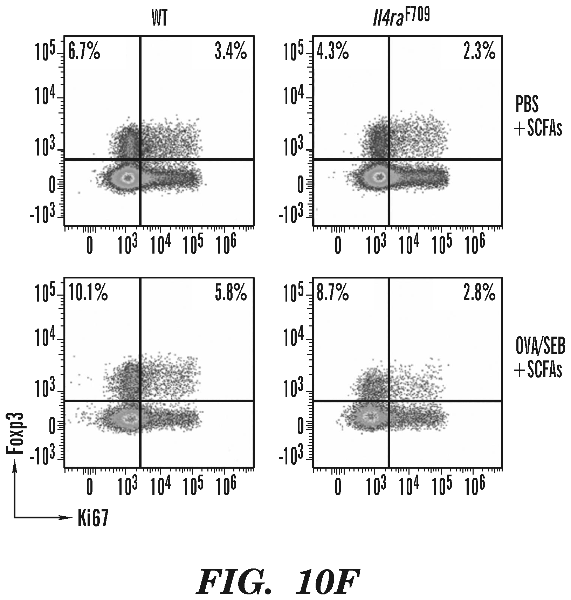

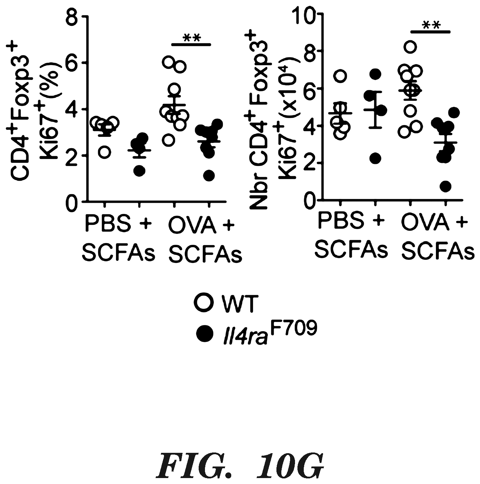

FIGs. 10A-10G show oral SCFA supplementation does not protect against FA. (FIG. 10A) Concentrations of the short chain fatty acids isovalerate, valerate, acetate, propionate and butyrate in fecal pellet samples of WT and *Il4ra*^{F709} mice that were either sham (PBS) sensitized or sensitized with OVA/SEB. N=3-10 mice per group. *p<0.05 by Student's unpaired two tailed t test. (FIG. 10B) Core body temperature changes in WT and *Il4ra*^{F709} mice that were kept without or with oral supplementation with short chain fatty acids (SCFAs) in their drinking water while being either sham- (PBS) or OVA/SEB-sensitized, as indicated, then challenged with OVA. (FIG. 10C) Total and OVA-specific serum IgE antibody concentrations. (FIG. 10D) Representative flow plots of CD4+Foxp3+ T cells in WT and *Il4ra*^{F709} mice treated with SCFAs. (FIG. 10E) Frequencies and numbers of small intestine (SI) CD4+Foxp3+ T cells. (FIG. 10F) Representative flow plots of KI67 expression on CD4+Foxp3+ T cells in WT and *Il4ra*^{F709} mice treated with SCFAs. (FIG. 10G) Frequencies and numbers of KI67+ expressing small intestinal (SI) CD4+Foxp3+ T cells. N=5-8 mice/group. ***P< 0.001, ****P< 0.0001 by one-way ANOVA with Dunnett post hoc analysis. For core body temperature measurements **P < 0.01 by repeat measures two-way ANOVA.

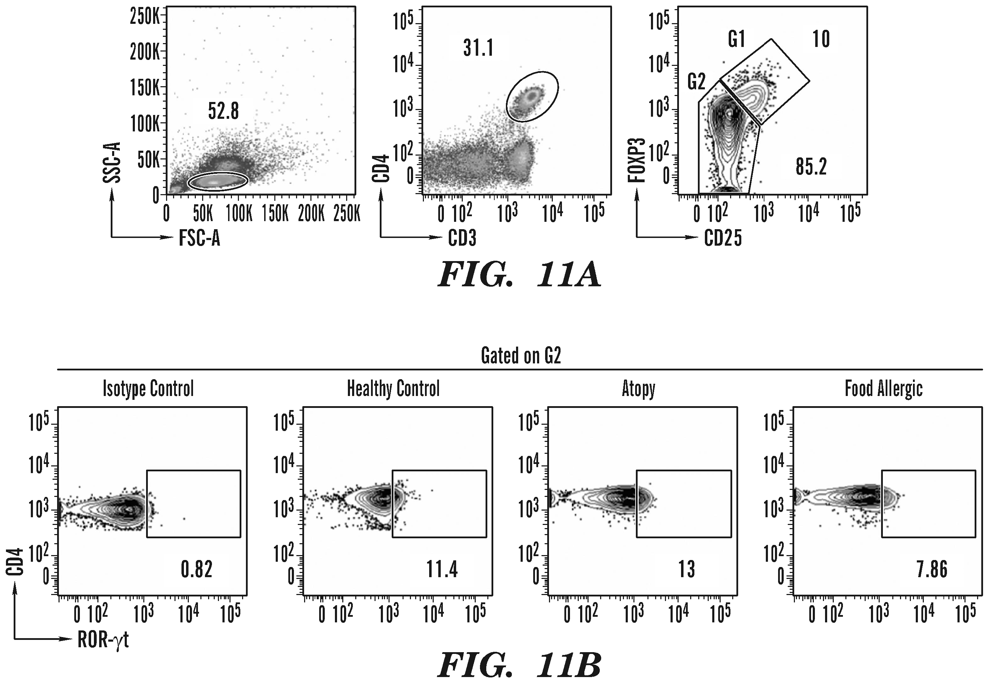

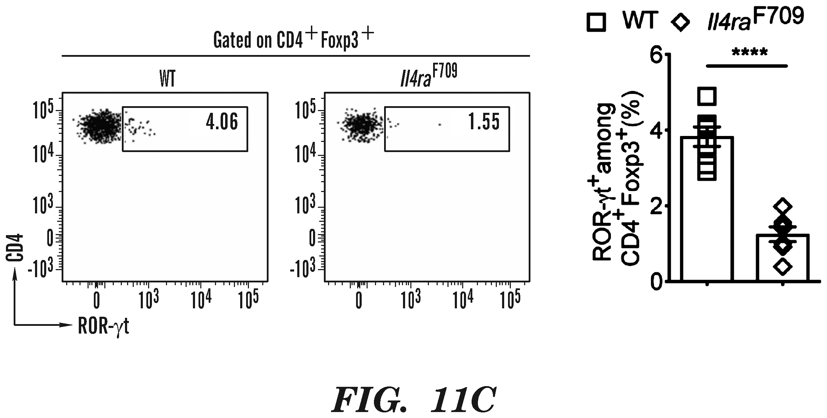

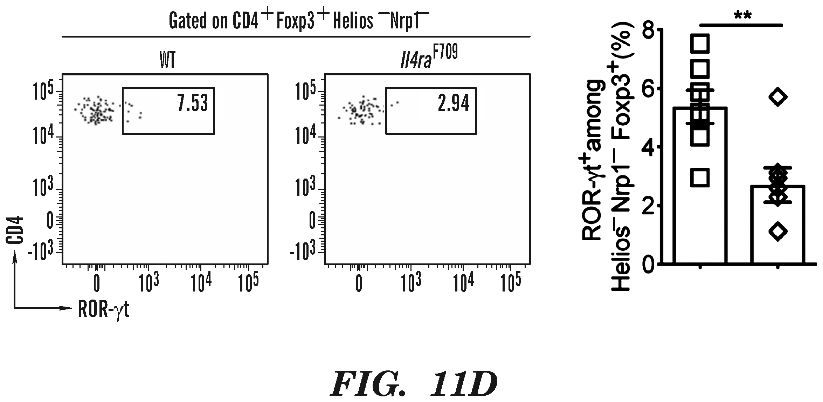

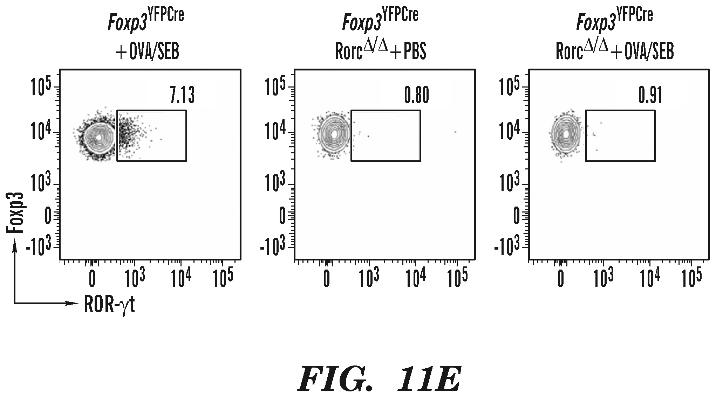

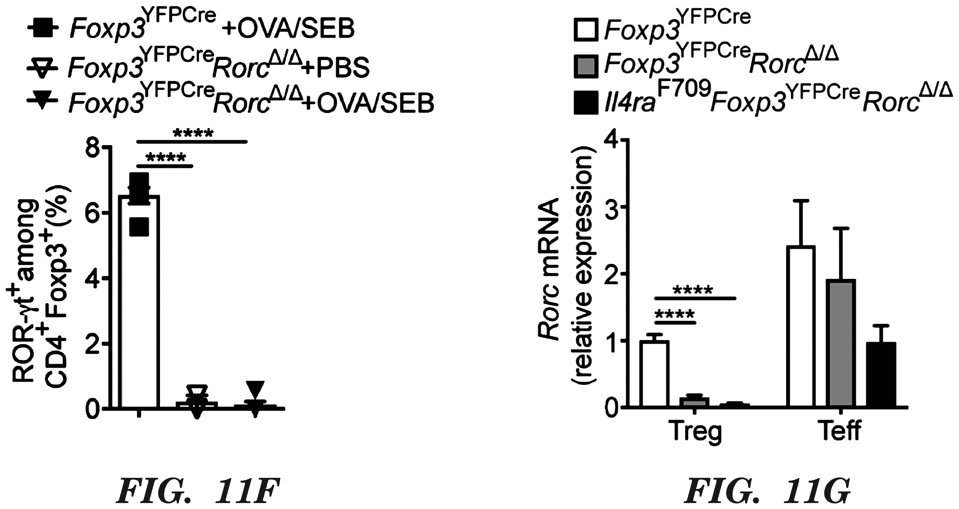

FIGs. 11A-11G show analysis of ROR-γt+ expression in human subjects and mutant mice. (FIG. 11A) Gating strategy for CD4+Foxp3+ (G1) and CD4+Foxp3- T (G2) cells ex vivo. (FIG. 11B) Gating strategy for the expression of ROR-γt in Teff cells (G2) from FA patients, healthy controls (HC) and atopic subjects (atopy), as compared to an isotype control. (FIG. 11C and 11D) Representative flow plots for the expression of ROR-γt in peripheral blood CD4+Foxp3+ total regulatory T cells and CD4+Foxp3+Helios-NRP1- induced regulatory T cells from wild-type (WT) BLAB/c Il4raF709 mice. (FIG. 11E and 11F) Representative flow plots and frequencies of ROR-γt+CD4+Foxp3+ regulatory T cells in mesenteric lymph nodes of Foxp3YFPCre mice sensitized with OVA/SEB, and Foxp3YFPCreRorcΔ/Δ either sham sensitized (PBS) or sensitized with OVA/SEB, as indicated, and challenged with OVA. (FIG. 11G) Quantitative PCR for Rorc gene expression in MLN CD4+Foxp3+ Treg and CD4+Foxp3-Teff cells from Foxp3YFPCre, Foxp3YFPCreRorcΔ/Δ, and Il4raF709Foxp3YFPCreRorcΔ/Δ mice. Data were normalized to the endogenous Hprt transcripts. Results represent Means ± S.E.M. collated from 2 independent experiments, N=5 individual mice/group. (FIG. 11D and 11E) ****p<0.0001 by one-way ANOVA with Dunnett post hoc analysis.

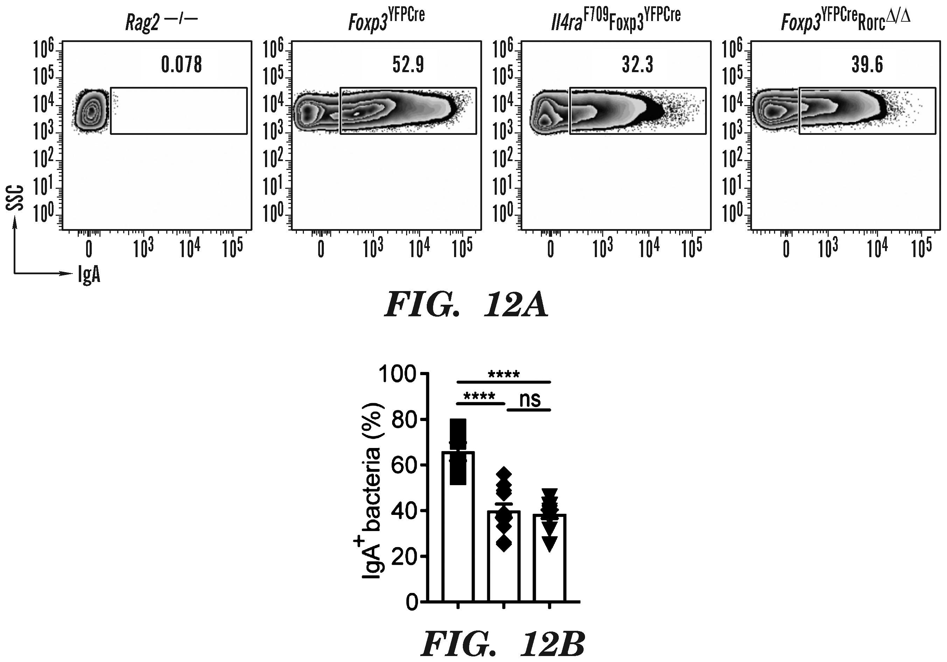

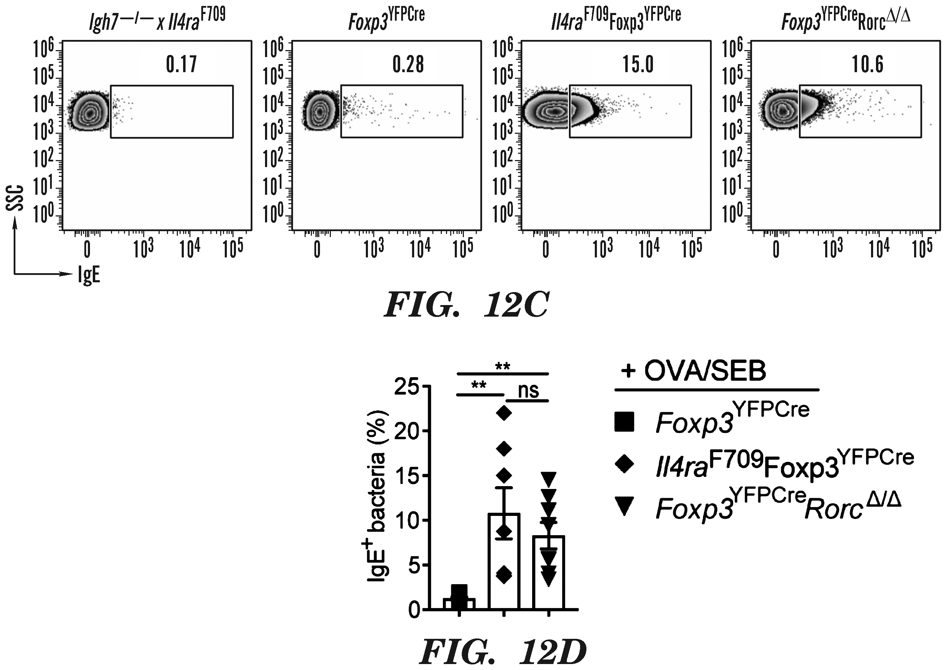

FIGs. 12A-12D show Treg cell-specific deletion of Rorc dysregulates the anti-commensal mucosal antibody responses. (FIG. 12A-12D) Flow cytometric analysis and frequencies of sIgA+ (FIG. 12A-12B) and IgE+ (FIG. 12C-12D) fecal bacteria in Foxp3YFPCre, Il4raF709Foxp3YFPCre and Foxp3YFPCreRorcΔ/Δ mice sensitized with OVA/SEB. Fecal pellets of Rag2-/- and Igh7-/-Il4raF709 mice were used as negative controls for the respective antibody staining. Each symbol in the scatter plots represents one mouse. **P< 0.01, ****P< 0.0001 by one-way ANOVA with Dunnett post hoc analysis.

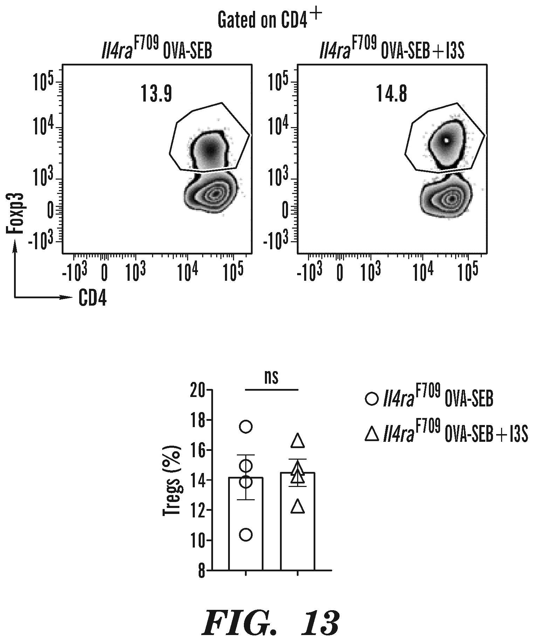

FIG. 13 Left: Representative flow cytometric analysis of the frequency of gut Treg cells (CD4+Foxp3+) in sham versus I3S-treated mice. Right: scatter plot representation of the Treg cell frequencies in the respective group. Note that the frequency of gut Treg cells (CD4+Foxp3+) in sham versus I3S-treated mice is not significantly different.

### DETAILED DESCRIPTION

The role of pathogenic dysbiosis in food allergy (FA) remains unclear. Work described herein shows that FA infants exhibited dysbiotic fecal microbiota that evolved compositionally over time. Both infants and mice with FA had decreased secretory IgA and increased IgE binding to fecal bacteria, indicative of a broader breakdown of oral tolerance in FA than hitherto appreciated. Cross-species fecal transplantation suppressed FA in mice and normalized the gut mucosal immune responses. Fecal transplants from HC, but not those from FA subjects, induced a distinct subset of regulatory T (Treg) cells expressing the transcription factor ROR-γt that was deficient in FA subjects and mice. Deletion of Rorc in Treg cells promoted FA and abrogated protection by the consortia. Different commensals act via ROR-γt+ Treg cells to protect against FA, while dysbiosis impairs this regulatory response to promote disease. Thus, described herein are agents that induce ROR-γt+ Treg cells for the treatment or prevention of a food allergy.

### RORγt and Regulatory T Cells

Methods and compositions described herein require that the levels and/or activity of RORγt are increased in, for example, on or in a regulatory T cell. Retinoic acid-related (RAR) orphan receptor gamma (RORγ) is a protein that in humans is encoded by the RORC (RAR-related orphan receptor C) gene. The RORC gene or the RORγt can also be referred to as RAR Related Orphan Receptor C, RAR-Related Orphan Receptor C, Nuclear Receptor Subfamily 1 Group F Member 3, Nuclear Receptor ROR-Gamma, Nuclear Receptor RZR-Gamma, NR1F3, RORG, RZRG, RAR-Related Orphan Nuclear Receptor Variant 2, Retinoid-Related Orphan Receptor Gamma, Retinoid-Related Orphan Receptor-Gamma, Retinoic Acid-Binding Receptor Gamma, RZR-GAMMA, IMD42, or TOR.

RORγt is produced from an mRNA identical to that of RORγ, with the exception that two 5'-most exons are replaced by an alternative exon, located downstream in the gene. The RORγt protein is a DNA-binding transcription factor and is a member of the NR1 subfamily of nuclear hormone receptors. RORγt is highly restricted to the thymus where it is expressed exclusively in immature CD4+/CD8+ thymocytes and in lymphoid tissue inducer (LTi) cells. RORγt plays an important regulatory role in thymopoiesis, by reducing apoptosis of thymocytes and promoting thymocyte differentiation into pro-inflammatory T helper 17 (Th17) cells. RORγt inhibitors are under development for the treatment of autoimmune diseases such as psoriasis and rheumatoid arthritis. RORγt sequences are known for a number of species, e.g., human RORγt (NCBI Gene ID: 6097, SEQ ID NO: 1) and mouse RORγt (NCBI Gene ID: 19885, SEQ ID NO: 2) polypeptide (e.g., NCBI Reference Sequence: NP_001001523.1, SEQ ID NO: 5; NCBI Reference Sequence: NP_005051.2 and GenBank: AAH14804.1) and mRNA (e.g., NCBI Ref Sequence: NM_001001523.2, SEQ ID NO: 3; NCBI Ref Sequence: NM_005060.4). RORγt can refer to human RORγt, including naturally occurring variants, molecules, and alleles thereof. RORγt refers to the mammalian RORγt of, e.g., mouse, rat, rabbit, dog, cat, cow, horse, pig, and the like. The nucleic sequence of SEQ ID NO: 1 and SEQ ID NO: 2 comprises a nucleic sequence which encodes RORγt.

Regulatory T cells (Tregs), also known as suppressor T cells, are a subpopulation of T cells that modulate the immune system, maintain tolerance to self-antigens, and prevent autoimmune disease. Tregs are immunosuppressive and generally suppress or downregulate induction and proliferation of effector T cells. Tregs can express the biomarkers CD4, FOXP3, and CD25 and are thought to be derived from the same lineage as naïve CD4 cells. Because effector T cells also express CD4 and CD25, Tregs can be very difficult to effectively discern from effector CD4+. The cytokine TGFβ promotes Tregs to differentiate from naïve CD4+ cells and is important in maintaining Treg homeostasis.

### Treating or Preventing Food Allergy

One aspect of the invention is a method for treating or preventing the onset of a food allergy in a subject, the method comprising: administering to a subject an agent that induces a regulatory T cell, or population thereof, that expresses RORγt.

Another aspect of the invention is a method for treating or preventing the onset of a food allergy in a subject, the method comprising: administering to a subject I3S.

Another aspect of the invention is a method for treating or preventing the onset of a food allergy in a subject, the method comprising: administering to a subject CA.

Another aspect of the invention is a method for treating or preventing the onset of a food allergy in a subject comprising (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (b) is statistically lower than the level in a healthy subject; and (d) if the subject has decreased RORγt in regulatory T cells, then administering an agent that induces regulatory T cells that express RORγt to the subject. In one embodiment, the method further comprises, prior to step (a), isolating regulatory T cells from the subject. Methods for isolating regulatory T cells from a subject are known in the art and can be done, for example, by flow cytometry that identifies a regulatory T cell via its unique cell surface markers, e.g., CD4 and FoxP3. In some embodiments, step (d) can further comprise: if the subject does not have decreased RORγt in regulatory T cells, then administering an alternative therapy, as described further herein, or administering no therapy.

Another aspect of the invention is a method for treating or preventing the onset of a food allergy in a subject comprising (a) receiving the results of an assay that determines if the subject has decreased levels of RORγt in regulatory T cells; and (b) if the subject has decreased RORγt in regulatory T cells, then administering an agent that induces regulatory T cells that express RORγt to the subject. In some embodiments, step (b) can further comprise: if the subject does not have decreased RORγt in regulatory T cells, then administering an alternative therapy, as described further herein, or administering no therapy.

Additionally, various aspects of this invention relate to the administration of a fecal matter transplant to a subject having reduced levels of regulatory T cells expressing RORγt.

Described herein are methods of inducing tolerance in a subject. As used herein, the term "tolerance" refers to the process of suppressing a portion of the immune system that recognizes an antigen as being foreign. It will be appreciated by persons skilled in the art that the term "tolerance" as used herein has the same meaning as "immune tolerance". As used herein, the expression "increasing tolerance" or "inducing tolerance" means an increase in tolerance to an antigen relative to the tolerance to the antigen prior to application of the method of the invention. In some embodiments, the term "tolerance" refers to the level of allergic response to a particular quantity of allergen. In some embodiments the tolerance can be oral tolerance and/or mucosal tolerance.

In another aspect, described herein is a method for reducing or eliminating a subject's immune reaction to a food allergen, the method comprising: administering to a subject an agent that induces a regulatory T cell, or population thereof, that expresses RORγt, thereby reducing or eliminating a subject's immune reaction to a food allergen.

Specifically, one aspect of the invention is a method for treating or preventing the onset of a food allergy in a subject comprising (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (a) is statistically lower than the level in a healthy subject; and (d) if the subject has decreased RORγt in regulatory T cells, then administering a fecal matter transplant to the subject, wherein the fecal matter is obtained from a healthy subject. In one embodiment, the method further comprises, prior to step (a), isolating regulatory T cells from the subject. Methods for isolating regulatory T cells from a subject are described herein above. In some embodiments, step (d) can further comprise: if the subject does not have decreased RORγt in regulatory T cells, then administering an alternative therapy, as described further herein, or administering no therapy.

In another aspect, described herein is a method for treating or preventing the onset of a food allergy in a subject, the method comprising: (a) measuring a level of RORγt in regulatory T cells in a subject; (b) comparing the level of RORγt from (a) to the level of level of RORγt in regulatory T cells in a healthy subject; (c) identifying a subject as having decreased RORγt in regulatory T cells if the level from step (b) is statistically lower than the level in a healthy subject; (d) identifying whether the subject has, or is likely to develop, a food allergy; and (e) if the subject has decreased RORγt in regulatory T cells and has, or is likely to develop, a food allergy, then administering an agent that induces regulatory T cells that express RORγt to the subject. In some embodiments, the method further comprises, prior to step (a), isolating regulatory T cells from the subject. In some embodiments, step (e) can further comprise: if the subject does not have decreased RORγt in regulatory T cells and/or the subject does not have, or is not likely to develop, a food allergy, then administering an alternative therapy, as described further herein, or administering no therapy. In some embodiments, step (d) of identifying whether the subject has, or likely to develop, a food allergy, is conducted first. In some embodiments, if the subject does not have, or is not likely to develop, a food allergy, then the method does not comprise steps (a)-(c), and further comprises administering no therapy to the subject.

Yet another aspect of the invention is a method for treating or preventing the onset of a food allergy in a subject comprising (a) receiving the results of an assay that determines if the subject has decreased levels of RORγt in regulatory T cells; and (b) if the subject has decreased RORγt in regulatory T cells, then administering a fecal matter transplant to the subject, wherein the fecal matter is obtained from a healthy subject. In some embodiments, step (b) can further comprise: if the subject does not have decreased RORγt in regulatory T cells, then administering an alternative therapy, as described further herein, or administering no therapy.

In another aspect, described herein is a method for treating or preventing the onset of a food allergy in a subject, the method comprising: (a) receiving the results of an assay that determines if the subject has decreased levels of RORγt in regulatory T cells; (b) receiving the results of an assay that determines if the subject has, or is likely to develop, a food allergy; and (c) if the subject has decreased RORγt in regulatory T cells and has, or is likely to develop, a food allergy, then administering an agent that induces regulatory T cells that express RORγt to the subject. In some embodiments, step (c) can further comprise: if the subject does not have decreased RORγt in regulatory T cells and/or the subject does not have, or is not likely to develop, a food allergy, then administering an alternative therapy, as described further herein, or administering no therapy. In some embodiments, step (b) of receiving the results of an assay that determines if the subject has, or is likely to develop, a food allergy is conducted first. In some embodiments, if the subject does not have, or is not likely to develop, a food allergy, then the method does not comprise step (a), and further comprises administering no therapy to the subject.

As used herein, "fecal matter transplant" refers to a transfer of stool from a healthy donor, e.g., a donor not having or at risk of having a food allergy, to a gastrointestinal tract of a subject. Previous terms for the procedure include fecal bacteriotherapy, fecal transfusion, fecal transplant, stool transplant, fecal enema, and human probiotic infusion (HPI). Because the procedure involves the complete restoration of the entire fecal microbiota, not just a single agent or combination of agents, these terms have now been replaced by the new term fecal microbiota transplantation. Methods for performing a fecal matter transplant are known in the art, for example, performed by colonoscopy and less commonly by nasoduodenal tube. During colonoscopy, the colonoscope is advanced through the entire colon. As the colonoscope is withdrawn, the fecal matter obtained from the healthy donor is delivered through the colonoscopy into the subject's colon. Fecal matter samples can be prepared and administered in various forms, for example, a freeze dried sample, a fresh sample, a blended sample, or a diluted sample. Methods for preparation and administration of a fecal matter sample are further described in, e.g., US Patent Nos 9192361, 9308226, and 9968638; and International Patent Application No. WO2014152484 which are incorporated herein, in their entirety.

As used herein, an "food allergy" refers to a failure of oral tolerance to food antigens associated with Th2 immunity and allergen-specific IgE responses. That is, an immune response is generated in response to particular food antigens. Food intolerance often presents with symptoms similar to a food allergy, but does not involve an immune response. The most common food allergies include, but are not limited to, allergies to cow's milk, eggs, tree nuts, peanuts, shellfish, wheat, soy, and fish. Food allergy affects an estimated 6 to 8 percent of children under age 3 and up to 3 percent of adults.

Common symptoms of food allergies include, but are not limited to, tingling or itching in the mouth; hives, itching or eczema; swelling of the lips, face, tongue and throat or other parts of the body; wheezing, nasal congestion or trouble breathing; abdominal pain, diarrhea, nausea or vomiting; dizziness, lightheadedness or fainting. During severe allergic reactions, anaphylaxis can occur, resulting in constriction and tightening of the airways; a swollen throat or the sensation of a lump in your throat that makes it difficult to breathe; shock with a severe drop in blood pressure; rapid pulse; and dizziness, lightheadedness or loss of consciousness. Untreated, anaphylaxis can cause a coma or even death.

In one embodiment, the food allergy is pollen-food allergy syndrome. Pollen-food allergy syndrome, also known as oral allergy syndrome, affects many people who have hay fever. In this condition, certain fresh fruits and vegetables or nuts and spices can trigger an allergic reaction that causes the mouth to tingle or itch. In serious cases, the reaction results in swelling of the throat or even anaphylaxis. Proteins in certain fruits, vegetables, nuts and spices cause the reaction because they're similar to allergy-causing proteins found in certain pollens. This is an example of cross-reactivity.

In one embodiment, the food allergy is exercised-induced food allergy. Eating certain foods may cause some people to feel itchy and lightheaded soon after starting to exercise. Serious cases may even involve hives or anaphylaxis.

In one embodiment, an agent is administered as a prophylactic treatment to prevent a food allergy in a subject at risk of developing a food allergy. Risk factors for developing a food allergy include, but are not limited to a family history of asthma, eczema, hives, food allergy or other allergies; having other allergies, for example, to hay, pet dander, or seasonal allergies; a young age (e.g., newborn, infant, toddler, or child); and having asthma.

In one embodiment, the method further comprises, prior to administration, diagnosing a subject as having, or likely to develop, a food allergy. In another embodiment, the method further comprises, prior to administration, receiving the results of an assay that diagnoses a subject as having, or likely to develop, a food allergy. Methods and assays for diagnosing a food allergy include, but are not limited to a complete family history of allergic disease, a blood test, for example, ImmunoCAP test), and/or a skin prick food allergy test that indicates if a subject has food-specific IgE antibodies. These assays are commonly known in the art and can be executed by a skilled clinician.

In one embodiment, the agent or fecal matter transplant is administered prior to the subject's first exposure to an allergen. In one embodiment, the agent or fecal matter transplant is administered after the onset of clinical symptoms associated with a food allergy, or after a subject has been diagnosed as having at least one food allergy.

Additional aspects of the invention include methods for reducing or eliminating a subject's immune reaction to a food allergen by administering to a subject an agent that induces a regulatory T cell, or population thereof, that expresses RORγt, thereby reducing or eliminating a subject's immune reaction to a food allergen. In one embodiment, the agent is I3S. In one embodiment, the agent is CA. In another embodiment, the subject is administered a fecal matter transplant to reduce or eliminate a subject's immune reaction to a food allergen. Methods for measuring an immune reaction to a food allergen can be performed by a skilled clinician, and include, but are not limited to, identifying IgE antibodies produced by a subject following exposure to a food allergen. To identifying if an immune response is reduced or eliminated, one skilled in the art can, e.g., measure the level of IgE antibodies produced by a subject prior to and following administration of the agent or fecal matter transplant, compare the levels, and identify a subject as having a reduced or eliminated immune response if the level of IgE after administration is lower than the level prior to administration.

Additional aspects of the invention include a method of identifying a microorganism that induces a regulatory T cell, or a population thereof, that expresses RORγt, comprising (a) introducing at least one microorganism a subject for a time sufficient to allow for the colonization of the at least one microorganism in the gut of the germ-free mouse model; (b) measuring a level of expression of RORγt in regulatory T cells in the subject; and (c) identifying the at least one microorganism as an inducer of a regulatory T cell, or a population thereof, that expresses RORγt if the level of expression from step (b) is greater than zero. In some embodiments, the subject comprises a germ-free mouse model. Germ-free mice have no regulatory T cells which express RORγt, thus any increase in such regulatory T cell following colonization by the at least one microorganism would indicate a beneficial microorganism for treating and/or preventing food allergies.

Accordingly, in one aspect described herein is a method of inducing tolerance in a subject, the method comprising administering to a subject an agent that induces a regulatory T cell, or population thereof, that expresses RORγt. In some embodiments, the agent is I3S, CA, or a composition as described herein.

As described herein, levels of RORγt (e.g., in a regulatory T cell) can be decreased in a food allergy and/or in subjects with a food allergy. Accordingly, in one aspect of any of the embodiments, described herein is a method of treating a food allergy in a subject in need thereof, the method comprising administering an agent as described herein (e.g., I3S or CA) to a subject determined to have a level of RORγt that is decreased relative to a reference. In one aspect of any of the embodiments, described herein is a method of treating a food allergy in a subject in need thereof, the method comprising: a) determining the level of RORγt in a sample obtained from a subject; and b) administering an agent as described herein (e.g., I3S or CA) to the subject if the level of RORγt is decreased relative to a reference.

In some embodiments of any of the aspects, the method comprises administering an agent as described herein (e.g., I3S or CA) to a subject previously determined to have a level of RORγt that is decreased relative to a reference. In some embodiments of any of the aspects, described herein is a method of treating a food allergy in a subject in need thereof, the method comprising: a) first determining the level of RORγt in a sample obtained from a subject; and b) then administering an agent as described herein (e.g., I3S or CA) to the subject if the level of RORγt is decreased relative to a reference.

In one aspect of any of the embodiments, described herein is a method of treating a food allergy in a subject in need thereof, the method comprising: a) determining if the subject has a decreased level of RORγt; and b) administering an agent as described herein (e.g., I3S or CA) to the subject if the level of RORγt is decreased relative to a reference. In some embodiments of any of the aspects, the step of determining if the subject has a decreased level of RORγt can comprise i) obtaining or having obtained a sample from the subject and ii) performing or having performed an assay on the sample obtained from the subject to determine/measure the level of RORγt in the subject. In some embodiments of any of the aspects, the step of determining if the subject has a decreased level of RORγt can comprise performing or having performed an assay on a sample obtained from the subject to determine/measure the level of RORγt in the subject. In some embodiments of any of the aspects, the step of determining if the subject has a decreased level of RORγt can comprise ordering or requesting an assay on a sample obtained from the subject to determine/measure the level of RORγt in the subject. In some embodiments of any of the aspects, the step of determining if the subject has a decreased level of RORγt can comprise receiving the results of an assay on a sample obtained from the subject to determine/measure the level of RORγt in the subject. In some embodiments of any of the aspects, the step of determining if the subject has a decreased level of RORγt can comprise receiving a report, results, or other means of identifying the subject as a subject with an decreased level of RORγt.

In one aspect of any of the embodiments, described herein is a method of treating a food allergy in a subject in need thereof, the method comprising: a) determining if the subject has a decreased level of RORγt; and b) instructing or directing that the subject be administered an agent as described herein (e.g., I3S or CA) if the level of RORγt is decreased relative to a reference. In some embodiments of any of the aspects, the step of determining if the subject has a decreased level of RORγt can comprise i) obtaining or having obtained a sample from the subject and ii) performing or having performed an assay on the sample obtained from the subject to determine/measure the level of RORγt in the subject. In some embodiments of any of the aspects, the step of determining if the subject has a decreased level of RORγt can comprise performing or having performed an assay on a sample obtained from the subject to determine/measure the level of RORγt in the subject. In some embodiments of any of the aspects, the step of determining if the subject has a decreased level of RORγt can comprise ordering or requesting an assay on a sample obtained from the subject to determine/measure the level of RORγt in the subject. In some embodiments of any of the aspects, the step of instructing or directing that the subject be administered a particular treatment can comprise providing a report of the assay results. In some embodiments of any of the aspects, the step of instructing or directing that the subject be administered a particular treatment can comprise providing a report of the assay results and/or treatment recommendations in view of the assay results.

### Agents

In various embodiment, agents described herein induce a regulatory T cell, or population thereof, that expresses RORγt. In various embodiment, agents described herein upmodulate RORγt in a regulatory T cell, or population thereof. The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and non-proteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In certain embodiments, agents are small molecule having a chemical moiety. For example, chemical moieties included unsubstituted or substituted alkyl, aromatic, or heterocyclyl moieties including macrolides, leptomycins and related natural products or analogues thereof. Compounds can be known to have a desired activity and/or property, or can be selected from a library of diverse compounds.

Such an agent can take the form of any entity which is normally not present or not present at the levels being administered in the cell. Agents such as chemicals; small molecules; nucleic acid sequences; nucleic acid analogues; proteins; peptides; aptamers; antibodies; or fragments thereof, can be identified or generated for use to upmodulate RORγt.

The agent can be a molecule from one or more chemical classes, e.g., organic molecules, which may include organometallic molecules, inorganic molecules, genetic sequences, etc. Agents may also be fusion proteins from one or more proteins, chimeric proteins (for example domain switching or homologous recombination of functionally significant regions of related or different molecules), synthetic proteins or other protein variations including substitutions, deletions, insertion and other variants.

The agent may function directly in the form in which it is administered. Alternatively, the agent can be modified or utilized intracellularly to produce something which upmodulates RORγt, such as introduction of a nucleic acid sequence into the cell and its transcription resulting in the production of the nucleic acid and/or protein inhibitor or activator of RORγt within the cell. In some embodiments, the agent is any chemical, entity or moiety, including without limitation synthetic and naturally-occurring non-proteinaceous entities. In certain embodiments the agent is a small molecule having a chemical moiety. For example, chemical moieties included unsubstituted or substituted alkyl, aromatic, or heterocyclyl moieties including macrolides, leptomycins and related natural products or analogues thereof. Agents can be known to have a desired activity and/or property, or can be selected from a library of diverse compounds.

Agents in the form of a protein and/or peptide or fragment thereof can also be designed to upmodulate RORγt. Such agents encompass proteins which are normally absent or proteins that are normally endogenously expressed in the host cell. Examples of useful proteins are mutated proteins, genetically engineered proteins, peptides, synthetic peptides, recombinant proteins, chimeric proteins (any of which may take the form of a dominant negative protein for LIN28B), antibodies, midibodies, minibodies, triabodies, humanized proteins, humanized antibodies, chimeric antibodies, modified proteins and fragments thereof. Agents also include antibodies (polyclonal or monoclonal), neutralizing antibodies, antibody fragments, peptides, proteins, peptide-mimetics, aptamers, oligonucleotides, hormones, small molecules, nucleic acids, nucleic acid analogues, carbohydrates or variants thereof that function to inactivate the nucleic acid and/or protein of the gene products identified herein, and those as yet unidentified.

In one embodiment, the agent that increased RORγt expression in a regulatory T cell is Indoxyl sulfate (I3S). The molecular formula of I3S is C₈H₇NO₄S, and its average mass is 213.210 Da. The structure for I3S is shown below in formula I.

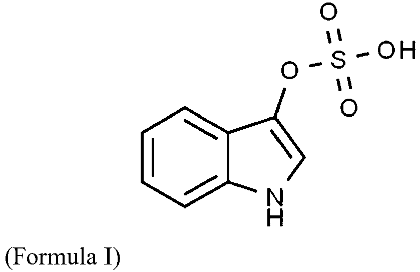

Indoxyl sulfate, also known as 3-indoxylsulfate and 3-indoxylsulfuric acid, is a metabolite of dietary L-tryptophan that acts as a cardiotoxin and uremic toxin. High concentrations of indoxyl sulfate in blood plasma are known to be associated with the development and progression of various diseases, for example chronic kidney disease and vascular disease in humans. As a uremic toxin, it stimulates glomerular sclerosis and renal interstitial fibrosis. Indoxyl sulfate is a metabolite of dietary L-tryptophan that is synthesized through the following metabolic pathway: L-tryptophan → indole → indoxyl → indoxyl sulfate.

Indole is produced from L-tryptophan in the human intestine via tryptophanase-expressing gastrointestinal bacteria. Indoxyl is produced from indole via enzyme-mediated hydroxylation in the liver; in vitro experiments with liver microsomes suggest that the CYP450 enzyme CYP2E1 hydroxylates indole into indoxyl. Subsequently, indoxyl is converted into indoxyl sulfate by sulfotransferase enzymes in the liver; based upon in vitro experiments with recombinant human sulfotransferases, SULT1A1 appears to be the primary sulfotransferase enzyme involved in the conversion of indoxyl into indoxyl sulfate.

In one embodiment, the agent that increased RORγt expression in a regulatory T cell is indol-3-carboxaldehyde (CA). The molecular formula of CA is C₉H₇NO, and its average mass is 145.158 Da. The structure for CA is shown below in formula II.

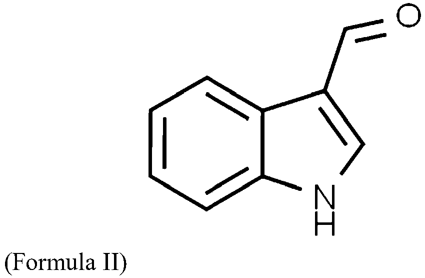

Indol-3-carboxaldehyde (CA), also known as I3A, 1*H*-Indole-3-carbaldehyde, indole-3-aldehyde, and 3-formylindole, is a metabolite of dietary l-tryptophan which is synthesized by human gastrointestinal bacteria, particularly species of the *Lactobacillus* genus. Accordingly, in some embodiments of any of the aspects, the agent is a bacterial metabolite, i.e., a product produced during a metabolic reaction in a prokaryote.

CA is a biologically active metabolite which acts as a receptor agonist at the aryl hydrocarbon receptor in intestinal immune cells, in turn stimulating the production of interleukin-22 which facilitates mucosal reactivity.

Indole-3-carbaldehyde is a heteroarenecarbaldehyde that is indole in which the hydrogen at position 3 has been replaced by a formyl group. It has a role as a plant metabolite, a human xenobiotic metabolite, a bacterial metabolite and a marine metabolite. It is a heteroarenecarbaldehyde, an indole alkaloid and a member of indoles.

In some embodiments of any of the aspects, the agent is an indole. In some embodiments of any of the aspects, the agent is a substituted indole, as shown in formulas III, IV, and V. In some embodiments of any of the aspects, the agent is an indole wherein the hydrogen at least one of positions 1, 2, and/or 3 of the pyrrole ring is replaced with an R¹ group (see e.g., formula III). In some embodiments of any of the aspects, the agent is an indole wherein the hydrogen at least one of positions 4, 5, 6, and/or 7 of the benzene ring is replaced with an R² group (see e.g., formula III). In some embodiments of any of the aspects, the agent is an indole wherein the hydrogen at position 3 has been replaced by an R¹ group (see e.g., formulas VI and V).

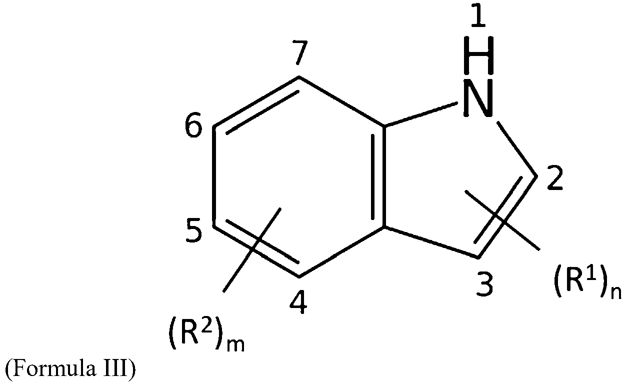

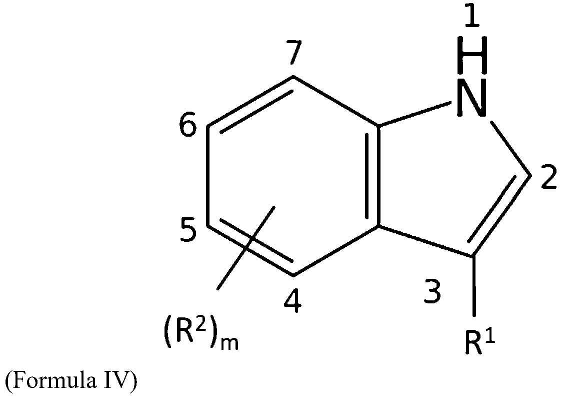

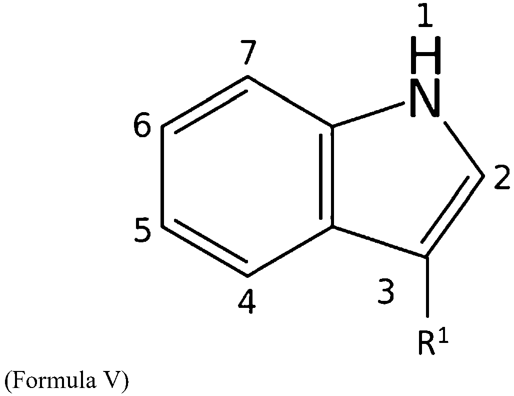

In the above structures (e.g., formulas III or IV) m is 0, 1, 2, 3, or 4. In some embodiments, m is 0, 1 or 2. In some further embodiments, m is 0.

In the above structures (e.g., formula III), n is 0, 1, 2 or 3. In some embodiments, n is 0, or 1. In some further embodiments, n is 1.

Non-limiting examples of R¹ and R² groups include -OH, an alkoxy group, an acyloxy group, a formyl group, an amino group, an amido group, a sulfate group, a sulfonate group, a phosphate group, a phosphonate group, a silicone group, a carboxyaldehyde group, a nitro group, a fluoro group, a chloro group, an iodo group, a bromo group, a -CF₃ group, a nitrile group, a ketone group, a carbonyl group, an aldehyde group, a carboxylic acid group, a mercapto group, a methoxy group, a cyano group, hydroxy methyl, methyl, ethyl, propyl, isopropyl, butyl, tert-butyl, pentyl, cyclopropyl, cyclopentyl, benzyl, beta-phenylethyl, gamma-tolylpropyl, phenyl, tolyl, xylyl, and naphthyl.

In some embodiments of any of the aspects, the compound is of formula I. In some embodiments of any of the aspects, the compound is of formula II. In some embodiments of any of the aspects, the compound is of formula III. In some embodiments of any of the aspects, the compound is of formula IV. In some embodiments of any of the aspects, the compound is of formula V.

In one embodiment, RORγt is upmodulated in a regulatory T cell by a nucleic acid encoding RORγt expressed in the cell e.g., via a vector comprising a nucleic acid encoding RORγt. In another embodiment, a nucleic acid encoding RORγt is expressed in the cell e.g., via expression of a nucleic acid encoding RORγt as naked DNA. In one embodiment, the nucleic acid encoding RORγt has a sequence corresponding to the sequence of SEQ ID NO: 1, SEQ ID NO: 2, SEQ ID NO: 3, or SEQ ID NO: 4; or comprises the sequence of SEQ ID NOs: 1-4; or comprises a sequence with at least 80%, at least 85%, at least 90%, at least 91%, at least 92%, at least 93%, at least 94%, at least 95%, at least 96%, at least 97%, at least 98%, at least 99%, or at least 100% sequence identity to the sequence of SEQ ID NOs: 1-4, and having the same activity as the sequence of SEQ ID NOs: 1-4 (e.g., prevent the onset of a food allergy or treat a food allergy).

In one embodiment, the nucleic acid encoding RORγt has a sequence corresponding to the amino acid sequence of SEQ ID NO: 5 or SEQ ID NO: 6; or encodes a sequence comprising SEQ ID NO: 5 or SEQ ID NO: 6; or encodes a polypeptide comprising a sequence with at least 80%, at least 85%, at least 90%, at least 91%, at least 92%, at least 93%, at least 94%, at least 95%, at least 96%, at least 97%, at least 98%, at least 99%, or at least 100% sequence identity to the sequence of SEQ ID NO: 5 or SEQ ID NO: 6, and having the same activity as the sequence of SEQ ID NO: 5 or SEQ ID NO: 6 (e.g., prevent the onset of a food allergy or treat a food allergy).

SEQ ID NO: 1 is a nucleotide sequence that encodes human RORγt ortholog RAR related orphan receptor C. SEQ ID NO: 2 is a nucleotide sequence that encodes mouse RORγt. SEQ ID NO: 3 is a nucleotide sequence that encodes human RORγt ortholog RAR related orphan receptor C mRNA. SEQ ID NO: 4 is a nucleotide sequence that encodes mouse RORγt mRNA. SEQ ID NO: 5 is an amino acid sequence for human RORγt ortholog RAR related orphan receptor C polypeptide. SEQ ID NO: 6 is an amino acid sequence for mouse RORγt polypeptide.

In one embodiment, the agent is a small molecule that upmodulates RORγt. Methods for screening small molecules are known in the art and can be used to identify a small molecule that is efficient at, for example, upmodulating a desired target, e.g., RORγt.

One aspect provided herein is a composition comprising any of the agents that upmodulated RORγt, as described herein.

In one embodiment, the composition further comprises a pharmaceutically acceptable carrier. As used herein, the term "pharmaceutically acceptable", and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like. Each carrier must also be "acceptable" in the sense of being compatible with the other ingredients of the formulation. A pharmaceutically acceptable carrier will not promote the raising of an immune response to an agent with which it is admixed, unless so desired. The preparation of a pharmacological composition that contains active ingredients dissolved or dispersed therein is well understood in the art and need not be limited based on formulation. The pharmaceutical formulation contains a compound of the invention in combination with one or more pharmaceutically acceptable ingredients. The carrier can be in the form of a solid, semisolid or liquid diluent, cream or a capsule. Typically, such compositions are prepared as injectable either as liquid solutions or suspensions, however, solid forms suitable for solution, or suspensions, in liquid prior to use can also be prepared. The preparation can also be emulsified or presented as a liposome composition. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient. The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Physiologically tolerable carriers are well known in the art. Exemplary liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin, vegetable oils such as cottonseed oil, and water-oil emulsions. The amount of an active agent used in the invention that will be effective in the treatment of a particular disorder or condition will depend on the nature of the disorder or condition, and can be determined by standard clinical techniques. The phrase "pharmaceutically acceptable carrier or diluent" means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body.

In some embodiments, the composition further comprises a fecal matter transplant. In some embodiments, the composition comprises the agent (e.g., I3S or CA) and a fecal matter transplant, which is administered in the form of a suppository, pill, capsule, or the like. In some embodiments, the composition comprising the agent (e.g., I3S or CA) and a fecal matter transplant is administered orally, rectally, or enterically. In some embodiments, the composition comprising the agent (e.g., I3S or CA) and a fecal matter transplant is administered in the form of a liquid or solution. In some embodiments, the composition comprising the agent (e.g., I3S or CA) and a fecal matter transplant is administered using a colonoscopy, enema, or a plastic tube inserted through the nose into the gastrointestinal tract (e.g., stomach or intestines).

In one embodiment, the agent prevents and/or reverses Th2 programming of Tregs and other mucosal T cell populations. In some embodiments, the Th2 programming is measured by IL-4 production and/or GATA3 expression by the Tregs and other mucosal T cell populations. Methods for measuring IL-4 and GATA3 expression include but are not limited to flow cytometry, Western blotting, ELISA, or any other method known in the art.

In one embodiment of any aspect, the regulatory T cell expressing RORγt has a lower expression of the Helios marker as compared to a regulatory T cell that does not express RORγt. In one embodiment of any aspect, the regulatory T cell expressing RORγt has a higher expression of the Helios marker as compared to a regulatory T cell that does not express RORγt. In one embodiment of any aspect, the regulatory T cell expressing RORγt has the same expression of the Helios marker as compared to a regulatory T cell that does not express RORγt.

The Ikaros family member, Helios, has been reported as a marker to discriminate naturally occurring, thymic-derived Tregs from those peripherally induced from naïve CD4+ T cells. It was found that Helios-negative T cells are enriched for naïve T cell phenotypes and vice versa. Moreover, Helios can be induced during T cell activation and proliferation, but regresses in the same cells under resting conditions.

In one embodiment, the agent upmodulates RORγt in a regulatory T cell by at least 2x, 3x, 4x, 5x, 6x, 7x, 8x, 9x, or more as compared to an appropriate control. As used herein, an appropriate control refers to an identical cell population or subject that is not contacted with an agent described herein or not administered an agent described herein, or not administered a fecal matter transplant. Compositions described herein can be formulated for any route of administration described herein below. Methods for formulating a composition for a desired administration are further discussed herein.

### Administration

In some embodiments, the methods described herein relate to treating a subject having, diagnosed as having, at risk of having, or diagnosed as being at risk of having a food allergy comprising administering an agent that increases RORγt in a regulatory T cell as described herein. Subjects having or at risk of having a food allergy can be identified by a physician using current methods (i.e. assays) of diagnosing a condition. Symptoms and/or complications of food allergy, which characterize these disease and aid in diagnosis are well known in the art and include but are not limited to, skin rash, digestive distress, anaphylactic shock, or edema. Tests that may aid in a diagnosis of, e.g. food allergy, include but are not limited to skin tests that exposes the skin to concentrated amounts of a common food allergen, or blood tests. A family history of, e.g., food allergy, will also aid in determining if a subject is likely to have the condition or in making a diagnosis of food allergy.

The agents described herein (e.g., an agent that increases RORγt in a regulatory T cell) can be administered to a subject having or diagnosed as having a food allergy. The agents described herein (e.g., an agent that increases RORγt in a regulatory T cell) can be administered to a subject at risk of having or diagnosed as being at risk of having a food allergy. In some embodiments, the methods described herein comprise administering an effective amount of an agent to a subject in order to alleviate at least one symptom of, e.g., food allergy. As used herein, "alleviating at least one symptom of a food allergy" is ameliorating any condition or symptom associated with, e.g., food allergy (e.g., skin rash, digestive distress, anaphylactic shock, or edema). As compared with an equivalent untreated control, such reduction is by at least 5%, 10%, 20%, 40%, 50%, 60%, 80%, 90%, 95%, 99% or more as measured by any standard technique. A variety of means for administering the agents described herein to subjects are known to those of skill in the art. Such methods can include, but are not limited to oral, parenteral, intravenous, intramuscular, subcutaneous, transdermal, airway (aerosol), pulmonary, cutaneous, topical, or injection administration. In one embodiment, the agent is administered systemically or locally (e.g., to the lungs). In one embodiment, the agent is administered intravenously. In one embodiment, the agent is administered continuously, in intervals, or sporadically. The route of administration of the agent will be optimized for the type of agent being delivered (e.g., an antibody, a small molecule, an RNAi, a fecal transplant), and can be determined by a skilled practitioner. In some embodiments, the agent is administered orally, rectally, enterically, using a colonoscopy, using an enema, or using a plastic tube inserted through the nose into the gastrointestinal tract (e.g., stomach or intestines).

The term "effective amount" as used herein refers to the amount of an agent (e.g., an agent that increases RORγt in a regulatory T cell) can be administered to a subject having, diagnosed as having, or at risk of having a food allergy needed to alleviate at least one or more symptom of, e.g., food allergy. The term "therapeutically effective amount" therefore refers to an amount of an agent that is sufficient to provide, e.g., a particular anti-food allergy effect when administered to a typical subject. An effective amount as used herein, in various contexts, would also include an amount of an agent sufficient to delay the development of a symptom of, e.g., a food allergy, alter the course of a symptom of, e.g., a food allergy, or reverse a symptom of a food allergy. Thus, it is not generally practicable to specify an exact "effective amount". However, for any given case, an appropriate "effective amount" can be determined by one of ordinary skill in the art using only routine experimentation.

In one embodiment, the agent is administered continuously (e.g., at constant levels over a period of time). Continuous administration of an agent can be achieved, e.g., by epidermal patches, continuous release formulations, or on-body injectors.

In one embodiment, the agent, for example I3S or CA, is administered once every 2 weeks or once every 4 weeks. An agent described herein can be administered at least once a day, a week, every 3 weeks, a month, every 2 months, every 3 months, every 4 months, every 5 months, every 6 months, every 7 months, every 8 months, every 9 months, every 10 months, every 11 months, a year, or more.

Effective amounts, toxicity, and therapeutic efficacy can be evaluated by standard pharmaceutical procedures in cell cultures or experimental animals. The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (*i.e.,* the concentration of the agent, which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay, e.g., measuring neurological function, or blood work, among others. The dosage can be determined by a physician and adjusted, as necessary, to suit observed effects of the treatment.

In some embodiments, the technology described herein relates to a pharmaceutical composition comprising an agent as described herein (e.g., I3S or CA) as described herein, and optionally a pharmaceutically acceptable carrier. In some embodiments, the active ingredients of the pharmaceutical composition comprise an agent as described herein (e.g., I3S or CA). In some embodiments, the active ingredients of the pharmaceutical composition consist essentially of an agent as described herein (e.g., I3S or CA). In some embodiments, the active ingredients of the pharmaceutical composition consist of an agent as described herein (e.g., I3S or CA). Pharmaceutically acceptable carriers and diluents include saline, aqueous buffer solutions, solvents and/or dispersion media. The use of such carriers and diluents is well known in the art. Some non-limiting examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (23) C₂-C₁₂ alcohols, such as ethanol; and (24) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein. In some embodiments, the carrier inhibits the degradation of the active agent, e.g. an agent as described herein (e.g., I3S or CA).

Pharmaceutical compositions comprising an agent as described herein (e.g., I3S or CA) can also be formulated to be suitable for oral administration, for example as discrete dosage forms, such as, but not limited to, tablets (including without limitation scored or coated tablets), pills, caplets, capsules, chewable tablets, powder packets, cachets, troches, wafers, aerosol sprays, or liquids, such as but not limited to, syrups, elixirs, solutions or suspensions in an aqueous liquid, a non-aqueous liquid, an oil-in-water emulsion, or a water-in-oil emulsion. Such compositions contain a predetermined amount of the pharmaceutically acceptable salt of the disclosed compounds, and may be prepared by methods of pharmacy well known to those skilled in the art. See generally, Remington: The Science and Practice of Pharmacy, 21st Ed., Lippincott, Williams, and Wilkins, Philadelphia PA. (2005).

In certain embodiments, an effective dose of a composition comprising an agent as described herein (e.g., I3S or CA) can be administered to a patient once. In certain embodiments, an effective dose of a composition comprising an agent as described herein (e.g., I3S or CA) can be administered to a patient repeatedly. For systemic administration, subjects can be administered a therapeutic amount of a composition comprising an agent as described herein (e.g., I3S or CA), such as, e.g. 0.1 mg/kg, 0.5 mg/kg, 1.0 mg/kg, 2.0 mg/kg, 2.5 mg/kg, 5 mg/kg, 10 mg/kg, 15 mg/kg, 20 mg/kg, 25 mg/kg, 30 mg/kg, 40 mg/kg, 50 mg/kg, or more.

In some embodiments, after an initial treatment regimen, the treatments can be administered on a less frequent basis. For example, after treatment biweekly for three months, treatment can be repeated once per month, for six months or a year or longer. Treatment according to the methods described herein can reduce levels of a marker or symptom of a condition, e.g. a food allergy by at least 10%, at least 15%, at least 20%, at least 25%, at least 30%, at least 40%, at least 50%, at least 60%, at least 70%, at least 80 % or at least 90% or more.

The dosage of a composition as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule can vary from once a week to daily depending on a number of clinical factors, such as the subject's sensitivity to an agent as described herein (e.g., I3S or CA). The desired dose or amount of activation can be administered at one time or divided into subdoses, e.g., 2-4 subdoses and administered over a period of time, e.g., at appropriate intervals through the day or other appropriate schedule. In some embodiments, administration can be chronic, e.g., one or more doses and/or treatments daily over a period of weeks or months. Examples of dosing and/or treatment schedules are administration daily, twice daily, three times daily or four or more times daily over a period of 1 week, 2 weeks, 3 weeks, 4 weeks, 1 month, 2 months, 3 months, 4 months, 5 months, or 6 months, or more. A composition comprising an agent as described herein (e.g., I3S or CA) can be administered over a period of time, such as over a 5 minute, 10 minute, 15 minute, 20 minute, or 25 minute period.

### Dosage

"Unit dosage form" as the term is used herein refers to a dosage for suitable one administration. By way of example a unit dosage form can be an amount of therapeutic disposed in a delivery device, e.g., a syringe or intravenous drip bag. In one embodiment, a unit dosage form is administered in a single administration. In another, embodiment more than one unit dosage form can be administered simultaneously.

The dosage of the agent as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to administer further cells, discontinue treatment, resume treatment, or make other alterations to the treatment regimen. The dosage should not be so large as to cause adverse side effects, such as cytokine release syndrome. Generally, the dosage will vary with the age, condition, and sex of the patient and can be determined by one of skill in the art. The dosage can also be adjusted by the individual physician in the event of any complication.

The dosage ranges for the administration of an agent as described herein (e.g., I3S or CA), according to the methods described herein depend upon, for example, the form of an agent as described herein (e.g., I3S or CA), its potency, and the extent to which symptoms, markers, or indicators of a condition described herein are desired to be reduced, for example the percentage reduction desired for a food allergy or the extent to which, for example, RORγT levels in a T regulatory cell or population thereof or levels of RORγT T regulatory cells are desired to be induced. The dosage should not be so large as to cause adverse side effects, such as immunosuppression or immunodeficiency. Generally, the dosage will vary with the age, condition, and sex of the patient and can be determined by one of skill in the art. The dosage can also be adjusted by the individual physician in the event of any complication.

In some embodiments, the methods described herein comprise administering an effective amount of an agent (e.g., I3S or CA) to a subject in order to alleviate at least one symptom of, e.g., food allergy. In some embodiments, the dosage of the agent (e.g., I3S or CA) is approximately 100 mg per kilogram of the subject (mg/kg). In some embodiments, the dosage of the agent (e.g., I3S or CA) is 1 mg/kg - 5 mg/kg, 5 mg/kg - 10 mg/kg, 10 mg/kg - 15 mg/kg, 15 mg/kg - 20 mg/kg, 20 mg/kg - 25 mg/kg, 25 mg/kg - 30 mg/kg, 30 mg/kg - 35 mg/kg, 35 mg/kg - 40 mg/kg, 40 mg/kg - 45 mg/kg, 45 mg/kg - 50 mg/kg, 50 mg/kg - 55 mg/kg, 55 mg/kg - 60 mg/kg, 60 mg/kg - 65 mg/kg, 65 mg/kg - 70 mg/kg, 70 mg/kg - 75 mg/kg, 75 mg/kg - 80 mg/kg, 80 mg/kg - 85 mg/kg, 85 mg/kg - 90 mg/kg, 90 mg/kg - 95 mg/kg, 95 mg/kg - 100 mg/kg, 101 mg/kg - 105 mg/kg, 105 mg/kg - 110 mg/kg, 110 mg/kg - 115 mg/kg, 115 mg/kg - 120 mg/kg, 120 mg/kg - 125 mg/kg, 125 mg/kg - 130 mg/kg, 130 mg/kg - 135 mg/kg, 135 mg/kg - 140 mg/kg, 140 mg/kg - 145 mg/kg, 145 mg/kg - 150 mg/kg, 150 mg/kg - 155 mg/kg, 155 mg/kg - 160 mg/kg, 160 mg/kg - 165 mg/kg, 165 mg/kg - 170 mg/kg, 170 mg/kg - 175 mg/kg, 175 mg/kg - 180 mg/kg, 180 mg/kg - 185 mg/kg, 185 mg/kg - 190 mg/kg, 190 mg/kg - 195 mg/kg, 195 mg/kg - 200 mg/kg, 200 mg/kg - 250 mg/kg, 250 mg/kg - 300 mg/kg, 300 mg/kg - 350 mg/kg, 350 mg/kg - 400 mg/kg, 400 mg/kg - 450 mg/kg, or 450 mg/kg - 500 mg/kg.

### Combinational Therapy

In one embodiment, the agent described herein is used as a monotherapy. In one embodiment, the agents described herein can be used in combination with other known agents and therapies for treatment or prevention of food allergy. Administered "in combination," as used herein, means that two (or more) different treatments are delivered to the subject during the course of the subject's affliction with the disorder, e.g., the two or more treatments are delivered after the subject has been diagnosed with the disorder or disease (for example, food allergy) and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery." In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. The agents described herein and the at least one additional therapy can be administered simultaneously, in the same or in separate compositions, or sequentially. For sequential administration, the agent described herein can be administered first, and the additional agent can be administered second, or the order of administration can be reversed. The agent and/or other therapeutic agents, procedures or modalities can be administered during periods of active disorder, or during a period of remission or less active disease. The agent can be administered before another treatment, concurrently with the treatment, post-treatment, or during remission of the disorder.

Exemplary therapeutics used to treat or prevent a food allergy include, but are not limited to, Antihistamine, e.g., for the reduction or halting of an allergic reaction, Diphenhydramine (Benadryl, Banophen, Diphenhist, Wal-Dryl, and Nytol), Cetirizine (Zyrtec, Children's Cetirizine, Child Allergy Relf(cetirizine), All Day Allergy Relief(cetir), and Child's All Day Allergy(cetir)); Vasoconstrictor, e.g., for narrowing of blood vessels; Epinephrine (Adrenaclick, EpiPen, EpiPen Jr 2-Pak, Bronchial Mist Refill, and EPIsnap); and oral immunotherapy, e.g., Omalizumab (Xolair^{®}).

When administered in combination, the agent and the additional agent (e.g., second or third agent), or all, can be administered in an amount or dose that is higher, lower or the same as the amount or dosage of each agent used individually, e.g., as a monotherapy. In certain embodiments, the administered amount or dosage of the agent, the additional agent (e.g., second or third agent), or all, is lower (e.g., at least 20%, at least 30%, at least 40%, or at least 50%) than the amount or dosage of each agent used individually. In other embodiments, the amount or dosage of agent, the additional agent (e.g., second or third agent), or all, that results in a desired effect (e.g., treatment or prevention of a food allergy) is lower (e.g., at least 20%, at least 30%, at least 40%, or at least 50% lower) than the amount or dosage of each agent individually required to achieve the same therapeutic effect.

### Alternative Therapy

In some embodiments, for example if the subject does not have decreased RORγt in regulatory T cells, then an alternative therapy can be administered to the subject. Exemplary therapeutics used to treat or prevent a food allergy include, but are not limited to, Antihistamine, e.g., for the reduction or halting of an allergic reaction, Diphenhydramine (Benadryl, Banophen, Diphenhist, Wal-Dryl, and Nytol), Cetirizine (Zyrtec, Children's Cetirizine, Child Allergy Relf(cetirizine), All Day Allergy Relief(cetir), and Child's All Day Allergy(cetir)); Vasoconstrictor, e.g., for narrowing of blood vessels; Epinephrine (Adrenaclick, EpiPen, EpiPen Jr 2-Pak, Bronchial Mist Refill, and EPIsnap); and oral immunotherapy, e.g., Omalizumab (Xolair^{®}). Such therapeutics can be administered at an effective amount or a dosage as known in the art.

In some embodiments, if a subject does not have decreased RORγt in regulatory T cells, then an alternative therapy can be administered to the subject selected from the group consisting of: Antihistamine, e.g., for the reduction or halting of an allergic reaction, Diphenhydramine, Benadryl, Banophen, Diphenhist, Wal-Dryl, Nytol, Cetirizine, Zyrtec, Children's Cetirizine, Child Allergy Relf(cetirizine), All Day Allergy Relief(cetir), Child's All Day Allergy(cetir), Vasoconstrictor, Epinephrine, Adrenaclick, EpiPen, EpiPen Jr 2-Pak, Bronchial Mist Refill, EPIsnap, oral immunotherapy, and Omalizumab (Xolair^{®}).

In some embodiments, if the subject does not have decreased RORγt in regulatory T cells and the subject does not have, or is not at risk of developing, a food allergy, then no therapy is administered to the subject. In some embodiments, if the subject does not have, or is not at risk of developing, a food allergy, then no therapy is administered to the subject.

### Parenteral Dosage Forms

Parenteral dosage forms of an agents described herein can be administered to a subject by various routes, including, but not limited to, subcutaneous, intravenous (including bolus injection), intramuscular, and intra-arterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms of the disclosure are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

### Controlled and Delayed Release Dosage Forms

Conventional dosage forms generally provide rapid or immediate drug release from the formulation. Depending on the pharmacology and pharmacokinetics of the drug, use of conventional dosage forms can lead to wide fluctuations in the concentrations of the drug in a patient's blood and other tissues. These fluctuations can impact a number of parameters, such as dose frequency, onset of action, duration of efficacy, maintenance of therapeutic blood levels, toxicity, side effects, and the like. Advantageously, controlled-release formulations can be used to control a drug's onset of action, duration of action, plasma levels within the therapeutic window, and peak blood levels. In particular, controlled- or extended-release dosage forms or formulations can be used to ensure that the maximum effectiveness of a drug is achieved while minimizing potential adverse effects and safety concerns, which can occur both from under-dosing a drug (i.e., going below the minimum therapeutic levels) as well as exceeding the toxicity level for the drug. In some embodiments, the agent as described herein (e.g., I3S or CA) can be administered in a sustained release formulation.

In some embodiments of the aspects described herein, an agent is administered to a subject by controlled- or delayed-release means. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control a compound of formula (I)'s onset of action, duration of action, plasma levels within the therapeutic window, and peak blood levels. In particular, controlled- or extended-release dosage forms or formulations can be used to ensure that the maximum effectiveness of an agent is achieved while minimizing potential adverse effects and safety concerns, which can occur both from under-dosing a drug (i.e., going below the minimum therapeutic levels) as well as exceeding the toxicity level for the drug.

Most controlled-release formulations are designed to initially release an amount of drug (active ingredient) that promptly produces the desired therapeutic effect, and gradually and continually release other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, ionic strength, osmotic pressure, temperature, enzymes, water, and other physiological conditions or compounds.

A variety of known controlled- or extended-release dosage forms, formulations, and devices can be adapted for use with any agent described herein. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185, each of which is incorporated herein by reference in their entireties. These dosage forms can be used to provide slow or controlled-release of one or more active ingredients using, for example, hydroxypropylmethyl cellulose, other polymer matrices, gels, permeable membranes, osmotic systems (such as OROS^{®} (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, DUOLITE^{®} A568 and DUOLITE^{®} AP143 (Rohm&Haas, Spring House, Pa. USA).

### Efficacy

The efficacy of an agents described herein, e.g., for the treatment or prevention of a food allergy, can be determined by the skilled practitioner. However, a treatment is considered "effective treatment," as the term is used herein, if one or more of the signs or symptoms of, e.g., a food allergy, are altered in a beneficial manner, other clinically accepted symptoms are improved, or even ameliorated, or a desired response is induced e.g., by at least 10% following treatment according to the methods described herein. Efficacy can be assessed, for example, by measuring a marker, indicator, symptom, and/or the incidence of a condition treated according to the methods described herein or any other measurable parameter appropriate, e.g., decreased susceptibility to a food allergen. Efficacy can also be measured by a failure of an individual to worsen as assessed by hospitalization, or need for medical interventions (i.e., progression and/or severity of a food allergy). Methods of measuring these indicators are known to those of skill in the art and/or are described herein.

Treatment includes any treatment of a disease in an individual or an animal (some non-limiting examples include a human or an animal) and includes: (1) inhibiting the disease, e.g., preventing a worsening of symptoms (e.g. pain or inflammation); or (2) relieving the severity of the disease, e.g., causing regression of symptoms. An effective amount for the treatment of a disease means that amount which, when administered to a subject in need thereof, is sufficient to result in effective treatment as that term is defined herein, for that disease. Efficacy of an agent can be determined by assessing physical indicators of a condition or desired response, (e.g. increase of RORγt in a regulatory T cell). It is well within the ability of one skilled in the art to monitor efficacy of administration and/or treatment by measuring any one of such parameters, or any combination of parameters.

Efficacy can be assessed in animal models of a condition described herein, for example, a mouse model or an appropriate animal model of food allergy, as the case may be. When using an experimental animal model, efficacy of treatment is evidenced when a statistically significant change in a marker is observed, e.g., decreased susceptibility to a food allergen.

Efficacy of an agent that increases RORγt in a regulatory T cell can additionally be assessed using methods described herein.

### Vectors

In some embodiments, one or more of the genes described herein is expressed in a recombinant expression vector or plasmid. As used herein, the term "vector" refers to a polynucleotide sequence suitable for transferring transgenes into a host cell. The term "vector" includes plasmids, mini-chromosomes, phage, naked DNA and the like. See, for example, U.S. Pat. Nos. 4,980,285; 5,631,150; 5,707,828; 5,759,828; 5,888,783 and, 5,919,670, and, Sambrook et al., Molecular Cloning: A Laboratory Manual, 2nd Ed., Cold Spring Harbor Press (1989). One type of vector is a "plasmid," which refers to a circular double stranded DNA loop into which additional DNA segments are ligated. Another type of vector is a viral vector, wherein additional DNA segments are ligated into the viral genome. Certain vectors are capable of autonomous replication in a host cell into which they are introduced (e.g., bacterial vectors having a bacterial origin of replication and episomal mammalian vectors). Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" is used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

A cloning vector is one which is able to replicate autonomously or integrated in the genome in a host cell, and which is further characterized by one or more endonuclease restriction sites at which the vector may be cut in a determinable fashion and into which a desired DNA sequence can be ligated such that the new recombinant vector retains its ability to replicate in the host cell. In the case of plasmids, replication of the desired sequence can occur many times as the plasmid increases in copy number within the host cell such as a host bacterium or just a single time per host before the host reproduces by mitosis. In the case of phage, replication can occur actively during a lytic phase or passively during a lysogenic phase.

In some embodiments of any of the aspects, the vector or nucleic acid described herein is codon-optimized, e.g., the native or wild-type sequence of the nucleic acid sequence has been altered or engineered to include alternative codons such that altered or engineered nucleic acid encodes the same polypeptide expression product as the native/wild-type sequence, but will be transcribed and/or translated at an improved efficiency in a desired expression system. In some embodiments of any of the aspects, the expression system is an organism other than the source of the native/wild-type sequence (or a cell obtained from such organism). In some embodiments of any of the aspects, the vector and/or nucleic acid sequence described herein is codon-optimized for expression in a mammal or mammalian cell, e.g., a mouse, a murine cell, or a human cell. In some embodiments of any of the aspects, the vector and/or nucleic acid sequence described herein is codon-optimized for expression in a human cell. In some embodiments of any of the aspects, the vector and/or nucleic acid sequence described herein is codon-optimized for expression in a yeast or yeast cell. In some embodiments of any of the aspects, the vector and/or nucleic acid sequence described herein is codon-optimized for expression in a bacterial cell. In some embodiments of any of the aspects, the vector and/or nucleic acid sequence described herein is codon-optimized for expression in an *E. coli* cell.

As used herein, the term "expression vector" refers to a vector that directs expression of an RNA or polypeptide (e.g., a polypeptide encoding RORγt) from nucleic acid sequences contained therein linked to transcriptional regulatory sequences on the vector. The sequences expressed will often, but not necessarily, be heterologous to the cell. An expression vector may comprise additional elements, for example, the expression vector may have two replication systems, thus allowing it to be maintained in two organisms, for example in human cells for expression and in a prokaryotic host for cloning and amplification. The term "expression" refers to the cellular processes involved in producing RNA and proteins and as appropriate, secreting proteins, including where applicable, but not limited to, for example, transcription, transcript processing, translation and protein folding, modification and processing.

A vector can be integrating or non-integrating. "Integrating vectors" have their delivered RNA/DNA permanently incorporated into the host cell chromosomes. "Non-integrating vectors" remain episomal which means the nucleic acid contained therein is never integrated into the host cell chromosomes. Examples of integrating vectors include retroviral vectors, lentiviral vectors, hybrid adenoviral vectors, and herpes simplex viral vector.

One example of a non-integrative vector is a non-integrative viral vector. Non-integrative viral vectors eliminate the risks posed by integrative retroviruses, as they do not incorporate their genome into the host DNA. One example is the Epstein Barr oriP/Nuclear Antigen-1 ("EBNA1") vector, which is capable of limited self-replication and known to function in mammalian cells. As containing two elements from Epstein-Barr virus, oriP and EBNA1, binding of the EBNA1 protein to the virus replicon region oriP maintains a relatively long-term episomal presence of plasmids in mammalian cells. This particular feature of the oriP/EBNA1 vector makes it ideal for generation of integration-free iPSCs. Another non-integrative viral vector is adenoviral vector and the adeno-associated viral (AAV) vector.

Another non-integrative viral vector is RNA Sendai viral vector, which can produce protein without entering the nucleus of an infected cell. The F-deficient Sendai virus vector remains in the cytoplasm of infected cells for a few passages, but is diluted out quickly and completely lost after several passages (e.g., 10 passages).

Another example of a non-integrative vector is a minicircle vector. Minicircle vectors are circularized vectors in which the plasmid backbone has been released leaving only the eukaryotic promoter and cDNA(s) that are to be expressed.

An expression vector is one into which a desired DNA sequence can be inserted by restriction and ligation such that it is operably joined to regulatory sequences and can be expressed as an RNA transcript. Vectors can further contain one or more marker sequences suitable for use in the identification of cells which have or have not been transformed or transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase, luciferase or alkaline phosphatase), and genes which visibly affect the phenotype of transformed or transfected cells, hosts, colonies or plaques (e.g., green fluorescent protein). In certain embodiments, the vectors used herein are capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript can be translated into the desired protein or polypeptide.

When the nucleic acid molecule that encodes any of the polypeptides described herein is expressed in a cell, a variety of transcription control sequences (e.g., promoter/enhancer sequences) can be used to direct its expression. The promoter can be a native promoter, i.e., the promoter of the gene in its endogenous context, which provides normal regulation of expression of the gene. In some embodiments the promoter can be constitutive, i.e., the promoter is unregulated allowing for continual transcription of its associated gene. A variety of conditional promoters also can be used, such as promoters controlled by the presence or absence of a molecule.

The precise nature of the regulatory sequences needed for gene expression can vary between species or cell types, but in general can include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Regulatory sequences can also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA). That heterologous DNA (RNA) is placed under operable control of transcriptional elements to permit the expression of the heterologous DNA in the host cell.

In some embodiments, the vector is non-integrative or integrative.

In some embodiments, the vector is selected from the group consisting of an episomal vector, an EBNA1 vector, a minicircle vector, a non-integrative adenovirus, a non-integrative RNA, and a Sendai virus.

In some embodiments, the vector is a lentivirus vector.

Without limitations, the genes described herein can be included in one vector or separate vectors. For example, the RORγt gene can be included in a vector. For example, any one of SEQ ID NOs: 1-4 can be included in a vector.

In some embodiments, one or more of the recombinantly expressed gene can be integrated into the genome of the cell.

A nucleic acid molecule that encodes the enzyme of the claimed invention can be introduced into a cell or cells using methods and techniques that are standard in the art. For example, nucleic acid molecules can be introduced by standard protocols such as transformation including chemical transformation and electroporation, transduction, particle bombardment, etc. Expressing the nucleic acid molecule encoding the enzymes of the claimed invention also may be accomplished by integrating the nucleic acid molecule into the genome.

### Definitions

For convenience, the meaning of some terms and phrases used in the specification, examples, and appended claims, are provided below. Unless stated otherwise, or implicit from context, the following terms and phrases include the meanings provided below. The definitions are provided to aid in describing particular embodiments, and are not intended to limit the claimed technology, because the scope of the technology is limited only by the claims. Unless otherwise defined, all technical and scientific terms used herein have the same meaning as commonly understood by one of ordinary skill in the art to which this technology belongs. If there is an apparent discrepancy between the usage of a term in the art and its definition provided herein, the definition provided within the specification shall prevail. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737), the contents of which are all incorporated by reference herein in their entireties.

As used herein, the terms "treat," "treatment," "treating," or "amelioration" refer to therapeutic treatments, wherein the object is to reverse, alleviate, ameliorate, inhibit, slow down or stop the progression or severity of a condition associated with food allergies. The term "treating" includes reducing or alleviating at least one adverse effect or symptom of a food allergy (e.g., itching, gastric distress, or inflamed airway). Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (*i.e.*, not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

As used herein "preventing" or "prevention" refers to any methodology where the disease state or disorder (e.g., food allergy) does not occur due to the actions of the methodology (such as, for example, administration of an agent that induces a regulatory T cell that expresses RORγt, or a composition described herein). In one aspect, it is understood that prevention can also mean that the disease is not established to the extent that occurs in untreated controls. For example, there can be a 5, 10, 15, 20, 25, 30, 35, 40, 50, 60, 70, 80, 90, or 100% reduction in the establishment of disease frequency relative to untreated controls. Accordingly, prevention of a disease encompasses a reduction in the likelihood that a subject will develop the disease, relative to an untreated subject (e.g. a subject who is not treated with a composition comprising an agent as described herein).

As used herein, the term "food allergy" refers to a failure of oral tolerance to food antigens associated with Th2 immunity and allergen-specific IgE responses. That is, an immune response is generated in response to particular food antigens and can lead to hives, gastrointestinal symptoms, abdominal pain, anaphylaxis and even death.

As used herein, the term "tolerance" refers to the level of allergic response to a particular quantity of allergen.

As used herein, the term "antigen" refers to a substance or substances alone or in combination that when introduced into the lymphatic system induces production of antibodies that bind to a fraction of the molecule or molecules.

As used herein, the term "allergen" refers to a substance that could cause an allergic reaction in a patient.

As used herein, the term "allergic reaction" refers to any untoward response to an allergen.

As used herein, the term "administering," refers to the placement of a therapeutic (e.g., an agent that induces a regulatory T cell that expresses RORγt) or pharmaceutical composition as disclosed herein into a subject by a method or route which results in at least partial delivery of the agent to the subject. Pharmaceutical compositions comprising agents as disclosed herein can be administered by any appropriate route which results in an effective treatment in the subject.

As used herein, "transplanting" refers to the placement of fecal matter, e.g. from a healthy subject, as described herein into a subject, by a method or route which results in at least partial localization of the introduced fecal matter at a desired site, such as the intestines or a region thereof, such that a desired effect(s) is produced (e.g., tolerance to a food allergen). The fecal matter can be administered by any appropriate route which results in delivery to a desired location in the subject where at least a portion of the delivered fecal matter remain viable. The period of viability of the fecal matter after administration to a subject can be as short as a few hours, *e.g.,* twenty-four hours, to a few days, to as long as several years, *i.e.,* long-term engraftment. In some embodiments, the fecal matter transplant is administered in the form of a suppository, pill, capsule, or the like. In some embodiments, the fecal matter transplant is administered orally, rectally, or enterically. In some embodiments, the fecal matter transplant is administered in the form of a liquid or solution. In some embodiments, the fecal matter transplant is administered using a colonoscopy, enema, or a plastic tube inserted through the nose into the gastrointestinal tract (e.g., stomach or intestines).

As used herein, a "subject" means a human or animal. Usually the animal is a vertebrate such as a primate, rodent, domestic animal or game animal. Primates include, for example, chimpanzees, cynomologus monkeys, spider monkeys, and macaques, e.g., Rhesus. Rodents include, for example, mice, rats, woodchucks, ferrets, rabbits and hamsters. Domestic and game animals include, for example, cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In some embodiments, the subject is a mammal, e.g., a primate, e.g., a human. The terms, "individual," "patient" and "subject" are used interchangeably herein.

Preferably, the subject is a mammal. The mammal can be a human, non-human primate, mouse, rat, dog, cat, horse, or cow, but is not limited to these examples. Mammals other than humans can be advantageously used as subjects that represent animal models of disease e.g., food allergy. A subject can be male or female. A subject can be a newborn (e.g., birth to 2 months), an infant (e.g., 2 months to 1 year), a toddler (e.g., 1 year to 4 year), a child (e.g., less than 18 years of age), or an adult (e.g., greater than 18 years of age).

A subject can be one who has been previously diagnosed with or identified as suffering from or having a disease or disorder in need of treatment (e.g., a food allergy) or one or more complications related to such a disease or disorder, and optionally, have already undergone treatment for the disease or disorder or the one or more complications related to the disease or disorder. Alternatively, a subject can also be one who has not been previously diagnosed as having such disease or disorder (e.g., a food allergy) or related complications. For example, a subject can be one who exhibits one or more risk factors for the disease or disorder or one or more complications related to the disease or disorder or a subject who does not exhibit risk factors. A "subject in need" of treatment for a particular condition can be a subject having that condition, diagnosed as having that condition, or at risk of developing that condition.

The terms "upmodulate", "increase", "enhance", or "activate" are all used herein to mean an increase by a reproducible statistically significant amount. In some embodiments, the terms "upmodulate", "increase", "enhance", or "activate" can mean an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, a 20 fold increase, a 30 fold increase, a 40 fold increase, a 50 fold increase, a 6 fold increase, a 75 fold increase, a 100 fold increase, etc. or any increase between 2-fold and 10-fold or greater as compared to a reference level. In the context of a marker, an "increase" is a reproducible statistically significant increase in such level.

The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and non-proteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In certain embodiments, agents are small molecule having a chemical moiety. For example, chemical moieties included unsubstituted or substituted alkyl, aromatic, or heterocyclyl moieties including macrolides, leptomycins and related natural products or analogues thereof. Compounds can be known to have a desired activity and/or property, or can be selected from a library of diverse compounds.

The agent can be a molecule from one or more chemical classes, e.g., organic molecules, which may include organometallic molecules, inorganic molecules, genetic sequences, etc. Agents may also be fusion proteins from one or more proteins, chimeric proteins (for example domain switching or homologous recombination of functionally significant regions of related or different molecules), synthetic proteins or other protein variations including substitutions, deletions, insertion and other variants.

As used herein, the term "small molecule" refers to a chemical agent which can include, but is not limited to, a peptide, a peptidomimetic, an amino acid, an amino acid analog, a polynucleotide, a polynucleotide analog, an aptamer, a nucleotide, a nucleotide analog, an organic or inorganic compound (e.g., including heterorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

As used herein, the terms "protein" and "polypeptide" are used interchangeably herein to refer to a polymer of amino acids. A peptide is a relatively short polypeptide, typically between about 2 and 60 amino acids in length. Polypeptides used herein typically contain amino acids such as the 20 L-amino acids that are most commonly found in proteins. However, other amino acids and/or amino acid analogs known in the art can be used. One or more of the amino acids in a polypeptide may be modified, for example, by the addition of a chemical entity such as a carbohydrate group, a phosphate group, a fatty acid group, a linker for conjugation, functionalization, etc. A polypeptide that has a non-polypeptide moiety covalently or noncovalently associated therewith is still considered a "polypeptide." Exemplary modifications include glycosylation and palmitoylation. Polypeptides can be purified from natural sources, produced using recombinant DNA technology or synthesized through chemical means such as conventional solid phase peptide synthesis, etc.

In the various embodiments described herein, it is further contemplated that variants (naturally occurring or otherwise), alleles, homologs, conservatively modified variants, and/or conservative substitution variants of any of the particular polypeptides described are encompassed. As to amino acid sequences, one of skill will recognize that individual substitutions, deletions or additions to a nucleic acid, peptide, polypeptide, or protein sequence which alters a single amino acid or a small percentage of amino acids in the encoded sequence is a "conservatively modified variant" where the alteration results in the substitution of an amino acid with a chemically similar amino acid and retains the desired activity of the polypeptide. Such conservatively modified variants are in addition to and do not exclude polymorphic variants, interspecies homologs, and alleles consistent with the disclosure.

A given amino acid can be replaced by a residue having similar physiochemical characteristics, e.g., substituting one aliphatic residue for another (such as Ile, Val, Leu, or Ala for one another), or substitution of one polar residue for another (such as between Lys and Arg; Glu and Asp; or Gln and Asn). Other such conservative substitutions, e.g., substitutions of entire regions having similar hydrophobicity characteristics, are well known. Polypeptides comprising conservative amino acid substitutions can be tested in any one of the assays described herein to confirm that a desired activity, e.g. activity and specificity of a native or reference polypeptide is retained.

Amino acids can be grouped according to similarities in the properties of their side chains (in A. L. Lehninger, in Biochemistry, second ed., pp. 73-75, Worth Publishers, New York (1975)): (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); (4) basic: Lys (K), Arg (R), His (H). Alternatively, naturally occurring residues can be divided into groups based on common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; (6) aromatic: Trp, Tyr, Phe. Non-conservative substitutions will entail exchanging a member of one of these classes for another class. Particular conservative substitutions include, for example; Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.

In some embodiments, the polypeptide described herein (or a nucleic acid encoding such a polypeptide) can be a functional fragment of one of the amino acid sequences described herein. As used herein, a "functional fragment" is a fragment or segment of a peptide which retains at least 50% of the wild-type reference polypeptide's activity according to the assays described below herein. A functional fragment can comprise conservative substitutions of the sequences disclosed herein.

In some embodiments, the polypeptide described herein can be a variant of a sequence described herein. In some embodiments, the variant is a conservatively modified variant. Conservative substitution variants can be obtained by mutations of native nucleotide sequences, for example. A "variant," as referred to herein, is a polypeptide substantially homologous to a native or reference polypeptide, but which has an amino acid sequence different from that of the native or reference polypeptide because of one or a plurality of deletions, insertions or substitutions. Variant polypeptide-encoding DNA sequences encompass sequences that comprise one or more additions, deletions, or substitutions of nucleotides when compared to a native or reference DNA sequence, but that encode a variant protein or fragment thereof that retains activity. A wide variety of PCR-based site-specific mutagenesis approaches are known in the art and can be applied by the ordinarily skilled artisan.

A variant amino acid or DNA sequence can be at least 90%, at least 91%, at least 92%, at least 93%, at least 94%, at least 95%, at least 96%, at least 97%, at least 98%, at least 99%, or more, identical to a native or reference sequence. The degree of homology (percent identity) between a native and a mutant sequence can be determined, for example, by comparing the two sequences using freely available computer programs commonly employed for this purpose on the world wide web (e.g. BLASTp or BLASTn with default settings).

Alterations of the native amino acid sequence can be accomplished by any of a number of techniques known to one of skill in the art. Mutations can be introduced, for example, at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered nucleotide sequence having particular codons altered according to the substitution, deletion, or insertion required. Techniques for making such alterations are very well established and include, for example, those disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); and U.S. Pat. Nos. 4,518,584 and 4,737,462, which are herein incorporated by reference in their entireties. Any cysteine residue not involved in maintaining the proper conformation of the polypeptide also can be substituted, generally with serine, to improve the oxidative stability of the molecule and prevent aberrant crosslinking. Conversely, cysteine bond(s) can be added to the polypeptide to improve its stability or facilitate oligomerization.

As used herein, the term "nucleic acid" or "nucleic acid sequence" refers to any molecule, preferably a polymeric molecule, incorporating units of ribonucleic acid, deoxyribonucleic acid or an analog thereof. The nucleic acid can be either single-stranded or double-stranded. A single-stranded nucleic acid can be one nucleic acid strand of a denatured double- stranded DNA. Alternatively, it can be a single-stranded nucleic acid not derived from any double-stranded DNA. In one aspect, the nucleic acid can be DNA. In another aspect, the nucleic acid can be RNA. Suitable DNA can include, e.g., genomic DNA or cDNA. Suitable RNA can include, e.g., mRNA.

The term "expression" refers to the cellular processes involved in producing RNA and proteins and as appropriate, secreting proteins, including where applicable, but not limited to, for example, transcription, transcript processing, translation and protein folding, modification and processing. Expression can refer to the transcription and stable accumulation of sense (mRNA) or antisense RNA derived from a nucleic acid fragment or fragments of the invention and/or to the translation of mRNA into a polypeptide.

In some embodiments, the expression of a biomarker(s), target(s), or gene/polypeptide described herein is/are tissue-specific. In some embodiments, the expression of a biomarker(s), target(s), or gene/polypeptide described herein is/are global. In some embodiments, the expression of a biomarker(s), target(s), or gene/polypeptide described herein is systemic.

"Expression products" include RNA transcribed from a gene, and polypeptides obtained by translation of mRNA transcribed from a gene. The term "gene" means the nucleic acid sequence which is transcribed (DNA) to RNA in vitro or in vivo when operably linked to appropriate regulatory sequences. The gene may or may not include regions preceding and following the coding region, e.g. 5' untranslated (5'UTR) or "leader" sequences and 3' UTR or "trailer" sequences, as well as intervening sequences (introns) between individual coding segments (exons).

"Marker" in the context of the present invention refers to an expression product, e.g., nucleic acid or polypeptide which is differentially present in a sample taken from subjects having a food allergy, as compared to a comparable sample taken from control subjects (e.g., a healthy subject). The term "biomarker" is used interchangeably with the term "marker."

In some embodiments, the methods described herein relate to measuring, detecting, or determining the level of at least one marker. As used herein, the term "detecting" or "measuring" refers to observing a signal from, e.g. a probe, label, or target molecule to indicate the presence of an analyte in a sample. Any method known in the art for detecting a particular label moiety can be used for detection. Exemplary detection methods include, but are not limited to, spectroscopic, fluorescent, photochemical, biochemical, immunochemical, electrical, optical or chemical methods. In some embodiments of any of the aspects, measuring can be a quantitative observation.

In some embodiments of any of the aspects, a polypeptide, nucleic acid, or cell as described herein can be engineered. As used herein, "engineered" refers to the aspect of having been manipulated by the hand of man. For example, a polypeptide is considered to be "engineered" when at least one aspect of the polypeptide, e.g., its sequence, has been manipulated by the hand of man to differ from the aspect as it exists in nature. As is common practice and is understood by those in the art, progeny of an engineered cell are typically still referred to as "engineered" even though the actual manipulation was performed on a prior entity.

In some embodiments of any of the aspects, the agent described herein (e.g., I3C) is exogenous. In some embodiments of any of the aspects, the agent described herein (e.g., I3C) is ectopic. In some embodiments of any of the aspects, the agent described herein (e.g., I3C) is not endogenous.

The term "exogenous" refers to a substance present in a cell other than its native source. The term "exogenous" when used herein can refer to a nucleic acid (e.g. a nucleic acid encoding a polypeptide) or a polypeptide that has been introduced by a process involving the hand of man into a biological system such as a cell or organism in which it is not normally found and one wishes to introduce the nucleic acid or polypeptide into such a cell or organism. Alternatively, "exogenous" can refer to a nucleic acid or a polypeptide that has been introduced by a process involving the hand of man into a biological system such as a cell or organism in which it is found in relatively low amounts and one wishes to increase the amount of the nucleic acid or polypeptide in the cell or organism, e.g., to create ectopic expression or levels. In contrast, the term "endogenous" refers to a substance that is native to the biological system or cell. As used herein, "ectopic" refers to a substance that is found in an unusual location and/or amount. An ectopic substance can be one that is normally found in a given cell, but at a much lower amount and/or at a different time. Ectopic also includes substance, such as a polypeptide or nucleic acid that is not naturally found or expressed in a given cell in its natural environment.

The term "vector", as used herein, refers to a nucleic acid construct designed for delivery to a host cell or for transfer between different host cells. As used herein, a vector can be viral or non-viral. The term "vector" encompasses any genetic element that is capable of replication when associated with the proper control elements and that can transfer gene sequences to cells. A vector can include, but is not limited to, a cloning vector, an expression vector, a plasmid, phage, transposon, cosmid, artificial chromosome, virus, virion, etc.

As used herein, the term "viral vector" refers to a nucleic acid vector construct that includes at least one element of viral origin and has the capacity to be packaged into a viral vector particle. The viral vector can contain a nucleic acid encoding a polypeptide as described herein in place of non-essential viral genes. The vector and/or particle may be utilized for the purpose of transferring nucleic acids into cells either *in vitro* or *in vivo*. Numerous forms of viral vectors are known in the art.

The term "decrease", "reduced", "reduction", or "inhibit" are all used herein to mean a decrease by a statistically significant amount. In some embodiments, "decrease", "reduced", "reduction", or "inhibit" typically means a decrease by at least 10% as compared to an appropriate control (e.g. the absence of a given treatment) and can include, for example, a decrease by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , or more. As used herein, "reduction" or "inhibition" does not encompass a complete inhibition or reduction as compared to a reference level. "Complete inhibition" is a 100% inhibition as compared to an appropriate control.

The terms "increase", "enhance", or "activate" are all used herein to mean an increase by a reproducible statistically significant amount. In some embodiments, the terms "increase", "enhance", or "activate" can mean an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, a 20 fold increase, a 30 fold increase, a 40 fold increase, a 50 fold increase, a 6 fold increase, a 75 fold increase, a 100 fold increase, etc. or any increase between 2-fold and 10-fold or greater as compared to an appropriate control. In the context of a marker, an "increase" is a reproducible statistically significant increase in such level.

As used herein, a "reference level" refers to a normal, otherwise unaffected cell population or tissue (e.g., a biological sample obtained from a healthy subject, or a biological sample obtained from the subject at a prior time point, e.g., a biological sample obtained from a patient prior to being diagnosed with a food allergy, or a biological sample that has not been contacted with an agent disclosed herein).

As used herein, an "appropriate control" refers to an untreated, otherwise identical cell, population, or healthy subject (e.g., a patient or cell that was not administered an agent described herein, as compared to a patient with a food allergy or a cell treated with an agent as described herein).

As used herein, "contacting" refers to any suitable means for delivering, or exposing, an agent to at least one cell. Exemplary delivery methods include, but are not limited to, direct delivery to cell culture medium, perfusion, injection, or other delivery method well known to one skilled in the art. In some embodiments, contacting comprises physical human activity, e.g., an injection; an act of dispensing, mixing, and/or decanting; and/or manipulation of a delivery device or machine.

The term "statistically significant" or "significantly" refers to statistical significance and generally means a two standard deviation (2SD) or greater difference.

Other than in the operating examples, or where otherwise indicated, all numbers expressing quantities of ingredients or reaction conditions used herein should be understood as modified in all instances by the term "about." The term "about" when used in connection with percentages can mean ±1%.

As used herein the term "comprising" or "comprises" is used in reference to compositions, methods, and respective component(s) thereof, that are essential to the method or composition, yet open to the inclusion of unspecified elements, whether essential or not.

The term "consisting of" refers to compositions, methods, and respective components thereof as described herein, which are exclusive of any element not recited in that description of the embodiment.

As used herein the term "consisting essentially of" refers to those elements required for a given embodiment. The term permits the presence of additional elements that do not materially affect the basic and novel or functional characteristic(s) of that embodiment of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

All patents, and other publications; including literature references, issued patents, published patent applications, and co-pending patent applications; cited throughout this application are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein or agent structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

The technology described herein is further illustrated by the following examples which in no way should be construed as being further limiting.

Some embodiments of the technology described herein can be defined according to any of the following numbered paragraphs:
1. A method for treating or preventing the onset of a food allergy in a subject, the method comprising: administering to a subject an agent that induces a regulatory T cell, or population thereof, that expresses RORyt.
2. The method of paragraph 1, further comprising, prior to administration, diagnosing a subject as having, or likely to develop, a food allergy.
3. The method of any one of paragraphs 1-2, further comprising, prior to administration, receiving the results of an assay that diagnoses a subject as having, or likely to develop, a food allergy.
4. The method of any one of paragraphs 1-3, wherein the subject is selected from the group consisting of: a newborn, an infant, a toddler, a child, and an adult.
5. The method of any one of paragraphs 1-4, wherein the agent is administered prior to the first exposure to a potential food allergen.
6. The method of any one of paragraphs 1-5, wherein the agent is administered upon clinical signs of atopic symptoms.
7. The method of any one of paragraphs 1-6, wherein the subject has been diagnosed with at least one food allergy.
8. The method of any one of paragraphs 1-7, wherein the food allergy comprises allergy to soy, wheat, eggs, dairy, peanuts, tree nuts, shellfish, fish, mushrooms, stone fruits and other fruits.
9. The method of any one of paragraphs 1-8, wherein the agent is selected from the group consisting of: a small molecule, a compound, an antibody, a peptide, and an expression vector encoding RORyt.
10. The method of any one of paragraphs 1-9, wherein the vector is non-integrative or integrative.
11. The method of any one of paragraphs 1-10, wherein the non-integrative vector is selected from the group consisting of an episomal vector, an EBNA1 vector, a minicircle vector, a non-integrative adenovirus, a non-integrative RNA, and a Sendai virus.
12. The method of any one of paragraphs 1-11, wherein the vector is a lentivirus vector.
13. The method of any one of paragraphs 1-12, wherein the compound is indoxyl 3 sulfate (I3S) or indole-3-carboxaldehyde (CA).
14. The method of any one of paragraphs 1-13, wherein the agent prevents and/or reverses Th2 programming of Tregs and other mucosal T cell populations.
15. The method of any one of paragraphs 1-14, wherein the regulatory T cell expressing RORyt has low expression of the Helios marker as compared to a regulatory T cell that does not express RORyt.
16. The method of any one of paragraphs 1-15, wherein the expression of RORγt is increased by at least 2x, 3x, 4x, 5x, 6x, 7x, 8x, 9x, or more as compared to an appropriate control.
17. The method of any one of paragraphs 1-16, wherein the agent upmodulates the expression of RORyt on a regulatory T cell, or population thereof.
18. A method for treating or preventing the onset of a food allergy in a subject, the method comprising: administering to a subject an agent that induces a regulatory T cell, or population thereof, that expresses RORyt, wherein the agent is I3S or CA.
19. A method for inducing tolerance in a subject, the method comprising: administering to a subject an agent that induces a regulatory T cell, or population thereof, that expresses RORyt, wherein the agent is I3S or CA.
20. A method for reducing or eliminating a subject's immune reaction to a food allergen, the method comprising: administering to a subject an agent that induces a regulatory T cell, or population thereof, that expresses RORyt, thereby reducing or eliminating a subject's immune reaction to a food allergen.
21. A method for treating or preventing the onset of a food allergy in a subject, the method comprising:
   a. measuring a level of RORyt on regulatory T cells in a subject;
   b. comparing the level of RORyt from (a) to the level of level of RORyt on regulatory T cells in a healthy subject;
   c. identifying a subject as having decreased RORyt on regulatory T cells if the level from step (b) is statistically lower than the level in a healthy subject; and
   d. if the subject has decreased RORyt on regulatory T cells, then administering an agent that induces regulatory T cells that express RORyt to the subject.
22. The method of paragraph 21, further comprising, prior to step (a), isolating regulatory T cells from the subject.
23. A method for treating or preventing the onset of a food allergy in a subject, the method comprising:
   a. receiving the results of an assay that determines if the subject has decreased levels of RORyt on regulatory T cells; and
   b. if the subject has decreased RORyt on regulatory T cells, then administering an agent that induces regulatory T cells that express RORyt to the subject.
24. A composition comprising an agent that induces a regulatory T cell, or population thereof, that expresses RORyt.
25. The composition of paragraph 23, further comprising a pharmaceutically acceptable carrier.
26. Use of the composition of paragraphs 23-24 for the treatment or prevention of a food allergy in a subject having or likely to develop a food allergy.
27. Use of the composition of paragraphs 23-24 for reducing or eliminating a subject's immune reaction to a food allergen.
28. A method of identifying a microorganism that induces a regulatory T cell, or a population thereof, that expresses RORyt, the method comprising:
   a. introducing at least one microorganism to the germ-free mouse model for a time sufficient to allow for the colonization the at least one microorganism in the gut of the germ-free mouse model;
   b. measuring a level of expression of RORyt on regulatory T cells in the germ-free mouse model; and
   c. identifying the at least one microorganism as an inducer of a regulatory T cell, or a population thereof, that expresses RORyt if the level of expression from step (b) is greater than zero.
29. A method for treating or preventing the onset of a food allergy in a subject, the method comprising:
   a. measuring a level of RORyt on regulatory T cells in a subject;
   b. comparing the level of RORyt from (a) to the level of level of RORyt on regulatory T cells in a healthy subject;
   c. identifying a subject as having decreased RORyt on regulatory T cells if the level from step (a) is statistically lower than the level in a healthy subject; and
   d. if the subject has decreased RORyt on regulatory T cells, then administering a fecal matter transplant to the subject,
   wherein the fecal matter is obtained from a healthy subject.
30. The method of paragraph 28, further comprising, prior to step (a), isolating regulatory T cells from the subject.
31. A method for treating or preventing the onset of a food allergy in a subject, the method comprising:
   a. receiving the results of an assay that determines if the subject has decreased levels of RORyt on regulatory T cells; and
   b. if the subject has decreased RORyt on regulatory T cells, then administering a fecal matter transplant to the subject,
   wherein the fecal matter is obtained from a healthy subject.

Some embodiments of the technology described herein can be defined according to any of the following numbered paragraphs:
1) A method for treating or preventing the onset of a food allergy in a subject, the method comprising: administering to a subject an agent that induces a regulatory T cell, or population thereof, that expresses RORyt.
2) The method of paragraph 1, further comprising, prior to administration, diagnosing a subject as having, or likely to develop, a food allergy.
3) The method of any one of paragraphs 1-2, further comprising, prior to administration, receiving the results of an assay that diagnoses a subject as having, or likely to develop, a food allergy.
4) The method of any one of paragraphs 1-3, wherein the subject is selected from the group consisting of: a newborn, an infant, a toddler, a child, and an adult.
5) The method of any one of paragraphs 1-4, wherein the agent is administered prior to the first exposure to a potential food allergen.
6) The method of any one of paragraphs 1-5, wherein the agent is administered upon clinical signs of atopic symptoms.
7) The method of any one of paragraphs 1-6, wherein the subject has been diagnosed with at least one food allergy.
8) The method of any one of paragraphs 1-7, wherein the food allergy comprises allergy to soy, wheat, eggs, dairy, peanuts, tree nuts, shellfish, fish, mushrooms, stone fruits and other fruits.
9) The method of any one of paragraphs 1-8, wherein the agent is selected from the group consisting of: a small molecule, a compound, an antibody, a peptide, and an expression vector encoding RORyt.
10) The method of any one of paragraphs 1-9, wherein the vector is non-integrative or integrative.
11) The method of any one of paragraphs 1-10, wherein the non-integrative vector is selected from the group consisting of an episomal vector, an EBNA1 vector, a minicircle vector, a non-integrative adenovirus, a non-integrative RNA, and a Sendai virus.
12) The method of any one of paragraphs 1-11, wherein the vector is a lentivirus vector.
13) The method of any one of paragraphs 1-12, wherein the compound is indoxyl 3 sulfate (I3S).
14) The method of any one of paragraphs 1-3, wherein the compound is indole-3-carboxaldehyde (CA).
15) The method of any one of paragraphs 1-14, wherein the agent prevents and/or reverses Th2 programming of Tregs and other mucosal T cell populations.
16) The method of any one of paragraphs 1-15, wherein the regulatory T cell expressing RORyt has low expression of the Helios marker as compared to a regulatory T cell that does not express RORyt.
17) The method of any one of paragraphs 1-16, wherein the expression of RORγt is increased by at least 2x, 3x, 4x, 5x, 6x, 7x, 8x, 9x, or more as compared to an appropriate control.
18) The method of any one of paragraphs 1-17, wherein the agent upmodulates the expression of RORyt in a regulatory T cell, or population thereof.
19) A method for treating or preventing the onset of a food allergy in a subject, the method comprising: administering to a subject an agent that induces a regulatory T cell, or population thereof, that expresses RORyt, wherein the agent is I3S or CA.
20) A method for inducing tolerance in a subject, the method comprising: administering to a subject an agent that induces a regulatory T cell, or population thereof, that expresses RORyt, wherein the agent is I3S or CA.
21) A method for reducing or eliminating a subject's immune reaction to a food allergen, the method comprising: administering to a subject an agent that induces a regulatory T cell, or population thereof, that expresses RORyt, thereby reducing or eliminating a subject's immune reaction to a food allergen.
22) A method for treating or preventing the onset of a food allergy in a subject, the method comprising:
   a. measuring a level of RORyt in regulatory T cells in a subject;
   b. comparing the level of RORyt from (a) to the level of level of RORyt in regulatory T cells in a healthy subject;
   c. identifying a subject as having decreased RORyt in regulatory T cells if the level from step (b) is statistically lower than the level in a healthy subject; and
   d. if the subject has decreased RORyt in regulatory T cells, then administering an agent that induces regulatory T cells that express RORyt to the subject.
23) The method of paragraph 22, further comprising, prior to step (a), isolating regulatory T cells from the subject.
24) A method for treating or preventing the onset of a food allergy in a subject, the method comprising:
   a. measuring a level of RORyt in regulatory T cells in a subject;
   b. comparing the level of RORyt from (a) to the level of level of RORyt in regulatory T cells in a healthy subject;
   c. identifying a subject as having decreased RORyt in regulatory T cells if the level from step (b) is statistically lower than the level in a healthy subject;
   d. identifying whether the subject has, or is likely to develop, a food allergy; and
   e. if the subject has decreased RORyt in regulatory T cells and has, or is likely to develop, a food allergy, then administering an agent that induces regulatory T cells that express RORyt to the subject.
25) The method of paragraph 24, further comprising, prior to step (a), isolating regulatory T cells from the subject.
26) A method for treating or preventing the onset of a food allergy in a subject, the method comprising:
   a. receiving the results of an assay that determines if the subject has decreased levels of RORyt in regulatory T cells; and
   b. if the subject has decreased RORyt in regulatory T cells, then administering an agent that induces regulatory T cells that express RORyt to the subject.
27) A method for treating or preventing the onset of a food allergy in a subject, the method comprising:
   a. receiving the results of an assay that determines if the subject has decreased levels of RORyt in regulatory T cells;
   b. receiving the results of an assay that determines if the subject has, or is likely to develop, a food allergy; and
   c. if the subject has decreased RORyt in regulatory T cells and has, or is likely to develop, a food allergy, then administering an agent that induces regulatory T cells that express RORyt to the subject.
28) A composition comprising an agent that induces a regulatory T cell, or population thereof, that expresses RORyt.
29) The composition of paragraph 28, wherein the agent is selected from the group consisting of: a small molecule, a compound, an antibody, a peptide, and an expression vector encoding RORγt.
30) The composition of any one of paragraphs 28-29, wherein the compound is indoxyl 3 sulfate (I3S).
31) The composition of any one of paragraphs 28-30, wherein the compound is indole-3-carboxaldehyde (CA).
32) The composition of any one of paragraphs 28-31, further comprising a pharmaceutically acceptable carrier.
33) Use of the composition of paragraphs 28-32 for the treatment or prevention of a food allergy in a subject having or likely to develop a food allergy.
34) Use of the composition of paragraphs 28-32 for reducing or eliminating a subject's immune reaction to a food allergen.
35) A method of identifying a microorganism that induces a regulatory T cell, or a population thereof, that expresses RORyt, the method comprising:
   a. introducing at least one microorganism to a subject for a time sufficient to allow for the colonization of the at least one microorganism in the gut of the subject;
   b. measuring a level of expression of RORyt in regulatory T cells in the subject; and
   c. identifying the at least one microorganism as an inducer of a regulatory T cell, or a population thereof, that expresses RORyt if the level of expression from step (b) is greater than zero.
36) The method of paragraph 35, wherein the subject comprises a germ-free mouse model.
37) A method for treating or preventing the onset of a food allergy in a subject, the method comprising:
   a. measuring a level of RORyt in regulatory T cells in a subject;
   b. comparing the level of RORyt from (a) to the level of level of RORyt in regulatory T cells in a healthy subject;
   c. identifying a subject as having decreased RORyt in regulatory T cells if the level from step (a) is statistically lower than the level in a healthy subject; and
   d. if the subject has decreased RORyt in regulatory T cells, then administering a fecal matter transplant to the subject,
   wherein the fecal matter is obtained from a healthy subject.
38) The method of paragraph 37, further comprising, prior to step (a), isolating regulatory T cells from the subject.
39) A method for treating or preventing the onset of a food allergy in a subject, the method comprising:
   a. receiving the results of an assay that determines if the subject has decreased levels of RORyt in regulatory T cells; and
   b. if the subject has decreased RORyt in regulatory T cells, then administering a fecal matter transplant to the subject,
   wherein the fecal matter is obtained from a healthy subject.

### EXAMPLES

### Example 1

Food allergy (FA) is a major public health concern, whose prevalence has grown dramatically over the past decade. FA now affects 6% of children under 5 years, and 3% of teens and adults. Most FA is acquired in the first or second year of life, indicating that early childhood exposures have profound long-term health consequences. Without wishing to be bound by theory, it is noted that the hygiene hypothesis stipulates that microbial exposures play a critical role in the development of protection against allergic diseases, and that alterations in those exposures, including changes in the host microbial flora, can underlie the rise in allergic diseases. In that regard, it has been shown that factors impacting gut microbial colonization and composition early in life, including method of delivery (e.g., cesarean section), antibiotic use, and breastfeeding influence the development of atopic disease. Less information is available on the role of gut microbiota in human FA. Reduced gut microbiota diversity and an elevated ratio of the abundance of *Enterobacteriaceae* to *Bacteroidaceae* species in early infancy have been associated with subsequent food sensitization, indicating that the initial stages of gut colonization with particular microbial communities can contribute to the development of atopic disease, including FA.

It has been shown that the presence and composition of the gut microbiota influences the host's susceptibility to FA. Mice raised in a sterile environment cannot be tolerized to antigens given orally, have reduced IgA levels and IL-10 producing regulatory T (Treg) cells. In contrast, colonization with Segmented Filamentous Bacteria (SFB) and Clostridia species promotes the development of IL-17 producing T cells and Treg cells, respectively. It was found that in a FA-prone genetic mouse model (*Il4ra*^{F709} mice), the acquisition of FA is associated with a gut microbiota signature that is distinct from that of FA-tolerant mice. Furthermore, transfer of fecal microbiota from FA but not tolerant mice to germ-free (GF) recipients transmitted susceptibility to FA. It has been found that sensitization to a food allergen was increased in mice that have been treated with antibiotics or were devoid of commensal microbiota. By selectively colonizing gnotobiotic mice, allergy-protective capacity was conferred by a Clostridia-predominant microbiota.

These findings indicate that unfavorable alterations in the development of the gut microbiota early in life favor the emergence of dysbiotic communities with concomitant reductions in beneficial species. In combination, such changes can result in a failure to promote tolerant immune responses, thus raising the host's susceptibility to allergic and inflammatory responses. Without wishing to be bound by theory, mechanisms by which the commensal microbiota may promote oral tolerance to food allergens include their enhancement of epithelial cell barrier integrity and elicitation of protective mucosal Treg cell responses. The production of short-chain fatty acids, such as acetate, propionate and butyrate, by commensals such as Clostridia species, reinforce mucosal tolerance by recruiting and stabilizing Treg cells in the gut. Colonization with commensal bacteria also expands a population of induced Treg (iTreg) cells in the gut that expresses the transcription factor retinoic acid orphan receptor-gamma (ROR-γt) (see e.g., Ohnmacht, C., et al. MUCOSAL IMMUNOLOGY, The microbiota regulates type 2 immunity through RORgammat(+) T cells, Science 349, 989-993 (2015); Sefik, E., et al. MUCOSAL IMMUNOLOGY, Individual intestinal symbionts induce a distinct population of RORgamma(+) regulatory T cells, Science 349, 993-997 (2015); the content of each of which are incorporated herein by reference in their entirety).. Without wishing to be bound by theory, it has been proposed that ROR-γt iTreg cells can regulate allergic inflammation by inhibiting Th2 cell responses in the gut via a CTLA-4-dependent mechanism (see e.g., Ohnmacht (2015) *supra*).

### Results

Patients with FA manifest early onset dynamic gut dysbiosis. Without wishing to be bound by a particular theory, it was hypothesized that food allergy early in life is associated with gut microbial dysbiosis, which was explored by analyzing the fecal microbiota of 56 food allergic (FA) and 98 age matched control infants recruited at 1-15 months of age and periodically sampled every 4-6 months for up to 30 months of age. Table 1 summarizes subject demographics, and FIG. 7A summarizes the distribution of samples collected by subject age. Analysis of the ecological diversity of the fecal microbiota revealed no significant difference between those of food allergic versus control subjects, as assessed by alpha and beta diversity measures (see e.g., FIG. 7B and 7C). However, differences in the fecal microbiota were observed at the level of bacterial taxa, with relative abundances of 77 Operational Taxonomic Units (OTUs) differing significantly between age-stratified food allergic subjects and controls [False discovery rate (FDR)-adjusted p-value <0.1] (see e.g., FIG. 1A-1D, and data not shown). Some taxa were significantly different between FA and controls across more than one age group, while others were altered only in specific age groups. The dysbiotic associations in FA patients occurred even when controlling for factors, including gender, mode of delivery for all age groups, and breastfeeding until 18 months of age, using multivariate statistical models including both taxa abundance and potential confounding variables.

Several taxa were found increased in FA subjects across multiple age windows, with the earliest alterations found in closest reference species (CRS) *Bilophila wadsworthia* and *Clostridium disporicum,* followed by CRS *Parasutterella excrementihominis, Veillonella ratti, Bacteroides stercoris, Alistipes onderdonkii* and *Prevotella copri,* and *Roseburia inulinivorans* (see e.g., FIG. 1A-1D, and data not shown). Some taxa that were initially more abundant in younger FA subjects became more so in control subjects as age increased (e.g. CRS *Subdoligranulum variabile*). Others were initially more abundant in the younger control group and became more abundant in the older FA cohort (e.g. CRS *Bacteroides acidifaciens*).

In addition to the aforementioned changes that were noted across several age groups, age-specific differences emerged in the microbiota of FA versus control subjects. Some of the earliest changes involved increased relative abundance of several taxa in FA infants, including CRS *Clostridium aldenense, Clostridium lentocellulum, Peptostreptococcus anaerobius*/*stomatis* and *Lactobacillus johnsonii* (1-6 months of age), and CRS *Faecalibacterium prausnitzii, Blautia wexlerae, Anaerostipes caccae,* and *Lactobacillus rossiae* (7-12 months of age). Older FA subjects showed decrease in several Clostridiales taxa, including CRS *Clostridium hathewayi, Clostridium symbiosum, Clostridium lavalense,* and *Clostridium scindens.*

As cow's milk is an important food staple in childhood, and because milk avoidance due to allergy could represent a confounder that influences the composition of the microbiota, the gut microbiota of control subjects who were consuming milk products were compared to that of FA patients who were tolerant and consuming milk but allergic to other food(s). In total, 61 differentially abundant OTUs were identified that strongly overlapped with those identified in FA subjects not segregated for milk allergy (FDR-adjusted p-value <0.1), with 16 detected across more than one age group (see e.g., FIG. 2A-2I, and data not shown). Overall, these results indicated that dysbiosis was a general attribute of food allergy early in life.

To assess the functional significance of dysbiosis in FA, *Il4ra*^{F709} mice were employed. *Il4ra*^{F709} mice are genetically prone to develop FA upon oral sensitization with food allergens. Adult germ-free (GF) *Il4ra*^{F709} mice that were either left un-reconstituted or that received fecal matter transplants (FMT) from HC or FA infants were sensitized with chicken egg ovalbumin (OVA) in the presence of the mucosal adjuvant staphylococcal enterotoxin B (SEB) and subsequently challenged with OVA (see e.g., Noval Rivas et al., J Allergy Clin Immunol 131, 201-212 (2013); Fagarasan et al. Science 298, 1424-1427 (2002); Kubinak et al. Cell Host Microbe 17, 153-163 (2015); Wang et al. Immunity 43, 289-303 (2015); the content of each of which are incorporated by reference herein in their entirety). GF *Il4ra*^{F709} mice or those that received FMT from FA subjects exhibited a rapid and sustained drop in their core body temperature, consistent with anaphylaxis, whereas those that received FMT from HC had a mild drop that rapidly reversed (see e.g., FIG. 1E). Furthermore, and whereas the total serum IgE concentrations in the three OVA-sensitized mouse groups were similar, induction of OVA-specific IgE was markedly decreased in mice that received FMT from HC subjects as compared to those that received FMT from FA subjects or were GF (see e.g., FIG. 1F). Also, the increase in serum mouse mast cell protease 1 (MMCP1) concentrations post anaphylaxis was notably higher in those *Il4ra*^{F709} mice that were GF or that have received FMT from FA subjects as compared to mice that received FMT from healthy subjects (see e.g., FIG. 1G). These results indicated that the capacity of the gut commensal flora to impart protection against FA was profoundly impaired in FA as compared to HC subjects.

Similar to FA human subjects, *Il4ra*^{F709} mice also exhibit dysbiotic microbiota which, upon transfer to germ free BALB/c mice heightens their susceptibility to food allergy induction. The capacity of microbiota derived from specific pathogen-free (SPF) WT BALB/c mice were analyzed, which are relatively resistant to FA induction, to rescue the FA phenotype of *Il4ra*^{F709} mice. GF *Il4ra*^{F709} mice that were reconstituted with FMT from WT BALB/c mice then sensitized with OVA/SEB and challenged with OVA were resistant to the induction of FA, while those that were reconstituted with microbiota derived from SPF *Il4ra*^{F709} mice developed disease, as evidenced by a precipitous drop in core body temperature and increased serum MMCP1 concentrations upon oral challenge with OVA, with increased total and OVA-specific serum IgE concentrations (see e.g., FIGS. 3A-3F). These results indicated that dysbiosis is also an essential pathogenic attribute of FA in the *Il4ra*^{F709} mice, and that the promotion by dysbiosis of FA in both the human subjects and mouse model can proceed by common mechanisms.

Dysbiosis in FA is associated with an altered immune response to the gut microbiota. Without wishing to be bound by theory, it was reasoned that dysbiosis in FA subjects could reflect an altered immune response to the gut microbiota. The gut secretory IgA (sIgA) response shapes the composition of the microbial flora and helps maintain commensalism. Accordingly, the binding of sIgA to the fecal flora of FA and control subjects was analyzed by flow cytometry. The gating strategy for immunoglobulin staining of human fecal flora is demonstrated in FIG. 11A and 11B. FA subjects displayed decreased sIgA binding of their fecal bacteria as compared to control subjects (see e.g., FIG. 2A and 2B). Without wishing to be bound by a particular theory, it was hypothesized that FA involves dysregulated allergic responses to both food and bacteria. This concept was explored by analyzing the binding of IgE to fecal bacteria in FA and healthy subjects. Notably, it was discovered that FA subjects exhibited increased IgE binding to fecal bacteria, consistent with an allergic response against commensal species (see e.g., FIG. 2C and 2D).

To more thoroughly investigate the mucosal antibody responses in FA, the binding of sIgA and IgE to the fecal bacteria of *Il4ra*^{F709} mice were analyzed, which are genetically prone to develop FA upon oral sensitization with food allergens, following gating strategies shown in FIG. 11C and 11D. The *Il4ra*^{F709} mice also exhibit dysbiotic microbiota which, upon transfer to germ free BALB/c mice heightens their susceptibility to food allergy induction. Accordingly, *Il4ra*^{F709} mice and control WT BALB/c mice were either sham sensitized with PBS or orally sensitized with chicken egg ovalbumin (OVA) in the presence of the mucosal adjuvant staphylococcal enterotoxin B (SEB) and subsequently challenged with OVA, as detailed previously (see e.g., Noval Rivas et al. (2013) *supra*; Burton et al. Immunity 41, 141-151 (2014); Noval Rivas et al. Immunity 42, 512-523 (2015); Noval Rivas et al. J Allergy Clin Immunol (2016); the content of each of which are incorporated by reference herein in their entirety). OVA sensitized *Il4ra*^{F709} but not WT control mice challenged with OVA exhibited a rapid drop in their core body temperature, consistent with anaphylaxis (see e.g., FIG. 2E). Similar to FA subjects, the fecal bacteria of OVA-sensitized *Il4ra*^{F709}mice exhibited decreased sIgA binding as compared to similarly sensitized WT mice or sham sensitized *Il4ra*^{F709} and WT mice (see e.g., FIG. 2F and 2G). In addition, sensitization with OVA also resulted in an increased IgE binding to fecal bacteria of *Il4ra*^{F709} mice, but not WT controls (see e.g., FIG. 2H and 2I). The specificity of these results was further confirmed by the lack of sIgA or IgE binding to fecal bacteria of Rag2-deficient mice, which do not express immunoglobulins, and the lack of IgE binding to the fecal bacteria of double mutant Igh7-/-Il4raF709 mice, which carry a targeted deletion of the IgE heavy chain gene (see e.g., FIG. 2F-2I). Overall, these results established that dysbiosis in FA human subjects and *Il4ra*^{F709} mice is associated with decreased sIgA responses and heightened T helper cell type 2 (Th2)/IgE responses to components of the commensal flora.

Given the capacity of fecal transplantation to increase the frequencies and numbers of gut ROR-γt+ Treg cells in FA mice, the frequencies of circulating ROR-γt+ Treg cells in human FA subjects were analyzed as compared to subjects who were atopic but not allergic to foods and to non-atopic subjects (see e.g., Table 2). Results showed that FA subjects had decreased circulating ROR-γt+ Treg cells as compared to those of the other two groups, which were otherwise similar (see e.g., FIG. 4A and 4B). In contrast, the frequencies of circulating ROR-γt+ Teff cells were not significantly between FA and control subjects and slightly increased in atopics (see e.g., FIGs. 4C, 11A-11B). Furthermore, ROR-γt+ Treg cells were decreased in both sham and OVA/SEB-sensitized FA *Il4ra*^{F709} mice as compared to the respective WT control mice (see e.g., FIG. 4D). To establish the role of ROR-γt+ Treg cells in tolerance induction in FA, the consequences of deleting Rorc were examined, the gene encoding ROR-γt, specifically in Treg cells in promoting FA in otherwise disease resistant WT mice. Mice expressing a Foxp3 allele that drove Treg cell-specific expression of a Cre recombinase and a yellow fluorescent protein (Foxp3YFPCre) were crossed to homozygosity with a floxed Rorc allele. The Treg cells of the resultant Foxp3YFPCreRorcΔ/Δ mice were profoundly deficient in Rorc mRNA and ROR-γt expression as compared to those of Foxp3YFPCre mice (see e.g., FIG. 11C-11E). Foxp3YFPCreRorcΔ/Δ mice sensitized with OVA/SEB and challenged with OVA developed a vigorous anaphylactic response that was comparable to that of similarly treated *Il4ra*^{F709} mice, with increased total and OVA-specific IgE and mast cell expansion and release. In contrast, Foxp3YFPCre mice were resistant to FA induction (see e.g., FIG. 4E and 4H). Also, and similar to the case of *Il4ra*^{F709} mice, Foxp3YFPCreRorcΔ/Δ mice sensitized with OVA/SEB exhibited decreased sIgA and increased IgE binding to the commensal fecal bacteria, consistent with the dysregulation of the mucosal immune response (see e.g., FIG. 12A-12D). Treg cell-specific Rorc deletion did not affect the frequency or cell numbers of Treg cells in the MLN of OVA/SEB sensitized Foxp3YFPCreRorcΔ/Δ mice, but the Treg cells in those tissues did show evidence of disease-promoting Th2 cell-like skewing with increased GATA3 and IL-4 expression, similar to those of FA I*Il4ra*^{F709} mice (see e.g., FIG. 4F-4J).

Finally, it is shown here that ROR-γt Treg cells were induced by bacterial metabolites (see e.g., FIG. 5A). Mice were treated with indoxyl-3-sulfate (I3S), at 100 mg/kg in PBS given intraperitoneally once weekly, or indol-3-carboxaldehyde (CA), at 100 mg/kg in PBS given orally once weekly, while being orally sensitized with OVA/SEB once weekly for 8 weeks. Representative flow cytometric analysis of ROR-γt⁺ staining in CD4⁺Foxp3⁺ Treg cells showed a statistically significant increase in CD4⁺Foxp3⁺ Treg cells in mice treated with I3S or CA compared to sham treatment (see e.g., FIG. 5A-5B).

Furthermore, Indoxyl 3 Sulfate (I3S) expanded gut ROR-γt⁺ Treg cells and protected against food allergy in *Il4ra*^{F709} mice (see e.g., FIGs. 6A-6G). *Il4ra*^{F709} mice that were either sham treated (PBS) or treated with I3S (at 100 mg/kg in PBS given intraperitoneally once weekly) while being orally sensitized with OVA/SEB once weekly for 8 weeks, then challenged with OVA. Mice treated with I3S displayed a statistically significant increase in core body temperature changes compared to control (see e.g., FIG. 6A).

Mice treated with I3S displayed a statistically significant decrease in GATA3⁺ gut Treg cells and a statistically significant increase in ROR-γt⁺ gut Treg cells (see e.g., FIG. 6B-6C). This trend extended to Helios⁺ gut Treg cells as mice treated with I3S displayed a statistically significant decrease in GATA3⁺ Helios⁺ gut Treg cells and a statistically significant increase in ROR-γt⁺ Helios⁺ gut Treg cells (see e.g., FIG. 6D-6E). This trend also extended to Helios⁻ gut Treg cells as mice treated with I3S displayed a statistically significant decrease in GATA3⁺ Helios⁻ gut Treg cells and a statistically significant increase in ROR-γt⁺ Helios⁻ gut Treg cells (see e.g., FIG. 6F-6G).

Mice treated with I3S did not display a statistically significant change in the percentage of total gut Treg cells (see e.g., FIG. 13).

### Discussion

Several important aspects of the role of dysbiosis in FA are described herein. First, described herein is the identification of an altered gut microbiota in FA infants starting as early as 1-6 months of age, consistent with a dysbiotic process that begins very early in life. The dysbiosis evolved dynamically over time, with some of these differences found to be age group-specific, while others persisted across different age groups. Importantly, in both FA infants and the FA-prone *Il4ra*^{F709} mice, which have been shown to be dysbiotic, the dysbiosis was associated with altered gut mucosal immune responses to the microbiota, with decreased IgA and, strikingly, increased IgE responses to gut bacteria. Thus, the allergic response in FA extends beyond food allergens to involve the gut microbiota, indicative of a broader disturbance in oral tolerance than hitherto appreciated.

ROR-γt+ Treg cells have been implicated in mediating immune pro-tolerogenic functions of gut commensal bacteria, but it is unclear which immune responses were regulated by this Treg cell subpopulation. Gut commensals, including Bacteroidetes and Clostridia species, have been demonstrated to induce ROR-γt+ Treg cells, and such species have been shown to have a role in regulating Th1 and Th17 cell responses. It has been demonstrated that ROR-γt+ Treg cells are required for the suppression of inflammatory Th17 cell accumulation induced by pathobionts. By contrast, a role has been demonstrated for ROR-γt+ Treg cells in regulating gut Th2 responses, and their deficiency precipitated Th2 cell-mediated pathology. Thus, the induction of ROR-γt+ Treg cells by the commensal flora plays a requisite role in forestalling the development of FA. The frequencies of ROR-γt+ Treg cells were decreased in both human FA allergic subjects and food allergy-prone *Il4ra*^{F709} mice, consistent with the proposed role for these cells in mediating oral tolerance in FA.

It is important to understand the mechanisms by which RoR-γt+ Treg cells prevent FA. Without wishing to be bound by theory, it has been suggested that the specificity of the ROR-γt+ iTreg cell population is primarily directed at the gut microbiota (see e.g., Kim et al. Science 351, 858-863 (2016); Russler-Germain et al., Mucosal Immunol. 2017 Nov, 10(6):1375-1386; the content of each of which is incorporated by reference herein in its entirety). However, food antigens such as OVA have been shown to drive ROR-γt+ Treg cell differentiation in the context of a commensal-sufficient host, indicative of a food antigen-specific regulatory response (see e.g., Ohnmacht et al. (2015), *supra*). Upon Treg cell-specific deletion of Rorc or in conditions associated with their deficiency such as FA, a population of GATA3high, Th2 cell-like reprogrammed natural Treg cells that expressed the cytokine IL-4 expands in the gut. This population, which is also increased in FA subjects, contributes to disease pathogenesis, evidenced by regression of FA in I*Il4ra*^{F709} mice upon Treg cell-specific deletion of a genetic cassette encompassing the Il4 and Il13 genes (see e.g., Noval Rivas et al. (2015), *supra*). Without wishing to be bound by theory, these studies indicate that dysbiosis-related deficiency of ROR-γt+ Treg cells and the reciprocal expansion of GATA3high, Th2 cell-like reprogrammed Treg cells play a cardinal role in the pathogenesis of FA in early life. This early time window of disease vulnerability may be especially important given the malleability of the microbiota composition in infants and its susceptibility to pathogenic dysbiosis under environmental influences including diet and antibiotic usage. It is important to understand the mechanisms regulating ROR-γt+ Treg cell induction and the interplay of different Treg cell populations in maintaining oral tolerance to foods.

Finally, described herein is an expansion of the concept of tolerance breakdown in FA, for example by demonstrating the presence of a Th2 response not only against food but also against the gut microbiota. In both human subjects and mouse models with FA, there exists an altered mucosal immune response to the gut microbiota, with decreased sIgA and increased IgE binding. This dysregulation was precipitated by Rorc deficiency in Treg cells, indicating a direct role for ROR-γt+ Treg cells in shaping the immune tolerance to the gut microbiota. The contribution of an aberrant gut immune response to the microbiota in disease pathogenesis is important to establish. It has been shown that an immune response to the gut microbiota arises in the context of gastrointestinal infections and can contribute to recall immunity upon infectious rechallenge. Without wishing to be bound by theory, it is specifically contemplated herein that the presence of an anti-microbiota Th2 response similarly acts to aggravate pathogenic immune responses to food and can play a role in disease persistence.

### Summary

As described herein, ROR-γt+-expressing regulatory T (Treg) cells are an immune cell population critical for establishing oral immune tolerance to foods and whose deficiency can serve as a biomarker for food allergy. Food allergic children consistently exhibit profoundly lower frequencies of ROR-γt+ Treg cells in their peripheral blood as compared to healthy control subjects and to atopic subjects who otherwise do not suffer from food allergy. Similarly, food allergy prone *Il4ra*^{F709} mice also show a profound decrease in their ROR-γt+ Treg cells as compared to food allergy-resistant wild-type mice. Furthermore, fecal transplants from human subjects under the age of 3 years were employed into germ-free *Il4ra*^{F709} mice to show that transplants of fecal matter from control subjects offered recipient *Il4ra*^{F709} mice superior protection against the development of food allergy as compared to those mice that received fecal transplants from food allergic subjects. This protection correlated with increased frequencies of Treg cells expressing ROR-γt+. Wild-type mice then are normally resistant to the development of food allergy are rendered disease susceptible upon the abrogation of ROR-γt expression in their Treg cells. Treatment of food-allergy prone *Il4ra*^{F709} mice with indoxyl 3 sulfate (I3S), a bacterial metabolite that expands the population of ROR-γt+ Treg cells in the gut, is associated with protection against food allergy (see e.g., FIGs. 5A-5B, 6A-6G). As described herein, the induction of ROR-γt+ Treg cells can be used as a platform to screen probiotics that can be effective in the context of food allergy. Furthermore, agents, such as indole sulfate and indole-3-carboxaldehyde, that act to promote the induction of ROR-γt+ Treg cells can prevent food allergy and treat established disease. As described herein, the ROR-γt+ pathway can be targeted in food allergy in order to boost oral immune tolerance to food allergens.

### Material and Methods

**Subjects**. Subject Demographics. Table 1 summarizes subject demographics and FIG. 7A-7C summarizes the distribution of samples collected by subject age. One hundred and fifty-four subjects were enrolled, 56 (36%) with food allergies to at least one of the major food allergens including milk, soy, egg, tree nuts, fish, shellfish, wheat, or peanuts, and 98 (64%) healthy controls. Seventy-eight percent of FA subjects were poly-sensitized, as defined by a positive skin test and/or specific IgE to at least 2 foods. Other FA included sesame, oat, pea, avocado, apple, grape and cantaloupe. 26 FA infants (16.8%) were diagnosed with cow' milk allergy and were avoiding milk products at the time of stool sampling.

Healthy control subjects or subjects with FA age 1-15 months were enrolled. FA criteria included a history of allergic reactions to one or more of the major food allergens (e.g. milk, soy, egg, tree nuts, fish, shellfish, wheat, or peanuts), such as urticaria, angioedema, wheezing, diarrhea, vomiting, and moderate to severe eczema that was clearly triggered by food exposure and improving markedly after food avoidance, and a confirmatory positive food-specific skin prick test (SPT) ≥3mm compared to saline control and/or serum food-specific IgE ≥0.35. Exclusion criteria included: 1) prematurity, defined as delivery before 37 weeks of gestation, 2) recurrent or chronic infections necessitating frequent systemic (including oral) antibiotic administration, 3) history of chronic immunosuppressive therapies, 4) history of gastroenterological conditions, including non-IgE mediated colitis, eosinophilic esophagitis and food protein induced enterocolitis, allergic colitis, GE reflux or constipation necessitating medication, and 5) history of other chronic diseases, except for atopic conditions.

For studies on circulating ROR-γt+ Treg and Teff cells, demographic details on the human subjects involved, including FA, atopic but not FA and healthy controls are detailed in Table 2. The diagnosis of FA was ascertained as detailed in the above section. Subjects who were atopic but not FA carried a diagnosis of allergic rhinitis, asthma and/or eczema, as detailed in Table 2, while healthy control subjects did not have a history of FA or atopic diseases. All human subjects were recruited from regional hospital main and satellite sites. All human studies were approved by an Institutional Review Board.

**Sample collection**. Parents were asked to collect stools from subjects every 4-6 months for up to 30 months of age, using a clean wood stick in Eppendorf tubes and RNA-later^{™} tubes (Thermofisher^{™}) and to freeze the sample in their home freezer immediately. Samples were collected from subjects' homes within 1-3 weeks of the specimen collection. Overall, 60 subjects gave only one stool sample, 31 subjects gave 2 serial samples, 30 gave 3 samples, 17 gave 4 samples and 16 gave 5 samples. Each sample collection was spaced by 4-6 months. If systemic antibiotics were prescribed, sample collection was delayed for 4-6 weeks after the last dose of antibiotic administered, as guided by studies tracking the recovery of gut microbiota following antibiotic treatment (see e.g., Dethlefsen & Relman, Proc Natl Acad Sci U S A 108 Suppl 1, 4554-4561 (2011); Gerber et al. PLoS Comput Biol 8, e1002624 (2012); the content of each of which are incorporated herein by reference in their entirety).

If a patient became tolerant to the food that they were initially allergic to (defined by being able to tolerate at least 4 grams of protein of that food), subsequent samples were not included in the analysis unless the subject had another FA confirmed by a history and positive skin and/or specific IgE. Samples from sensitized patients who lack a confirmatory history of an allergic food reaction were excluded from analysis. Parents completed an online questionnaire with each stool collection that included information regarding diet, breastfeeding, age, FA, infection, and use of antibiotics.

**16S rDNA gene phylotyping**. A multiplexed amplicon library covering the 16S rDNA gene V4 region was generated from human stool sample DNA (see e.g., Bucci et al. Genome Biol 17, 121 (2016); the content of each of which are incorporated herein by reference in their entirety). Briefly, bacterial genomic DNA was extracted using the Mo Bio^{™} Power Fecal DNA Isolation kit (Mo Bio Laboratories^{™}) according to the manufacturer's instructions. To increase the DNA yields, the following modifications were used. An additional bead beater step using the Faster Prep FP120 (Thermo^{™}) at 6 meters/second for 1 min was used instead of vortex agitation. Incubation with buffers C2 and C3 was done for 10 min at 4°C. Starting nucleic acid concentrations were determined by a Qubit^{™} Fluromoter (Life Technologies^{™}). The amplicon library was prepared using dual-index barcodes (see e.g., Bucci et al., (2016) *supra*)⁵³. The aggregated library pool was size selected from 300-500 base pairs (bp) on a pippin prep 1.5% agarose cassette (Sage Sciences^{™}) according to the manufacturer's instructions. The concentration of the pool was measured by qPCR (Kapa Biosystems^{™}) and loaded onto the MiSeq Illumina^{™} instrument (300 bp kit) at 6-9pM with 40% phiX spike-in to compensate for low base diversity according to Illumina's standard loading protocol.

**16S rDNA gene sequencing data preprocessing**. Sequencing of the 16S rDNA amplicons from the 368 human stool samples generated 14,279,132 total raw reads, with a mean of 38,802 reads per sample. Raw sequencing reads were processed using the mothur^{™} software package (v.1.35.1) 54 and custom Python^{™} scripts, which perform de-noising, quality filtering, alignment against the ARB Silva^{™} reference database of 16S rDNA gene sequences, and clustering into Operational Taxonomic Units (OTUs) at 97% identity. In total, 16387 OTUs were generated. OTUs with extremely low abundance were filtered using the following parameters: removed if mean relative abundance <0.001 across all samples or if zero read in >80% of samples in at least one cohort.

Since the composition of the microbiota is known to change throughout childhood, subjects were stratified by age into six month intervals (1-6, 7-12, 13-18, 19-24, and 25-30 months). This interval span was chosen to provide resolution with respect to age, while ensuring sufficient numbers of subjects in each group to support meaningful comparisons. Because insufficient numbers of subjects were available for the 30-36 month group, this group was omitted from the analyses. After stratifying subjects by age groups segregated at six month intervals, 47, 53, 78, 74 and 79 OTUs were available after filtering in age group 1-6 months, 7-12 months, 13-18 months, 19-24 months, and 25-30 months, respectively.

**16S rDNA gene sequencing data statistical analysis**. To assess for differences in ecological (alpha) diversity, Shannon entropy was calculated for each sample with statistical testing using the Wilcoxon rank-sum test. To assess differences in overall microbial community structure, beta-diversity was calculated using the unweighted and weighted Unifrac measures with statistical testing using the Analysis of Molecular Variance (AMOVA) method.

To statistically test for differences in the relevant abundances of fecal microbiota OTUs between control and food allergic subjects, the DESeq2 software package was employed with an analysis design depicted in FIG. 7A-7C (see e.g., McMurdie & Holmes, PloS ONE 8, e61217 (2013); McMurdie & Holmes, PLoS computational biology 10, e1003531 (2014)). Key covariates of interest (e.g., gender, mode of delivery, and breastfeeding only for younger than 18 months) were controlled for using the multi-factorial model in DESeq2. Since cow's milk protein (CMP) intake could directly alter the microbiota, and is highly correlated with FA status, a subset analysis removing subjects without CMP intake was also performed. P-values were adjusted for multiple hypothesis testing using the method of Benjamini and Hochberg (BH) (see e.g., Benjamini & Hochberg, Journal of the Royal Statistical Society Series B 57, 289-300 (1995)). OTUs reported met the following criteria: (1) adjusted p-value <=0.1; (2) absolute value of log2 fold change ≥2.

**16S rDNA gene sequencing data phylogenetic analysis**. To more accurately identify the microorganisms present in samples and their phylogenetic relationships to known species, the pplacer software package was used to perform phylogenetic placement (see e.g., Matsen et al., BMC bioinformatics 11, 538 (2010)). Pplacer uses a likelihood-based methodology to place short sequencing reads of 16S rDNA amplicons on a reference tree, and also generates taxonomic classifications of the short sequencing reads using a least common ancestor-based algorithm. The reference tree required for phylogenetic placement was generated using full-length or near full-length (>1,200 nt) 16S rDNA sequences of type strains from the Ribosomal Database Project (RDP). The taxa that were phylogenetically placed with a like weight ratio of ≥0.8 are reported herein. For purposes of describing OTUs as described herein, the closest reference species (CRS) to the phylogenetically placed consensus sequence for the OTU is used. While CRS does not represent an unambiguous species identification, it provides a point of reference for understanding microbiologically driven mechanisms in FA, and for deeper characterization using metagenomic or culture-based methods.

**Oral allergic sensitization of mice**. The following mice on BALB/c background were used: BALB/cByJ (designated as WT mice), Rag2-/- (C.129S6(B6)-Rag2tm1Fwa), *Il4ra*^{F709}(C.129X1-Il4ratm3.1Tch) 63, Igh7-/-Il4raF709 64 and Foxp3EGFP/DTR+. The following C57BL/6 congenic strains, Rorcfl/fl (ROR-gtf/fB6(Cg)-Rorctm3Litt/J), B6.129(Cg)-Foxp3tm4(YFP/cre)Ayr/J) (Foxp3YFPCre), and B6.129(Cg)-Il4raF709, were crossed to generate Il4raF709Foxp3YFPCre, Foxp3YFPCreRorcΔ/Δ, and Il4raF709Foxp3YFPCreRorcΔ/Δ mice. In all experiments, mice were matched for strain background. Mice were subjected to oral allergic sensitization with ovalbumin (OVA), mixed together with the mucosal adjuvant staphylococcal enterotoxin B (SEB) (Toxin Technology) (see e.g., Noval Rivas et al., 2015, *supra*; Noval Rivas et al., 2013, *supra*). For antibiotic treatment, mice were treated with an antibiotic cocktail (Sigma-Aldrich^{™}) containing ampicillin 2.5 mg/ml), metronidazole (2.5 mg/ml), gentamycin (0.4 mg/ml), streptomycin (0.5 mg/ml), vancomycin (0.5 mg/ml), administered by oral gavage in a final total volume of 100 µl PBS once daily for 1 week, as indicated. For Treg cell depletion with diphtheria toxin (DT), mice were injected intra-peritoneally (i.p.) DT (Sigma-Aldrich^{™}) at 250 ng/ml per injection/mouse (about 10 µg/kg), as indicated. For treatment with anti-CD25 or isotype control mAbs (BioXCell^{™}), mice were injected intraperitoneally (i.p.) with the indicated antibody at 100 µg/injection/mouse, as indicated.

**Detection of Fecal Bacteria-bound IgA and IgE by Flow Cytometry**. 50mg of fecal pellet was homogenized in 1ml of sterile cold PBS and centrifuged at 40g for 10 minutes at 4°C to remove large particles. Supernatant containing the bacteria were collected, filtered through a 70µm strainer and centrifuged at 8000 g for 5 minutes to pellet the bacteria. The pellets were then washed twice with 1ml of sterile PBS and incubated on ice for 15 minutes with blocking buffer (50% fetal calf serum (FCS) in PBS for human fecal samples and 50% FCS + 10mg/ml OVA in PBS for mouse fecal samples). Samples were centrifuged at 8000g for 5 minutes and subsequently stained with 5µM SYTO-BC (eBioscience-ThermoFisher^{™}) along with either anti-mouse IgA (clone mA-6E1, eBioscience-ThermoFisher^{™}) or anti-mouse IgE (clone RME-1, Biolegend^{™}). Similarly, human fecal samples were stained with 5µM SYTO-BC along with either anti-human IgA (clone IS11-8E10, Miltenyi Biotech^{™}) or anti-human IgE (clone G7-26, BD Biosciences^{™}). Samples were then washed 3 times with 1 ml of PBS before flow cytometric analysis on a BD LSR Fortessa^{™}.

**Short Chain Fatty Acid Analyses**. Samples of gut contents were kept frozen at - 80°C until analysis. The samples were removed from the freezer and thawed. 500 µl of HPLC water was added to each sample and vortexed for 10 minutes and then centrifuged at 5000g for 10 minutes. 400 µl of the clear supernatant was transferred to a 2.0 ml Eppendorf tube. The pH of each sample was adjusted to 2-3 by adding 50 µl of 50% sulfuric acid. 50 µl of the internal standard (1% 2-methyl pentanoic acid solution) and 400 µl of ethyl ether anhydrous were added (Sigma-Aldrich^{™}). The tubes were mixed end over end for 10 minutes and then centrifuged at 2500g for 2 minutes. The upper ether layer was transferred to an Agilent^{™} sampling vial for analysis. 1 µl of the upper ether layer was injected into the chromatogram for analysis.

Chromatographic analysis was carried out using an Agilent 7890B^{™} system with a flame ionization detector (FID) (Agilent Technologies^{™}). A high-resolution gas chromatography capillary column 30m x 0.25 mm coated with 0.25um film thickness was used (DB-FFAP) for the volatile acids (Agilent Technologies^{™}). Nitrogen was used as the carrier gas. The oven temperature was 145°C and the FID and injection port was set to 225 °C. The injected sample volume was 1 µl and the run time for each analysis was 12 minutes. Chromatograms and data integration was carried out using the OpenLab ChemStation^{™} software (Agilent Technologies^{™}).

**Standard Solutions**: A volatile acid mix containing 10 mM of acetic, propionic, isobutyric, butyric, isovaleric, valeric, isocaproic, caproic, and heptanoic acids was used (Supelco^{™}/Sigma-Aldrich^{™}). A standard stock solution containing 1% 2-methyl pentanoic acid (Sigma-Aldrich^{™}) was prepared as an internal standard control for the volatile acid extractions.

**Quantification of Acids**: 400 µl of the standard mix was used and the extracts prepared as described for the samples except that 400 µl of ethyl ether was added. The retention times and peak heights of the acids in the standard mix were used as references for the sample unknowns. These acids were identified by their specific retention times and the concentrations determined and expressed as mM concentrations per gram of sample.

**Quantitative real-time PCR for host immunological Targets**. RNA was extracted from cells using Quick-RNA MiniPrep^{™} kit (Zymo Research^{™}) according to the manufacturer protocol. Reverse transcription was performed with the SuperScript III^{™} RT-PCR system and random hexamer primers (Invitrogen^{™}) and quantitative real-time reverse transcription (RT)-PCR with Taqman^{®} Fast Universal PCR master mix, internal housekeeping gene mouse (Hprt VIC-MGB dye) and specific target gene primers for murine Rorc, as indicated (FAM Dye) (Applied Biosystems^{™}) on Step-One-Plus^{™} machine. Relative expression was normalized to Hprt and calculated as fold change compared to Foxp3YFPCre Treg cells.

**Flow cytometry**. The following anti-mouse antibodies were used: CD4 (RM4-5), CD3 (145-2C11), Foxp3 (FJK-16S), GATA-3 (TWAJ), ROR-γt (B2), IgA (mA-6E1), rat IgG1 Isotype control (eBRG1), (eBioscience^{™}), IL-4 (11B11) and rat IgG1 Isotype control (R3-34) (BD Biosciences^{™}), Neuropilin-1 (3E12), Helios (22F6), IgE (RME-1), (Biolegend^{™}). Anti-human antibodies used in this study included IgE (G7-26), mouse IgG2a isotype control (G155-178) (BD Biosciences^{™}), IgA (IS11-8E10) (Miltenyi Biotech^{™}) and mouse IgG1 (P3) (eBioscience^{™}). For cytokines cells were stimulated during 4 hours with PMA (50 ng/ml; Sigma-Aldrich^{™}) and ionomycin (500 ng/ml; Sigma-Aldrich^{™}) in the presence of Golgi Plug (BD Biosciences^{™}), then stained with the BD Cytofix/Cytoperm^{™} buffers (BD Biosciences^{™}) and the indicated anti-cytokine antibody. For intracellular staining of nuclear factors, the Foxp3 Transcription Factor buffer set (eBioscience^{™}) was used. Dead cells were routinely excluded from the analysis based on the staining of eFluor 506 fixable viability dye (eBioscience^{™}), and analyses were restricted to single cells using FSC-H and FSC-A signals. Stained cells were analyzed on an LSR Fortessa^{™} (BD Biosciences^{™}) and data were processed using Flowjo^{™} (Tree Star Inc.^{™}).

**ELISA**. Total, OVA-specific IgE and Murine mast cell protease 1 (MMCP-1) concentrations were measured in the sera of treated mice by ELISAs (see e.g., Noval Rivas et al., 2013, *supra*).

**Histology**. Intestinal mast cells were counted by microscopic examination of jejunal sections fixed in 10% formaldehyde and stored in ethanol 70% before staining with toluidine blue by a histopathology facility.

**Statistical analysis.** Anaphylaxis-related Core body temperature measurements were analyzed using repeat measures 2-way ANOVA. Student unpaired 2-tailed t-tests were used for 2-group comparisons. For more than 2 groups, 1-way ANOVA with post test analysis was used. Results are presented as means and SEMs, where each point represents 1 sample. In cases in which values were spread across multiple orders of magnitude, data were log-transformed for analysis with parametric tests.

**Treatment with bacterial metabolites.** Mice were treated with indoxyl-3-sulfate (I3S), at 100 mg/kg in PBS given intraperitoneally once weekly, or indol-3-carboxaldehyde (CA), at 100 mg/kg in PBS given orally once weekly, while being orally sensitized with OVA/SEB once weekly for 8 weeks. Core body temperature measurements were made, and Treg populations were analyzed flow cytometry, as described further herein.

**Table** 1 shows demographic characteristics of 56 FA and 98 controls subjects. Subjects were recruited ages 1-15 month and followed thereafter up to age 30 months. Samples that were collected from subjects were segregated by different age group: 1-6 months, 7-12 months-13-18 months, 19-24 months and 25-30 months. The attributes of patients contributing to the samples collected at the respective age groups, including gender, mode of delivery, breastfeeding and milk tolerance are shown. P -values were adjusted for multiple hypothesis using the Benjami-Hochberg method. A P value <0.05 was considered significant.

**Table 1: Demographic characteristics of FA and control infants.**

| | **1-6 months** | | **7-12 months** | | **13-18 months** | | **19-24 months** | | **25-30 months** | |
|---|---|---|---|---|---|---|---|---|---|---|
| | **Control (C)** | **Food allergic (FA)** | **C** | **FA** | **C** | **FA** | **C** | **FA** | **C** | **FA** |
| | **(n=32)** | **(n=10)** | **(n=61)** | **(n=22)** | **(n=56)** | **(n=33)** | **(n=33)** | **(n=24)** | **(n=9)** | **(n=15)** |
| **Demographic characteristics** | **n (%)** | **n (%)** | **n (%)** | **n (%)** | **n (%)** | **n (%)** | **n (%)** | **n (%)** | **n (%)** | **n (%)** |

| **Age group in months** | | | | | | | | | | |
|---|---|---|---|---|---|---|---|---|---|---|
| | **1-6 months** | | **7-12 months** | | **13-18 months** | | **19-24 months** | | **25-30 months** | |
| | **Control (C)** | **Food allergic (FA)** | **C** | **FA** | **C** | **FA** | **C** | **FA** | **C** | **FA** |
| | 32 (76.19) | 10 (23.81) | 61 (73.49) | 22 (26.51) | 56 (62.91) | 33 (37.09) | 33 (57.89) | 24 (42.11) | 9 (37.50) | 15 (62.50) |

| **Gender** | | | | | | | | | | |
|---|---|---|---|---|---|---|---|---|---|---|
| Female | 18 (56.25) | 3 (30) | 29 (47.54) | 9 (40.91) | 29 (51.79) | 10 (30.30) | 14 (42.42) | 7 (29.17) | 6 (66.67) | 3 (20) |
| Male | 14 (43.75) | 7 (70) | 32 (52.46) | 13 (59.09) | 27 (48.21) | 23 (69.70) | 19 (57.58) | 17 (70.83) | 3 (33.33) | 12 (80) |
| ***Adjusted p-values*** | 0.815 | | 1.000 | | 0.381 | | 0.815 | | 0.225 | |

| **Mode of delivery** | | | | | | | | | | |
|---|---|---|---|---|---|---|---|---|---|---|
| C/S | 6 (18.75) | 1 (10) | 18 (29.51) | 4 (18.18) | 20 (35.71) | 7 (21.21) | 13 (39.39) | 6 (25) | 4 (44.44) | 3 (20) |
| NVD | 26 (81.25) | 9 (90) | 43 (70.49) | 18 (81.82) | 36 (63.29) | 26 (78.79) | 20 (60.61) | 18 (75) | 5 (55.56) | 12 (80) |
| ***Adjusted p-values*** | 1.000 | | 0.815 | | 0.815 | | 0.815 | | 0.815 | |

| **Breast feeding** | | | | | | | | | | |
|---|---|---|---|---|---|---|---|---|---|---|
| No | 7 (31.82) | 3 (30) | 24 (39.34) | 9 (40.91) | 38 (67.86) | 21 (63.63) | 30 (90.91) | 22 (91.67) | 8 (88.89) | 12 (80) |
| Yes | 25 (68.18) | 7 (70) | 37 (60.66) | 13 (59.09) | 18 (32.14) | 12 (36.37) | 3 (9.09) | 2 (8.33) | 1 (11.11) | 3 (20) |
| ***Adjusted p-values*** | 1.000 | | 1.000 | | 1.000 | | 1.000 | | 1.000 | |

| **Cow's Milk Proteins intake** | | | | | | | | | | |
|---|---|---|---|---|---|---|---|---|---|---|
| No | 28 (87.50) | 10 (100) | 21 (34.43) | 16 (72.73) | 3 (5.36) | 16 (48.49) | 0 | 8 (33.33) | 0 | 4 (26.67) |

| | **1-6 months** | | **7-12 months** | | **13-18 months** | | **19-24 months** | | **25-30 months** | |
|---|---|---|---|---|---|---|---|---|---|---|
| | **Control (C)** | **Food allergic (FA)** | **C** | **FA** | **C** | **FA** | **C** | **FA** | **C** | **FA** |
| Yes | 4 (12.50) | 0 | **40** (65.57) | 6 (27.27) | 53 (94.64) | 17 (51.51) | 33 (100) | 18 (66.67) | 9 (100) | 11 (73.33) |
| ***Adjusted p-values*** | 0.994 | | 0.022 | | 0.0001 | | 0.009 | | 0.815 | |

Table 2 shows characteristics of 35 FA, 11 healthy subjects and 25 atopic controls recruited for the analysis of ROR-γt+ Treg and Teff cells in humans. Subjects were recruited ages 1-21 years of age. The key for Table 2 is as follows; HC: Healthy Controls; FA: Food Allergy; AS: Asthma; AR: Allergic Rhinitis; AD: Eczema.

**Table 2: Demographic characteristics of FA, Atopic but not FA, and Healthy Control subjects.**

| | **Gender** | **Age** | **Condition** |
|---|---|---|---|
| **HC** | F | 8 | HC |
| | M | 10 | HC |
| | F | 10 | HC |
| | M | 10 | HC |
| | F | 8 | HC |
| | M | 2 | HC |
| | M | 2 | HC |
| | M | 12 | HC |
| | M | 11 | HC |
| | F | 6 | HC |
| **Atopic Controls** | M | 11 | AS, ED, AR |
| | M | 9 | AS, AR, AD |
| | F | 8 | AR, AD |
| | M | 12 | FA, AS, AR, AD |
| | M | 11 | AR, AD |
| | M | 9 | AR |
| | M | 6 | AR, AD |
| | M | 10 | AR |
| | F | 8 | AS, AR, AD |
| | M | 9 | AS, AD |
| | M | 13 | AS |
| | F | 10 | FA, AS |
| | M | 14 | AS, AR, AD |
| | F | 17 | AS, AR |
| | M | 15 | AS, AR |
| | M | 4 | AS |
| | M | 13 | AR, AS |
| | M | 12 | AR |
| | F | 5 | AS |
| | F | 10 | AR |
| | M | 13 | AR, AD, AS |
| | F | 21 | AR, AS |
| | F | 8 | AS, AR |
| | M | 5 | AD |
| | M | 3 | AD |
| **Food Allergy** | F | 10 | FA (peanut) |
| | M | 11 | FA (peanut, treenut) |
| | M | 14 | FA (peanut) |
| | M | 17 | FA (peanut) |
| | M | 14 | FA (peanut) |
| | F | 4 | FA (peanut) |
| | M | 5 | FA (peanut) |
| | F | 10 | FA (peanut) |
| | F | 2 | FA (oat) |
| | M | 1 | FA (soy, egg, peanut, sesame, treenut, milk) |
| | M | 13 | FA (egg, peanut) |
| | M | 14 | FA (peanut) |
| | F | 1 | FA (milk, egg) |
| | F | 1 | FA (peanut) |
| | M | 1 | FA (peanut) |
| | F | 15 | FA (peanut, treenut, egg, legume, shellfish) |
| | M | 3 | FA (treenut, sesame) |
| | M | 8 | FA (fish) |
| | M | 1 | FA (peanut, milk, egg) |
| | M | 7 | FA (milk, banana) |
| | M | 23 | FA (peanut, treenut, chicken, pumpkin, turkey) |
| | M | 8 | FA (peanut) |
| | F | 2 | FA (peanut) |
| | M | 11 | FA (treenut, sesame) |
| | M | 11 | FA (egg) |
| | M | 16 | FA (shellfish, peanut, treenut) |
| | M | 19 | FA (peanut) |
| | F | 5 | FA (peanut, treenut, sesame) |
| | M | 6 | FA (peanut) |
| | F | 4 | FA (peanut, treenut, sesame) |
| | F | 1 | FA (egg) |
| | M | 3 | FA (milk) |
| | M | 3 | FA (peanut) |
| | F | 1 | FA (egg, peanut, sunflower, sesame, poppy) |
| | M | 2 | FA (egg, peanut) |

### Sequences

**SEQ ID NO: 1** (RAR related orphan receptor C- *Homo sapiens*)
NCBI Reference Gene ID: 6097; Homo sapiens chromosome 1, GRCh38.p13 Primary Assembly NCBI Reference Sequence: NC_000001.11, NC_000001 REGION:
complement(151806071..151832451) 26381 bp.

**SEQ ID NO: 2** (RAR-related orphan receptor gamma- *Mus musculus*) NCBI Reference Gene ID: 19885 Mus musculus strain C57BL/6J chromosome 3, GRCm38.p6 C57BL/6J NCBI Reference Sequence: NC_000069.6, NC_000069 REGION: 94372794..94398276; 25483 bp.

**SEQ ID NO: 3** Homo sapiens RAR related orphan receptor C (RORC), transcript variant 2, mRNA, NCBI Reference Sequence: NM_001001523.2, CDS, 1494 bp. 
1 atgagaacac aaattgaagt gatcccttgc aaaatctgtg gggacaagtc gtctgggatc
61 cactacgggg ttatcacctg tgaggggtgc aagggcttct tccgccggag ccagcgctgt
121 aacgcggcct actcctgcac ccgtcagcag aactgcccca tcgaccgcac cagccgaaac
181 cgatgccagc actgccgcct gcagaaatgc ctggcgctgg gcatgtcccg agatgctgtc
241 aagttcggcc gcatgtccaa gaagcagagg gacagcctgc atgcagaagt gcagaaacag
301 ctgcagcagc ggcaacagca gcaacaggaa ccagtggtca agacccctcc agcaggggcc
361 caaggagcag ataccctcac ctacaccttg gggctcccag acgggcagct gcccctgggc
421 tcctcgcctg acctgcctga ggcttctgcc tgtccccctg gcctcctgaa agcctcaggc
481 tctgggccct catattccaa caacttggcc aaggcagggc tcaatggggc ctcatgccac
541 cttgaataca gccctgagcg gggcaaggct gagggcagag agagcttcta tagcacaggc
601 agccagctga cccctgaccg atgtggactt cgttttgagg aacacaggca tcctgggctt
661 ggggaactgg gacagggccc agacagctac ggcagcccca gtttccgcag cacaccggag
721 gcaccctatg cctccctgac agagatagag cacctggtgc agagcgtctg caagtcctac
781 agggagacat gccagctgcg gctggaggac ctgctgcggc agcgctccaa catcttctcc
841 cgggaggaag tgactggcta ccagaggaag tccatgtggg agatgtggga acggtgtgcc
901 caccacctca ccgaggccat tcagtacgtg gtggagttcg ccaagaggct ctcaggcttt
961 atggagctct gccagaatga ccagattgtg cttctcaaag caggagcaat ggaagtggtg
1021 ctggttagga tgtgccgggc ctacaatgct gacaaccgca cggtcttttt tgaaggcaaa
1081 tacggtggca tggagctgtt ccgagccttg ggctgcagcg agctcatcag ctccatcttt
1141 gacttctccc actccctaag tgccttgcac ttttccgagg atgagattgc cctctacaca
1201 gcccttgttc tcatcaatgc ccatcggcca gggctccaag agaaaaggaa agtagaacag
1261 ctgcagtaca atctggagct ggcctttcat catcatctct gcaagactca tcgccaaagc
1321 atcctggcaa agctgccacc caaggggaag cttcggagcc tgtgtagcca gcatgtggaa
1381 aggctgcaga tcttccagca cctccacccc atcgtggtcc aagccgcttt ccctccactc
 1441 tacaaggagc tcttcagcac tgaaaccgag tcacctgtgg ggctgtccaa gtga

**SEQ ID NO: 4** Mus musculus RAR-related orphan receptor gamma (Rorc), transcript variant 2, mRNA, NCBI Reference Sequence: NM_001293734.1, CDS, 1488 bp.

1 atgagaacac aaattgaagt gatcccttgc aagatctgtg gggacaagtc atctgggatc
61 cactacgggg ttatcacctg tgaggggtgc aagggcttct tccgccgcag ccagcagtgt
121 aatgtggcct actcctgcac gcgtcagcag aactgcccca ttgaccgaac cagccgcaac
181 cgatgccagc attgccgcct gcagaagtgc ctggctctgg gcatgtcccg agatgctgtc
241 aagtttggcc gaatgtccaa gaagcagagg gacagtctac atgcagaagt gcagaaacaa
301 ctgcaacagc agcagcaaca ggaacaagtg gccaagactc ctccagctgg gagccgcgga
361 gcagacacac ttacatacac tttagggctc tcagatgggc agctaccact gggcgcctca
421 cctgacctac ccgaggcctc tgcttgtccc cctggcctcc tgagagcctc aggctctggc
481 ccaccalall ccaalaccll ggccaaaaca gagglccagg gggcclcclg ccaccllgag
541 tatagtccag aacgaggcaa agctgaaggc agagacagca tctatagcac tgacggccaa
601 cttactcttg gaagatgtgg acttcgtttt gaggaaacca ggcatcctga acttggggaa
661 ccagaacagg gtccagacag ccactgcatt cccagtttct gcagtgcccc agaggtacca
721 tatgcctctc tgacagacat agagtacctg gtacagaatg tctgcaagtc cttccgagag
781 acatgccagc tgcgactgga ggaccttcta cggcagcgca ccaacctctt ttcacgggag
841 gaggtgacca gctaccagag gaagtcaatg tgggagatgt gggagcgctg tgcccaccac
901 ctcactgagg ccattcagta tgtggtggag tttgccaagc ggctttcagg cttcatggag
961 ctctgccaga atgaccagat catactactg aaagcaggag caatggaagt cgtcctagtc
1021 agaatgtgca gggcctacaa tgccaacaac cacacagtct tttttgaagg caaatacggt
1081 ggtgtggagc tgtttcgagc cttgggctgc agcgagctca tcagctccat atttgacttt
1141 tcccacttcc tcagcgccct gtgtttttct gaggatgaga ttgccctcta cacggccctg
1201 gttctcatca atgccaaccg tcctgggctc caagagaaga ggagagtgga acatctgcaa
1261 tacaatttgg aactggcttt ccatcatcat ctctgcaaga ctcatcgaca aggcctccta
1321 gccaagctgc cacccaaagg aaaactccgg agcctgtgca gccaacatgt ggaaaagctg
1381 cagatcttcc agcacctcca ccccatcgtg gtccaagccg ccttccctcc actctataag
1441 gaactcttca gcactgatgt tgaatcccct gaggggctgt caaagtga

**SEQ ID NO: 5** (nuclear receptor ROR-gamma isoform b [Homo sapiens]), NCBI Reference Sequence: NP_001001523.1, 497 aa. 
1 mrtqievipc kicgdkssgi hygvitcegc kgffrrsqrc naaysctrqq ncpidrtsrn
61 rcqhcrlqkc lalgmsrdav kfgrmskkqr dslhaevqkq lqqrqqqqqe pvvktppaga
121 qgadtltytl glpdgqlplg sspdlpeasa cppgllkasg sgpsysnnla kaglngasch
181 leyspergka egresfystg sqltpdrcgl rfeehrhpgl gelgqgpdsy gspsfrstpe
241 apyasltele hlvqsvcksy retcqlrled llrqrsnifs reevtgyqrk smwemwerca
301 hhlteaiqyv vefakrlsgf melcqndqiv llkagamevv lvrmcrayna dnrtvffegk
361 yggmelfral gcselissif dfshslsalh fsedeialyt alvlinahrp glqekrkveq
 
421 lqynlelafh hhlckthrqs ilaklppkgk lrslcsqhve rlqifqhlhp ivvqaafppl

481 ykelfstete spvglsk
 **SEQ ID NO: 6** (nuclear receptor ROR-gamma isoform 2 [Mus musculus]) NCBI Reference Sequence: NP_001280663.1, 495 aa.
1 mrtqievipe kicgdkssgi hygvitcege kgffrrsqqc nvaysctrqq nepidrtsrn
61 rcqhcrlqkc lalgmsrdav kfgrmskkqr dslhaevqkq lqqqqqqeqv aktppagsrg
121 adtltytlgl sdgqlplgas pdlpeasacp pgllrasgsg ppysntlakt evqgaschle
181 yspergkaeg rdsiystdgq ltlgrcglrf eetrhpelge peqgpdshci psfesapevp
241 yasltdieyl vqnvcksfre tcqlrledll rqrtnlfsre evtsyqrksm wemwercahh
301 lteaiqyvve fakrlsgfme lcqndqiill kagamevvlv rmcraynann htvffegkyg
361 gvelfralge selissifdf shflsalcfs edeialytal vlinanrpgl qekrrvehlq
421 ynlelafhhh lckthrqgll aklppkgklr slcsqhvekl qifqhlhpiv vqaafpplyk
481 elfstdvesp eglsk

## Claims

1. A method of identifying a microorganism that induces a regulatory T cell, or a population thereof, that expresses RORγt, the method comprising:
a. measuring a level of expression of RORγt in regulatory T cells in a sample from a subject who has been exposed to at least one microorganism to a subject for a time sufficient to allow for the colonization of the at least one microorganism in the gut of the subject; and
b. identifying the at least one microorganism as an inducer of a regulatory T cell, or a population thereof, that expresses RORγt if the level of expression from step (a) is greater than zero.

2. The method of claim 1, wherein the subject comprises a germ-free mouse model.

3. A method for identifying a subject suitable for a fecal matter transplant for treating or preventing the onset of a food allergy in a subject, the method comprising:
a. measuring a level of RORγt in regulatory T cells in a sample from a subject;
b. comparing the level of RORγt from (a) to the level of level of RORγt in regulatory T cells in a healthy subject;
c. identifying a subject as having decreased RORγt in regulatory T cells if the level from step (a) is statistically lower than the level in a healthy subject; and
d. if the subject has decreased RORγt in regulatory T cells, identifying the subject as one suitable for a fecal matter transplant,,
wherein the fecal matter is obtained from a healthy subject.

4. A fecal matter transplant for use in a method for treating or preventing the onset of a food allergy in a subject, wherein the subject has decreased levels of RORγt in regulatory T cells; and,
wherein the fecal matter is obtained from a healthy subject.

5. The fecal matter transplant for use of claim 4, wherein the subject is selected from the group consisting of: a newborn, an infant, a toddler, a child, and an adult.

6. The fecal matter transplant for use of claim 4 or 5, wherein the fecal matter transplant is to be administered prior to the first exposure to a potential food allergen, or wherein the agent is to be administered upon clinical signs of atopic symptoms.

7. The fecal matter transplant for use of any one of claims 4 to 6, wherein the subject has been diagnosed with at least one food allergy.

8. The fecal matter transplant for use of any one of claims 4 to 7, wherein the food allergy comprises allergy to soy, wheat, eggs, dairy, peanuts, tree nuts, shellfish, fish, mushrooms, stone fruits and other fruits.
